# EUROPEAN PATENT APPLICATION

(11) **EP 4 756 024 A1**
(43) Date of publication of application: **10.06.2026**
(21) Application number: 24848177.2
(22) Date of filing: 26.07.2024
(51) Int. Cl.: C12N 15/113, A61K 48/00, A61P 11/00

(54) **SIRNA AND COMPOSITION THEREOF**

(30) Priority: 28.07.2023 CN 202310944130; 08.02.2024 CN 202410176581
(71) Applicant: Changchun Genescience Pharmaceutical Co., Ltd., Changchun, Jilin 130012 (CN)
(72) Inventor: REN, Huanhuan, Changchun, Jilin 130012 (CN); LI, Fu, Changchun, Jilin 130012 (CN); LIU, Guanchen, Changchun, Jilin 130012 (CN); YANG, Xue, Changchun, Jilin 130012 (CN); DAI, Yan, Changchun, Jilin 130012 (CN); LI, Meng, Changchun, Jilin 130012 (CN); WEI, Yong, Changchun, Jilin 130012 (CN); XU, Peipei, Changchun, Jilin 130012 (CN); CHEN, Liu, Changchun, Jilin 130012 (CN); ZHOU, Ling, Changchun, Jilin 130012 (CN); XU, John Liuzhong, Changchun, Jilin 130012 (CN); WANG, Siqin, Changchun, Jilin 130012 (CN); JIN, Lei, Changchun, Jilin 130012 (CN)
(74) Representative: E. Blum & Co. AG
(86) International application number: PCT/CN2024/107828
(87) International publication number: WO 2025/026220

(57) **Abstract**

Provided is an siRNA that inhibits the expression of an MMP7 gene, which siRNA contains a sense strand and an antisense strand. The provided siRNA has good stability, excellent MMP7 gene inhibitory activity, and negligible cytotoxicity and immunostimulation, and can significantly reduce MMP7 protein levels at the animal level. The siRNA has important clinical values in the treatment of diseases or conditions benefiting from the reduction of MMP7 levels or the inhibition of MMP7 expression.

## Description

### CROSS-REFERENCE TO RELATED APPLICATIONS

The present application claims the priority of Chinese patent application no. 202310944130.X filed on July 28, 2023, and Chinese patent application no. 202410176581.8 filed on February 8, 2024, the entire contents of which are incorporated herein by reference in their entireties.

### TECHNICAL FIELD

The present invention provides an siRNA for inhibiting the expression of matrix metalloproteinase 7 (MMP7) and a pharmaceutical composition thereof. The siRNA and the pharmaceutical composition thereof provided by the present invention can treat matrix metalloproteinase 7-related diseases.

### BACKGROUND ART

Matrix metalloproteinase 7 (MMP7) is the smallest member of the matrix metalloproteinase (MMP) family, which comprises 24 related secretory zinc-dependent endopeptidases. These endopeptidases have distinct substrates and functions, and are capable of degrading components of the extracellular matrix as well as cleaving and regulating the activity of non-extracellular matrix substrates. Although they are attractive as drug targets, the development of MMP inhibitors is challenging due to the structural similarity of the zinc-dependent catalytic domains among family members. MMP7 is constitutively expressed and secreted by epithelial cells throughout the body, and plays a role in epithelial repair. Increased expression of MMP7 is associated with pathogenic fibrosis of the lung and liver. The level of MMP7 enzyme is associated with pathogenic fibrosis through a variety of potential mechanisms, including promoting epithelial-mesenchymal transition, extracellular matrix degradation, abnormal matrix repair, and tissue remodeling. MMP7 activates epithelial cells by cleaving E-cadherin and proteolytically activates the heparin-binding epidermal growth factor precursor to release active HB-EGF. These processes promote the epithelial-mesenchymal transition of epithelial cells and the proliferation of lung fibroblasts, respectively, ultimately leading to or promoting fibrosis.

Idiopathic pulmonary fibrosis (IPF) is a usually fatal chronic lung disease, and its clinical course and the rate of disease progression are relatively unpredictable. In patients with IPF, increased MMP7 expression has been observed in peripheral blood, bronchoalveolar lavage fluid and lung tissue. Serum MMP7 expression serves as a serum biomarker for IPF and is associated with the severity and progression of IPF. At present, there are only two drug options available for IPF patients, namely pirfenidone and nintedanib. However, the median survival time of patients receiving these drugs remains relatively short (only about 3 years), indicating a need for more and more effective drug options.

The small interfering RNA (siRNA) is an emerging and highly promising candidate study object, which can inhibit or block the expression of target genes of interest in a sequence-specific manner based on the RNA interference mechanism, thereby achieving the goal of treating diseases. Specifically, siRNA is loaded into the RNA-induced silencing complex (RISC). Its guide strand, also known as the antisense strand, pairs complementarily with the target nucleic acid in the mRNA of the target gene, leading to the degradation of the target gene's mRNA, thereby inhibiting or blocking the expression of the target gene. Since the siRNA recognizes the target RNA in a sequence-specific manner through base complementary pairing, thereby enabling the cleavage of the target RNA to achieve the goals of downregulating target RNA levels and inhibiting target gene expression, the siRNA has extremely broad application prospects.

However, considering the interspecies differences in MMP7, the difficulty of siRNA drug development is increased. Meanwhile, compared with traditional drugs, siRNA has poor stability, and systemic administration is prone to degradation by nucleases, which is a drawback. In addition, further efforts are needed to enhance its activity while avoiding side effects such as off-target effects, immune stimulation, and cytotoxicity. Therefore, it has become an urgent problem to be solved to develop more candidate siRNAs that inhibit the MMP7 gene expression, with characteristics of stability in the blood, good biological activity, and low cytotoxicity. Meanwhile, there is both clinical research necessity and commercial practicality in developing drugs that can effectively prevent and/or treat MMP7-related diseases using the aforementioned candidate siRNAs that inhibit MMP7 gene expression.

### SUMMARY OF THE INVENTION

The present invention provides an siRNA that can effectively inhibit the MMP7 gene expression, and thus provides a medicament and method for preventing and/or treating MMP7-related diseases.

### siRNA

In one aspect, the present invention provides a small interfering RNA (siRNA) for inhibiting the MMP7 gene expression, wherein the siRNA comprises a sense strand and an antisense strand, wherein the antisense strand comprises at least 17 consecutive nucleotides that differ from the nucleotide sequence set forth in any of SEQ ID NO: 1 to SEQ ID NO: 124 by no more than 4 nucleotides (e.g., 0, 1, 2, 3, or 4 nucleotides), and the sense strand is at least partially complementary to the antisense strand.

The expression "at least partially complementary" means that the two sequences can be completely complementary, or have no more than 6, 5, 4, 3, 2 or 1 mismatched base pairs overall, while retaining the ability to hybridize under relevant conditions. Those skilled in the art will be able to determine the most suitable conditions for testing the complementarity of two sequences depending on the ultimate application of the hybridized nucleotides. Such conditions can, for example, be stringent conditions, such as 400 mM NaCl, 40 mM PIPES pH 6.4, 1 mM EDTA, 50°C or 70°C for 12-16 hours, followed by washing. Other conditions, such as physiologically relevant conditions that may be encountered in an organism, may also apply.

In some embodiments, the antisense strand comprises at least 17 consecutive nucleotides that differ from the nucleotide sequence set forth in any of SEQ ID NO: 1 to SEQ ID NO: 124 by 0 or 1 nucleotide.

In some embodiments, the antisense strand comprises at least 17 consecutive nucleotides that differ from the nucleotide sequence set forth in any of SEQ ID NO: 1 to SEQ ID NO: 124 by no more than 4 nucleotides (e.g., 0, 1, 2, 3 or 4 nucleotides). In some embodiments, the antisense strand comprises at least 17 consecutive nucleotides that differ from the nucleotide sequence set forth in any of SEQ ID NO: 1 to SEQ ID NO: 124 by no more than 3 nucleotides (e.g., 0, 1, 2 or 3 nucleotides). In some embodiments, the antisense strand comprises at least 17 consecutive nucleotides that differ from the nucleotide sequence set forth in any of SEQ ID NO: 1 to SEQ ID NO: 124 by no more than 2 nucleotides (e.g., 0, 1 or 2 nucleotides). In some embodiments, the antisense strand comprises at least 17 consecutive nucleotides that differ from the nucleotide sequence set forth in any of SEQ ID NO: 1 to SEQ ID NO: 124 by no more than 1 nucleotide (e.g., 0 or 1 nucleotide). In some embodiments, the antisense strand comprises at least 17 consecutive nucleotides of the nucleotide sequence set forth in any of SEQ ID NO: 1 to SEQ ID NO: 124. In some embodiments, the antisense strand is the nucleotide sequence set forth in any of SEQ ID NO: 1 to SEQ ID NO: 124.

In some embodiments, there are no more than 6 nucleotide mismatches between the sense strand and the antisense strand. In some embodiments, there are no more than 5 nucleotide mismatches between the sense strand and the antisense strand. In some embodiments, there are no more than 4 nucleotide mismatches between the sense strand and the antisense strand. In some embodiments, there are no more than 3 nucleotide mismatches between the sense strand and the antisense strand. In some embodiments, there are no more than 2 nucleotide mismatches between the sense strand and the antisense strand. In some embodiments, there are no more than 1 nucleotide mismatches between the sense strand and the antisense strand. In some embodiments, the sense strand and the antisense strand are fully complementary.

In some embodiments, the sense strand and the antisense strand have at least 15, 16, 17, 18, 19, 20 or 21 complementary nucleotides.

In some embodiments, the sense strand and the antisense strand are at least 85% (e.g., at least 90%, at least 95%, at least 99%) complementary or fully complementary over at least 17 consecutive nucleotides.

In some embodiments, the sense strand and the antisense strand form a duplex region of 15-30 nucleotide pairs in length, for example, a duplex region of 15-25 nucleotide pairs, 15-23 nucleotide pairs, or 15-21 nucleotide pairs, such as 15, 16, 17, 18, 19, 20 or 21 nucleotide pairs.

In some embodiments, the antisense strand has a length of 17-30 (e.g., 17-29, 17-28, 17-27, 19-30, 19-29, 19-28, 19-27, 19-25, 19-23, 21-25, 21-23) nucleotides, and the sense strand has a length of 17-30 (e.g., 17-29, 17-28, 17-27, 17-26, 17-25, 17-21, 19-21) nucleotides.

In some embodiments, the antisense strand has a length of 19-27 nucleotides; and the sense strand has a length of 17-25 nucleotides.

In some embodiments, the antisense strand has a length of 21-23 nucleotides; and the sense strand has a length of 19-21 nucleotides.

In some embodiments, the antisense strand has a length of 23 nucleotides, and the sense strand has a length of 21 nucleotides.

In some embodiments, the siRNA comprises a blunt end and/or an overhang.

In some embodiments, the siRNA comprises an overhang of one or more single-stranded nucleotides, for example, an overhang of 1, 2, 3 or 4 nucleotides. In some embodiments, the overhang can be on the sense strand, the antisense strand, or any combination thereof. In some embodiments, the overhang is present at the 5' end, the 3' end or both ends of the antisense strand or the sense strand of the siRNA.

In some embodiments, the 3' end of the antisense strand of the siRNA comprises an overhang of 2 nucleotides. In some embodiments, the 3' end of the sense strand of the siRNA is a blunt end.

In some embodiments, the sense strand of the siRNA comprises at least 17 consecutive nucleotides that differ from the nucleotide sequence set forth in any of SEQ ID NO: 125 to SEQ ID NO: 248 by no more than 4 nucleotides (e.g., 0, 1, 2, 3 or 4 nucleotides). The at least 17 consecutive nucleotides comprised by the sense strand and the antisense strand of the siRNA form a duplex region. In some embodiments, the sense strand comprises at least 17 consecutive nucleotides that differ from the nucleotide sequence set forth in any of SEQ ID NO: 125 to SEQ ID NO: 248 by no more than 3 nucleotides (e.g., 0, 1, 2 or 3 nucleotides). In some embodiments, the sense strand comprises at least 17 consecutive nucleotides that differ from the nucleotide sequence set forth in any of SEQ ID NO: 125 to SEQ ID NO: 248 by no more than 2 nucleotides (e.g., 0, 1 or 2 nucleotides). In some embodiments, the sense strand comprises at least 17 consecutive nucleotides that differ from the nucleotide sequence set forth in any of SEQ ID NO: 125 to SEQ ID NO: 248 by no more than 1 nucleotide (e.g., 0 or 1 nucleotide). In some embodiments, the sense strand has a region within the 17 consecutive nucleotides that is at least 85% (e.g., at least 90%, at least 95%, at least 99%) complementary or fully complementary to the antisense strand. In some embodiments, the sense strand comprises at least 17 consecutive nucleotides of the nucleotide sequence set forth in any of SEQ ID NO: 125 to SEQ ID NO: 248. In some embodiments, the sense strand is the nucleotide sequence set forth in any of SEQ ID NO: 125 to SEQ ID NO: 248.

In some embodiments, the antisense strand of the siRNA comprises the antisense strand sequence of any of duplex 1 to duplex 124 provided in Table 1, or a portion thereof (e.g., at least 17 consecutive nucleotides thereof), and the sense strand of the siRNA comprises the sense strand sequence of the duplex, or a portion thereof (e.g., at least 17 consecutive nucleotides thereof).

In some embodiments, the sequences of the sense strand and the antisense strand of the siRNA are selected from the sense strand and antisense strand sequences of any of duplex 1 to duplex 124 described in Table 1.

In some embodiments, the antisense strand of the siRNA comprises the antisense strand sequence of duplex 44, duplex 9, duplex 33, duplex 12, duplex 61, duplex 8, duplex 11, duplex 38 or duplex 73 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 27, duplex 85, duplex 86, duplex 20, duplex 65, duplex 2, duplex 5, duplex 14, duplex 58, duplex 4, duplex 52, duplex 95, duplex 103, duplex 3, duplex 67, duplex 54, duplex 111, duplex 46, duplex 108 or duplex 119 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 24, duplex 34, duplex 50, duplex 53, duplex 59, duplex 72, duplex 75, duplex 87, duplex 42, duplex 94, duplex 1, duplex 35, duplex 79, duplex 82, duplex 25, duplex 39, duplex 71, duplex 112, duplex 124, duplex 100, duplex 109, duplex 113, duplex 30, duplex 105, duplex 115, duplex 7, duplex 51, duplex 15, duplex 97 or duplex 121 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 69, duplex 81, duplex 88, duplex 96, duplex 22, duplex 32, duplex 47, duplex 40, duplex 21, duplex 29, duplex 31, duplex 45, duplex 63, duplex 18, duplex 70, duplex 114, duplex 123, duplex 41, duplex 90, duplex 122, duplex 84 or duplex 10 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 6, duplex 60, duplex 80, duplex 118, duplex 16, duplex 55, duplex 116, duplex 19, duplex 120, duplex 13, duplex 74, duplex 23, duplex 56 or duplex 76 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 2, duplex 5, duplex 8, duplex 9, duplex 11, duplex 12, duplex 33, duplex 35, duplex 38, duplex 46, duplex 50, duplex 67 or duplex 73 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

In some embodiments, the antisense strand of the siRNA comprises the antisense strand sequence of duplex 2, duplex 9, duplex 11, duplex 12, duplex 38, duplex 46, duplex 67 or duplex 73 provided in Table 1, or a portion thereof (e.g., at least 17 consecutive nucleotides thereof). Preferably, the sense strand of the siRNA comprises the sense strand sequence of the duplex or a portion thereof (e.g., at least 17 consecutive nucleotides thereof).

### Modification

The siRNA of the present invention can be modified in the nucleobase structure or in the ribose-phosphate backbone structure to reduce off-target effects and/or increase the biological stability of the molecule or increase the physical stability of the duplex formed between the antisense and sense nucleic acids. Therefore, siRNA sequences comprising any modification are also encompassed within the scope of the present invention. The siRNA molecules comprising ribonucleoside analogs or derivatives must retain the ability to form duplexes and allow or mediate specific degradation of the target RNA via the RISC pathway.

In some embodiments of the present invention, the siRNA at least comprises one modified nucleotide. The modifications need not be identical for each of the multiple modified ribonucleosides in the siRNA.

In some embodiments of the present invention, all nucleotides in the sense strand and/or antisense strand of the siRNA are modified nucleotides or nucleotide analogs.

In some embodiments of the present invention, the siRNA comprises 2'-modified nucleotides.

In some embodiments of the present invention, the modified nucleotides are selected from 2'-methoxynucleotides, 2'-fluoronucleotides, 2'-deoxynucleotides, 2',3'-seco-nucleotide analogs, 2'-fluoroarabinonucleotides, 2'-methoxyethyl nucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphate-containing nucleotides, 5'-phosphate mimic-containing nucleotides, diol-modified nucleotides, abasic nucleotides, morpholino nucleotides, locked nucleotides (LNA), unlocked nucleotides (UNA), or glycerol nucleotides (GNA), but not limited thereto in the present invention.

In some embodiments of the present invention, the antisense strand of the siRNA has a length of 23 nucleotides and comprises:
1) AS(5'-3'): mN*fN*mNmNmNfNmNfNfNmNmNfNmNfNmNfNmNmNmNmNmN*mN*mN (SEQ ID NO: 380);
2) AS(5'-3'): mN*fN*mNmNmNmNmNmNfNmNmNfNmNfNmNfNmNmNmNmNmN*mN*mN (SEQ ID NO: 381);
3) AS(5'-3'): mN*fN*mNfNmNmNmNmNmNmNmNfNmNfNmNfNmNmNmNmNmN*mN*mN (SEQ ID NO: 382);
4) AS(5'-3'): mN*fN*mNmNmNfNmNfNfNfNmNmNmNfNmNfNmNmNmNmNmN*mN*mN (SEQ ID NO: 15383);
5) AS(5'-3'): mN*fN*mNfNmNfNmNfNmNmNmNmNmNfNmNfNmNfNmNfNmN*mN*mN (SEQ ID NO: 384);
6) AS(5'-3'): mN*fN*mNmNmNfNmNmNmNmNmNmNmNfNmNfNmNfNmNmNmN*mN*mN (SEQ ID NO: 385);
7) AS(5'-3'): mN*fN*mNmNmNmNmNfNfNmNmNmNmNfNmNfNmNmNmNmNmN*mN*mN (SEQ ID NO: 386);
8) AS(5'-3'): mN*fN*mNfNmNfNmNfNmNfNmNmNmNfNmNfNmNfNmNfNmN*mN*mN (SEQ ID NO: 387);
9) AS(5'-3'): (E)-VPmN*fN*mNmNmNmNmNfNfNmNmNmNmNfNmNfNmNmNmNmNmN*mN*mN (SEQ ID NO: 388);
10) AS(5'-3'): (E)-VPmN*fN*mNmNmNfNmNfNfNmNmNfNmNfNmNfNmNmNmNmNmN*mN*mN (SEQ ID NO: 389);
11) AS(5'-3'): (E)-VPmN*fN*mNmNmNmNmNmNfNmNmNfNmNfNmNfNmNmNmNmNmN*mN*mN (SEQ ID NO: 390); or,
12) AS(5'-3'): (E)-VPmN*fN*mNmNmNfNmNfNfNfNmNmNmNfNmNfNmNmNmNmNmN*mN*mN (SEQ ID NO: 391);
wherein, mN is a methoxy-modified nucleotide, fN is a fluorinated nucleotide, (E)-VP indicates that the nucleotide adjacent to its right is a (E)-vinylphosphonate-modified nucleotide, and * indicates a phosphorothioate linkage.

In some embodiments of the present invention, the antisense strand of the siRNA has a length of 23 nucleotides and comprises:
1) 2'-fluoronucleotides at positions 2, 6, 8, 14 and 16 from the 5' end of the antisense strand, with the remaining positions being 2'-methoxynucleotides; or
2) 2'-fluoronucleotides at positions 2, 6, 14, 16 and 18 from the 5' end of the antisense strand, with the remaining positions being 2'-methoxynucleotides; or
3) 2'-fluoronucleotides at positions 2, 6, 14, 16 and 20 from the 5' end of the antisense strand, with the remaining positions being 2'-methoxynucleotides; or
4) 2'-fluoronucleotides at positions 2, 6, 8, 9, 12, 14 and 16 from the 5' end of the antisense strand, with the remaining positions being 2'-methoxynucleotides; or
5) 2'-fluoronucleotides at positions 2, 9, 12, 14 and 16 from the 5' end of the antisense strand, with the remaining positions being 2'-methoxynucleotides; or
6) 2'-fluoronucleotides at positions 2, 4, 12, 14 and 16 from the 5' end of the antisense strand, with the remaining positions being 2'-methoxynucleotides; or
7) 2'-fluoronucleotides at positions 2, 6, 8, 9, 10, 14 and 16 from the 5' end of the antisense strand, with the remaining positions being 2'-methoxynucleotides; or
8) 2'-fluoronucleotides at positions 2, 4, 6, 8, 10, 14, 16, 18 and 20 from the 5' end of the antisense strand, with the remaining positions being 2'-methoxynucleotides; or
9) 2'-fluoronucleotides at positions 2, 8, 9, 14 and 16 from the 5' end of the antisense strand, with the remaining positions being 2'-methoxynucleotides; or
10) 2'-fluoronucleotides at positions 2, 4, 6, 8, 14, 16, 18 and 20 from the 5' end of the antisense strand, with the remaining positions being 2'-methoxynucleotides.

In some embodiments of the present invention, the sense strand of the siRNA has a length of 21 nucleotides and comprises:
1) SS(5'-3'): mN*mN*mNmNmNmNfNmNfNmNfNmNmNmNmNmNmNmNmNmNmN (SEQ ID NO: 392);
2) SS(5'-3'): mN*mN*mNmNmNmNmNmNfNmNfNmNfNmNmNmNmNmNmNmNmN (SEQ ID NO: 393);
3) SS(5'-3'): mN*mN*mNmNmNmNfNmNfNfNfNmNmNmNfNmNmNmNmNmNmN (SEQ ID NO: 394);
4) SS(5'-3'): mN*mN*mNmNmNmNfNmNfNfNdNmNmNmNmNmNmNmNmNmNmN (SEQ ID NO: 395);
5) SS(5'-3'): mN*mN*mNmNmNmNfNmNfNfNfNmNfNmNmNmNmNmNmNmNmN (SEQ ID NO: 396);
6) SS(5'-3'): mN*mN*mNmNmNmNfNmNfNfNfNmNmNmNmNfNmNmNmNmNmN (SEQ ID NO: 397);
7) SS(5'-3'): mN*mN*mNmNmNmNfNmNfNfNfNmNmNmNmNmNfNmNmNmNmN (SEQ ID NO: 398);
8) SS(5'-3'): mN*mN*mNmNmNmNfNmNfNdNfNmNmNmNmNmNfNmNmNmN*mN (SEQ ID NO: 399);
9) SS(5'-3'): mN*mN*mNmNmNmNfNmNdNfNfNmNmNmNmNmNfNmNmNmN*mN (SEQ ID NO: 400);
10) SS(5'-3'): mN*mN*mNmNmNmNfNmNfNfNdNmNmNmNmNmNfNmNmNmN*mN (SEQ ID NO: 401);
11) SS(5'-3'): mN*mN*mNmNmNmNmNmNfNfNfNmNmNmNmNmNfNmNmNmN*mN (SEQ ID NO: 402);
12) SS(5'-3'): mN*mN*mNmNmNmNfNmNfNfNfNmNmNmNmNmNfNmNmNmN*mN (SEQ ID NO: 403);
13) SS(5'-3'): mN*mN*mNmNmNmNfNmNfNfNfNmNmNmNmNmNfNmNmN*mN*mN (SEQ ID NO: 404);
14) SS(5'-3'): mN*mN*mNmNmNmNfNmNfNfNfNmNmNmNmNfNfNmNmNmN*mN (SEQ ID NO: 405);
15) SS(5'-3'): mN*mN*mNmNmNmNfNmNfNfNfNmNmNmNfNmNfNmNmNmN*mN (SEQ ID NO: 406);
16) SS(5'-3'): mN*mN*mNmNmNmNfNmNfNdNfNmNmNmNmNmNmNmNmNmN*mN (SEQ ID NO: 407);
17) SS(5'-3'): mN*mN*mNmNmNmNfNmNfNmNfNmNmNmNmNmNmNmNmN*mN*mN (SEQ ID NO: 408);
18) SS(5'-3'): mN*mN*mNmNmNmNmNmNfNmNfNmNfNmNmNmNmNmNmN*mN*mN (SEQ ID NO: 409);
19) SS(5'-3'): mN*mN*mNmNmNmNfNmNfNfNdNmNmNmNmNmNmNmNmN*mN*mN (SEQ ID NO: 410);
20) SS(5'-3'): mN*mN*mNmNmNmNfNmNfNdNfNmNmNmNmNmNmNmNmN*mN*mN (SEQ ID NO: 411);
21) SS(5'-3'): mN*mN*mNmNmNmNfNmNfNmNfNmNmNmNmNmNmNmNmNmNmN*(iab) (SEQ ID NO: 412);
22) SS(5'-3'): mN*mN*mNmNmNmNmNmNfNmNfNmNfNmNmNmNmNmNmNmNmN*(iab) (SEQ ID NO: 413);
23) SS(5'-3'): mN*mN*mNmNmNmNfNmNfNfNdNmNmNmNmNmNmNmNmNmNmN*(iab) (SEQ ID NO: 414);
24) SS(5'-3'): mN*mN*mNmNmNmNfNmNfNdNfNmNmNmNmNmNmNmNmNmNmN*(iab) (SEQ ID NO: 415); or,
25) SS(5'-3'): mN*mN*mNmNmNmNfNmNfNfNfNmNmNmNmNmNfNmNmNmNmN*(iab) (SEQ ID NO: 416);
wherein mN is a methoxy-modified nucleotide, fN is a fluorinated nucleotide, dN is a deoxynucleotide, * is a phosphorothioate linkage, and (iab) is an inverted abasic residue.

In some embodiments of the present invention, the sense strand of the siRNA has a length of 21 nucleotides and comprises:
1) 2'-fluoronucleotides at positions 7, 9 and 11 from the 5' end of the sense strand, with the remaining positions being 2'-methoxynucleotides; or
2) 2'-fluoronucleotides at positions 7, 9 and 10 from the 5' end of the sense strand, and a deoxynucleotide at position 11, with the remaining positions being 2'-methoxynucleotides; or
3) 2'-fluoronucleotides at positions 7, 9, 10, 11 and 13 from the 5' end of the sense strand, with the remaining positions being 2'-methoxynucleotides; or
4) 2'-fluoronucleotides at positions 7, 9, 10, 11 and 14 from the 5' end of the sense strand, with the remaining positions being 2'-methoxynucleotides; or
5) 2'-fluoronucleotides at positions 7, 9, 10, 11 and 15 from the 5' end of the sense strand, with the remaining positions being 2'-methoxynucleotides; or
6) 2'-fluoronucleotides at positions 7, 9, 10, 11 and 17 from the 5' end of the sense strand, with the remaining positions being 2'-methoxynucleotides; or
7) 2'-fluoronucleotides at positions 7, 9, 10, 11 and 19 from the 5' end of the sense strand, with the remaining positions being 2'-methoxynucleotides; or
8) 2'-fluoronucleotides at positions 9, 11 and 13 from the 5' end of the sense strand, with the remaining positions being 2'-methoxynucleotides; or
9) 2'-fluoronucleotides at positions 7, 9, 10, 11 and 16 from the 5' end of the sense strand, with the remaining positions being 2'-methoxynucleotides; or
10) 2'-fluoronucleotides at positions 7, 9, 11 and 17 from the 5' end of the sense strand, and a deoxynucleotide at position 10, with the remaining positions being 2'-methoxynucleotides; or
11) 2'-fluoronucleotides at positions 7, 9 and 11 from the 5' end of the sense strand, and a deoxynucleotide at position 10, with the remaining positions being 2'-methoxynucleotides; or
12) 2'-fluoronucleotides at positions 7, 10, 11 and 17 from the 5' end of the sense strand, and a deoxynucleotide at position 9, with the remaining positions being 2'-methoxynucleotides; or
13) 2'-fluoronucleotides at positions 7, 9, 10 and 17 from the 5' end of the sense strand, and a deoxynucleotide at position 11, with the remaining positions being 2'-methoxynucleotides; or
14) 2'-fluoronucleotides at positions 9, 10, 11 and 17 from the 5' end of the sense strand, with the remaining positions being 2'-methoxynucleotides; or
15) 2'-fluoronucleotides at positions 7, 9, 10, 11, 16 and 17 from the 5' end of the sense strand, with the remaining positions being 2'-methoxynucleotides; or
16) 2'-fluoronucleotides at positions 7, 9, 10, 11, 15 and 17 from the 5' end of the sense strand, with the remaining positions being 2'-methoxynucleotides.

In some embodiments, the siRNA comprises a modification pattern selected from the following list, wherein the first SEQ ID NO corresponds to the antisense strand and the second SEQ ID NO corresponds to the sense strand: SEQ ID NO: 380 and SEQ ID NO: 392; SEQ ID NO: 380 and SEQ ID NO: 393; SEQ ID NO: 380 and SEQ ID NO: 394; SEQ ID NO: 380 and SEQ ID NO: 395; SEQ ID NO: 380 and SEQ ID NO: 396; SEQ ID NO: 380 and SEQ ID NO: 397; SEQ ID NO: 380 and SEQ ID NO: 398; SEQ ID NO: 380 and SEQ ID NO: 399; SEQ ID NO: 380 and SEQ ID NO: 400; SEQ ID NO: 380 and SEQ ID NO: 401; SEQ ID NO: 380 and SEQ ID NO: 402; SEQ ID NO: 380 and SEQ ID NO: 403; SEQ ID NO: 380 and SEQ ID NO: 404; SEQ ID NO: 380 and SEQ ID NO: 405; SEQ ID NO: 380 and SEQ ID NO: 406; SEQ ID NO: 380 and SEQ ID NO: 407; SEQ ID NO: 380 and SEQ ID NO: 408; SEQ ID NO: 380 and SEQ ID NO: 409; SEQ ID NO: 380 and SEQ ID NO: 410; SEQ ID NO: 380 and SEQ ID NO: 411; SEQ ID NO: 380 and SEQ ID NO: 412; SEQ ID NO: 380 and SEQ ID NO: 413; SEQ ID NO: 380 and SEQ ID NO: 414; SEQ ID NO: 380 and SEQ ID NO: 415; SEQ ID NO: 380 and SEQ ID NO: 416;
SEQ ID NO: 381 and SEQ ID NO: 392; SEQ ID NO: 381 and SEQ ID NO: 393; SEQ ID NO: 381 and SEQ ID NO: 394; SEQ ID NO: 381 and SEQ ID NO: 395; SEQ ID NO: 381 and SEQ ID NO: 396; SEQ ID NO: 381 and SEQ ID NO: 397; SEQ ID NO: 381 and SEQ ID NO: 398; SEQ ID NO: 381 and SEQ ID NO: 399; SEQ ID NO: 381 and SEQ ID NO: 400; SEQ ID NO: 381 and SEQ ID NO: 401; SEQ ID NO: 381 and SEQ ID NO: 402; SEQ ID NO: 381 and SEQ ID NO: 403; SEQ ID NO: 381 and SEQ ID NO: 404; SEQ ID NO: 381 and SEQ ID NO: 405; SEQ ID NO: 381 and SEQ ID NO: 406; SEQ ID NO: 381 and SEQ ID NO: 407; SEQ ID NO: 381 and SEQ ID NO: 408; SEQ ID NO: 381 and SEQ ID NO: 409; SEQ ID NO: 381 and SEQ ID NO: 410; SEQ ID NO: 381 and SEQ ID NO: 411; SEQ ID NO: 381 and SEQ ID NO: 412; SEQ ID NO: 381 and SEQ ID NO: 413; SEQ ID NO: 381 and SEQ ID NO: 414; 30 SEQ ID NO: 381 and SEQ ID NO: 415; SEQ ID NO: 381 and SEQ ID NO: 416;
SEQ ID NO: 382 and SEQ ID NO: 392; SEQ ID NO: 382 and SEQ ID NO: 393; SEQ ID NO: 382 and SEQ ID NO: 394; SEQ ID NO: 382 and SEQ ID NO: 395; SEQ ID NO: 382 and SEQ ID NO: 396; SEQ ID NO: 382 and SEQ ID NO: 397; SEQ ID NO: 382 and SEQ ID NO: 398; SEQ ID NO: 382 and SEQ ID NO: 399; SEQ ID NO: 382 and SEQ ID NO: 400; SEQ ID NO: 382 and SEQ ID NO: 401; SEQ ID NO: 382 and SEQ ID NO: 402; SEQ ID NO: 382 and SEQ ID NO: 403; SEQ ID NO: 382 and SEQ ID NO: 404; SEQ ID NO: 382 and SEQ ID NO: 405; SEQ ID NO: 382 and SEQ ID NO: 406; SEQ ID NO: 382 and SEQ ID NO: 407; SEQ ID NO: 382 and SEQ ID NO: 408; SEQ ID NO: 382 and SEQ ID NO: 409; SEQ ID NO: 382 and SEQ ID NO: 410; SEQ ID NO: 382 and SEQ ID NO: 411; SEQ ID NO: 382 and SEQ ID NO: 412; SEQ ID NO: 382 and SEQ ID NO: 413; SEQ ID NO: 382 and SEQ ID NO: 414; SEQ ID NO: 382 and SEQ ID NO: 415; SEQ ID NO: 382 and SEQ ID NO: 416;
SEQ ID NO: 383 and SEQ ID NO: 392; SEQ ID NO: 383 and SEQ ID NO: 393; SEQ ID NO: 383 and SEQ ID NO: 394; SEQ ID NO: 383 and SEQ ID NO: 395; SEQ ID NO: 383 and SEQ ID NO: 396; SEQ ID NO: 383 and SEQ ID NO: 397; SEQ ID NO: 383 and SEQ ID NO: 398; SEQ ID NO: 383 and SEQ ID NO: 399; SEQ ID NO: 383 and SEQ ID NO: 400; SEQ ID NO: 383 and SEQ ID NO: 401; SEQ ID NO: 383 and SEQ ID NO: 402; SEQ ID NO: 383 and SEQ ID NO: 403; SEQ ID NO: 383 and SEQ ID NO: 404; SEQ ID NO: 383 and SEQ ID NO: 405; SEQ ID NO: 383 and SEQ ID NO: 406; SEQ ID NO: 383 and SEQ ID NO: 407; SEQ ID NO: 383 and SEQ ID NO: 408; SEQ ID NO: 383 and SEQ ID NO: 409; SEQ ID NO: 383 and SEQ ID NO: 410; SEQ ID NO: 383 and SEQ ID NO: 411; SEQ ID NO: 383 and SEQ ID NO: 412; SEQ ID NO: 383 and SEQ ID NO: 413; SEQ ID NO: 383 and SEQ ID NO: 414; SEQ ID NO: 383 and SEQ ID NO: 415; SEQ ID NO: 383 and SEQ ID NO: 416;
SEQ ID NO: 384 and SEQ ID NO: 392; SEQ ID NO: 384 and SEQ ID NO: 393; SEQ ID NO: 384 and SEQ ID NO: 394; SEQ ID NO: 384 and SEQ ID NO: 395; SEQ ID NO: 384 and SEQ ID NO: 396; SEQ ID NO: 384 and SEQ ID NO: 397; SEQ ID NO: 384 and SEQ ID NO: 398; SEQ ID NO: 384 and SEQ ID NO: 399; SEQ ID NO: 384 and SEQ ID NO: 400; SEQ ID NO: 384 and SEQ ID NO: 401; SEQ ID NO: 384 and SEQ ID NO: 402; SEQ ID NO: 384 and SEQ ID NO: 403; SEQ ID NO: 384 and SEQ ID NO: 404; SEQ ID NO: 384 and SEQ ID NO: 405; SEQ ID NO: 384 and SEQ ID NO: 406; SEQ ID NO: 384 and SEQ ID NO: 407; SEQ ID NO: 384 and SEQ ID NO: 408; SEQ ID NO: 384 and SEQ ID NO: 409; SEQ ID NO: 384 and SEQ ID NO: 410; SEQ ID NO: 384 and SEQ ID NO: 411; SEQ ID NO: 384 and SEQ ID NO: 412; SEQ ID NO: 384 and SEQ ID NO: 413; SEQ ID NO: 384 and SEQ ID NO: 414; SEQ ID NO: 384 and SEQ ID NO: 415; SEQ ID NO: 384 and SEQ ID NO: 416;
SEQ ID NO: 385 and SEQ ID NO: 392; SEQ ID NO: 385 and SEQ ID NO: 393; SEQ ID NO: 385 and SEQ ID NO: 394; SEQ ID NO: 385 and SEQ ID NO: 395; SEQ ID NO: 385 and SEQ ID NO: 396; SEQ ID NO: 385 and SEQ 30ID NO: 397; SEQ ID NO: 385 and SEQ ID NO: 398; SEQ ID NO: 385 and SEQ ID NO: 399; SEQ ID NO: 385 and SEQ ID NO: 400; SEQ ID NO: 385 and SEQ ID NO: 401; SEQ ID NO: 385 and SEQ ID NO: 402; SEQ ID NO: 385 and SEQ ID NO: 403; SEQ ID NO: 385 and SEQ ID NO: 404; SEQ ID NO: 385 and SEQ ID NO: 405; SEQ ID NO: 385 and SEQ ID NO: 406; SEQ ID NO: 385 and SEQ ID NO: 407; SEQ ID NO: 385 and SEQ ID NO: 408; SEQ ID NO: 385 and SEQ ID NO: 409; SEQ ID NO: 385 and SEQ ID NO: 410; SEQ ID NO: 385 and SEQ ID NO: 411; SEQ ID NO: 385 and SEQ ID NO: 412; SEQ ID NO: 385 and SEQ ID NO: 413; SEQ ID NO: 385 and SEQ ID NO: 414; SEQ ID NO: 385 and SEQ ID NO: 415; SEQ ID NO: 385 and SEQ ID NO: 416;
SEQ ID NO: 386 and SEQ ID NO: 392; SEQ ID NO: 386 and SEQ ID NO: 393; SEQ ID NO: 386 and SEQ ID NO: 394; SEQ ID NO: 386 and SEQ ID NO: 395; SEQ ID NO: 386 and SEQ ID NO: 396; SEQ ID NO: 386 and SEQ ID NO: 397; SEQ ID NO: 386 and SEQ ID NO: 398; SEQ ID NO: 386 and SEQ ID NO: 399; SEQ ID NO: 386 and SEQ ID NO: 400; SEQ ID NO: 386 and SEQ ID NO: 401; SEQ ID NO: 386 and SEQ ID NO: 402; SEQ ID NO: 386 and SEQ ID NO: 403; SEQ ID NO: 386 and SEQ ID NO: 404; SEQ ID NO: 386 and SEQ ID NO: 405; SEQ ID NO: 386 and SEQ ID NO: 406; SEQ ID NO: 386 and SEQ ID NO: 407; SEQ ID NO: 386 and SEQ ID NO: 408; SEQ ID NO: 386 and SEQ ID NO: 409; SEQ ID NO: 386 and SEQ ID NO: 410; SEQ ID NO: 386 and SEQ ID NO: 411; SEQ ID NO: 386 and SEQ ID NO: 412; SEQ ID NO: 386 and SEQ ID NO: 413; SEQ ID NO: 386 and SEQ ID NO: 414; SEQ ID NO: 386 and SEQ ID NO: 415; SEQ ID NO: 386 and SEQ ID NO: 416;
SEQ ID NO: 387 and SEQ ID NO: 392; SEQ ID NO: 387 and SEQ ID NO: 393; SEQ ID NO: 387 and SEQ ID NO: 394; SEQ ID NO: 387 and SEQ ID NO: 395; SEQ ID NO: 387 and SEQ ID NO: 396; SEQ ID NO: 387 and SEQ ID NO: 397; SEQ ID NO: 387 and SEQ ID NO: 398; SEQ ID NO: 387 and SEQ ID NO: 399; SEQ ID NO: 387 and SEQ ID NO: 400; SEQ ID NO: 387 and SEQ ID NO: 401; SEQ ID NO: 387 and SEQ ID NO: 402; SEQ ID NO: 387 and SEQ ID NO: 403; SEQ ID NO: 387 and SEQ ID NO: 404; SEQ ID NO: 387 and SEQ ID NO: 405; SEQ ID NO: 387 and SEQ ID NO: 406; SEQ ID NO: 387 and SEQ ID NO: 407; SEQ ID NO: 387 and SEQ ID NO: 408; SEQ ID NO: 387 and SEQ ID NO: 409; SEQ ID NO: 387 and SEQ ID NO: 410; SEQ ID NO: 387 and SEQ ID NO: 411; SEQ ID NO: 387 and SEQ ID NO: 412; SEQ ID NO: 387 and SEQ ID NO: 413; SEQ ID NO: 387 and SEQ ID NO: 414; SEQ ID NO: 387 and SEQ ID NO: 415; SEQ ID NO: 387 and SEQ ID NO: 416;
SEQ ID NO: 388 and SEQ ID NO: 392; SEQ ID NO: 388 and SEQ ID NO: 393; SEQ ID NO: 388 and SEQ ID NO: 394; SEQ ID NO: 388 and SEQ ID NO: 395; SEQ ID NO: 388 and SEQ ID NO: 396; SEQ ID NO: 388 and SEQ ID NO: 397; SEQ ID NO: 388 and SEQ ID NO: 398; SEQ ID NO: 388 and SEQ ID NO: 399; SEQ ID NO: 388 and SEQ ID NO: 400; SEQ ID NO: 388 and SEQ ID NO: 401; SEQ ID NO: 388 and SEQ ID NO: 402; SEQ ID NO: 388 and SEQ ID NO: 403; SEQ ID NO: 388 and SEQ ID NO: 404; SEQ ID NO: 388 and SEQ ID NO: 405; SEQ ID NO: 30388 and SEQ ID NO: 406; SEQ ID NO: 388 and SEQ ID NO: 407; SEQ ID NO: 388 and SEQ ID NO: 408; SEQ ID NO: 388 and SEQ ID NO: 409; SEQ ID NO: 388 and SEQ ID NO: 410; SEQ ID NO: 388 and SEQ ID NO: 411; SEQ ID NO: 388 and SEQ ID NO: 412; SEQ ID NO: 388 and SEQ ID NO: 413; SEQ ID NO: 388 and SEQ ID NO: 414; SEQ ID NO: 388 and SEQ ID NO: 415; SEQ ID NO: 388 and SEQ ID NO: 416;
SEQ ID NO: 389 and SEQ ID NO: 392; SEQ ID NO: 389 and SEQ ID NO: 393; SEQ ID NO: 389 and SEQ ID NO: 394; SEQ ID NO: 389 and SEQ ID NO: 395; SEQ ID NO: 389 and SEQ ID NO: 396; SEQ ID NO: 389 and SEQ ID NO: 397; SEQ ID NO: 389 and SEQ ID NO: 398; SEQ ID NO: 389 and SEQ ID NO: 399; SEQ ID NO: 389 and SEQ ID NO: 400; SEQ ID NO: 389 and SEQ ID NO: 401; SEQ ID NO: 389 and SEQ ID NO: 402; SEQ ID NO: 389 and SEQ ID NO: 403; SEQ ID NO: 389 and SEQ ID NO: 404; SEQ ID NO: 389 and SEQ ID NO: 405; SEQ ID NO: 389 and SEQ ID NO: 406; SEQ ID NO: 389 and SEQ ID NO: 407; SEQ ID NO: 389 and SEQ ID NO: 408; SEQ ID NO: 389 and SEQ ID NO: 409; SEQ ID NO: 389 and SEQ ID NO: 410; SEQ ID NO: 389 and SEQ ID NO: 411; SEQ ID NO: 389 and SEQ ID NO: 412; SEQ ID NO: 389 and SEQ ID NO: 413; SEQ ID NO: 389 and SEQ ID NO: 414; SEQ ID NO: 389 and SEQ ID NO: 415; SEQ ID NO: 389 and SEQ ID NO: 416;
SEQ ID NO: 390 and SEQ ID NO: 392; SEQ ID NO: 390 and SEQ ID NO: 393; SEQ ID NO: 390 and SEQ ID NO: 394; SEQ ID NO: 390 and SEQ ID NO: 395; SEQ ID NO: 390 and SEQ ID NO: 396; SEQ ID NO: 390 and SEQ ID NO: 397; SEQ ID NO: 390 and SEQ ID NO: 398; SEQ ID NO: 390 and SEQ ID NO: 399; SEQ ID NO: 390 and SEQ ID NO: 400; SEQ ID NO: 390 and SEQ ID NO: 401; SEQ ID NO: 390 and SEQ ID NO: 402; SEQ ID NO: 390 and SEQ ID NO: 403; SEQ ID NO: 390 and SEQ ID NO: 404; SEQ ID NO: 390 and SEQ ID NO: 405; SEQ ID NO: 390 and SEQ ID NO: 406; SEQ ID NO: 390 and SEQ ID NO: 407; SEQ ID NO: 390 and SEQ ID NO: 408; SEQ ID NO: 390 and SEQ ID NO: 409; SEQ ID NO: 390 and SEQ ID NO: 410; SEQ ID NO: 390 and SEQ ID NO: 411; SEQ ID NO: 390 and SEQ ID NO: 412; SEQ ID NO: 390 and SEQ ID NO: 413; SEQ ID NO: 390 and SEQ ID NO: 414; SEQ ID NO: 390 and SEQ ID NO: 415; SEQ ID NO: 390 and SEQ ID NO: 416;
SEQ ID NO: 391 and SEQ ID NO: 392; SEQ ID NO: 391 and SEQ ID NO: 393; SEQ ID NO: 391 and SEQ ID NO: 394; SEQ ID NO: 391 and SEQ ID NO: 395; SEQ ID NO: 391 and SEQ ID NO: 396; SEQ ID NO: 391 and SEQ ID NO: 397; SEQ ID NO: 391 and SEQ ID NO: 398; SEQ ID NO: 391 and SEQ ID NO: 399; SEQ ID NO: 391 and SEQ ID NO: 400; SEQ ID NO: 391 and SEQ ID NO: 401; SEQ ID NO: 391 and SEQ ID NO: 402; SEQ ID NO: 391 and SEQ ID NO: 403; SEQ ID NO: 391 and SEQ ID NO: 404; SEQ ID NO: 391 and SEQ ID NO: 405; SEQ ID NO: 391 and SEQ ID NO: 406; SEQ ID NO: 391 and SEQ ID NO: 407; SEQ ID NO: 391 and SEQ ID NO: 408; SEQ ID NO: 391 and SEQ ID NO: 409; SEQ ID NO: 391 and SEQ ID NO: 410; SEQ ID NO: 391 and SEQ ID NO: 411; SEQ ID NO: 391 and SEQ ID NO: 412; SEQ ID NO: 391 and SEQ ID NO: 413; SEQ ID NO: 391 and SEQ ID NO: 414; SEQ ID NO: 391 and SEQ ID NO: 415; SEQ ID NO: 391 and SEQ ID NO: 416.

In some embodiments, the siRNA comprises a modification pattern selected from the following list, wherein the first SEQ ID NO corresponds to the antisense strand and the second SEQ ID NO corresponds to the sense strand: SEQ ID NO: 380 and SEQ ID NO: 392; SEQ ID NO: 380 and SEQ ID NO: 393; SEQ ID NO: 381 and SEQ ID NO: 393; SEQ ID NO: 382 and SEQ ID NO: 392; SEQ ID NO: 383 and SEQ ID NO: 394; SEQ ID NO: 383 and SEQ ID NO: 395; SEQ ID NO: 384 and SEQ ID NO: 395; SEQ ID NO: 385 and SEQ ID NO: 396; SEQ ID NO: 386 and SEQ ID NO: 397; SEQ ID NO: 386 and SEQ ID NO: 398; SEQ ID NO: 386 and SEQ ID NO: 399; SEQ ID NO: 386 and SEQ ID NO: 400; SEQ ID NO: 386 and SEQ ID NO: 401; SEQ ID NO: 386 and SEQ ID NO: 402; SEQ ID NO: 386 and SEQ ID NO: 403; SEQ ID NO: 386 and SEQ ID NO: 404; SEQ ID NO: 386 and SEQ ID NO: 405; SEQ ID NO: 386 and SEQ ID NO: 406; SEQ ID NO: 387 and SEQ ID NO: 395; SEQ ID NO: 387 and SEQ ID NO: 394; SEQ ID NO: 388 and SEQ ID NO: 403; SEQ ID NO: 388 and SEQ ID NO: 399; SEQ ID NO: 388 and SEQ ID NO: 407; SEQ ID NO: 388 and SEQ ID NO: 406; SEQ ID NO: 388 and SEQ ID NO: 415; SEQ ID NO: 388 and SEQ ID NO: 416; SEQ ID NO: 388 and SEQ ID NO: 404; SEQ ID NO: 388 and SEQ ID NO: 402; SEQ ID NO: 388 and SEQ ID NO: 411; SEQ ID NO: 389 and SEQ ID NO: 408; SEQ ID NO: 389 and SEQ ID NO: 412; SEQ ID NO: 390 and SEQ ID NO: 409; SEQ ID NO: 390 and SEQ ID NO: 413; SEQ ID NO: 391 and SEQ ID NO: 410; SEQ ID NO: 391 and SEQ ID NO: 414.

In some embodiments of the present invention, the antisense strand of the siRNA has a length of 23 nucleotides, wherein positions 2, 6, 8, 9, 12, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 9, 12, 14 and 16 from the 5' end of the antisense strand are 2'-fluoronucleotides, and the remaining positions are 2'-methoxynucleotides; and the sense strand of the siRNA has a length of 21 nucleotides, wherein positions 9, 11 and 13 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 9, 12, 14 and 16 from the 5' end of the antisense strand are 2'-fluoronucleotides, and the remaining positions are 2'-methoxynucleotides; and the sense strand of the siRNA has a length of 21 nucleotides, wherein positions 9, 11, and 13 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 4, 12, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 9, 10, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 15 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 9, 10, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 4, 6, 8, 14, 16, 18 and 20 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 13 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 16 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 11 and 17 from the 5' end of the sense strand are 2'-fluoronucleotides, position 10 is a deoxynucleotide, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 10, 11 and 17 from the 5' end of the sense strand are 2'-fluoronucleotides, position 9 is a deoxynucleotide, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10 and 17 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 9, 10, 11 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11, 16 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11, 15 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 8, 9, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, position 10 is a deoxynucleotide, and the remaining positions are 2'-methoxynucleotides.

In some embodiments of the present invention, the antisense strand of the siRNA has a length of 23 nucleotides, wherein positions 2, 4, 6, 8, 10, 14, 16, 18 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 15 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 4, 6, 8, 10, 14, 16, 18 and 20 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 13 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 14 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 15 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 8, 14 and 16 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 19 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 14 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 15 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 18 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 19 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, and 11 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, and 10 from the 5' end of the sense strand are 2'-fluoronucleotides, position 11 is a deoxynucleotide, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 13 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 14 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 15 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 17 from the 5' end of the sense strand are 2'-fluoronucleotides, and the remaining positions are 2'-methoxynucleotides.

In some embodiments of the present invention, the antisense strand of the siRNA has a length of 23 nucleotides, wherein positions 2, 6, 14, 16 and 20 from the 5' end of the antisense strand are 2'-fluoronucleotides, and the remaining positions are 2'-methoxynucleotides; and the sense strand of the siRNA has a length of 21 nucleotides, wherein positions 7, 9, 10, 11 and 19 from the 5' end of the sense strand are 2'-fluoronucleotides, and the remaining positions are 2'-methoxynucleotides.

In some embodiments of the present invention, the siRNA of the present invention comprises a modified internucleoside linkage or a modified backbone. The modified internucleoside linkage or backbone includes, but is not limited to, phosphorothioate, 2'-O methoxyethyl (MOE), 2'-fluoro, an alkyl phosphate, a phosphorodithioate, an alkyl phosphorothioate, a phosphoramidate, a carbamate, a carbonate, a phosphotriester, an acetamidate, a carboxymethyl ester, and a combination thereof.

In some embodiments of the present invention, the modified nucleotide is a nucleotide of which the phosphate group is modified with a phosphorothioate group. That is, a non-bridging oxygen atom in the phosphodiester bond is substituted with a sulfur atom, thereby replacing the phosphodiester bond with a thiophosphodiester bond.

In some embodiments of the present invention, each of the 5' end and 3' end of the sense strand independently comprises 1 or 2 phosphorothioate linkages; and/or each of the 5' end and 3' end of the antisense strand independently comprises 1 or 2 phosphorothioate linkages.

In some embodiments of the present invention, a phosphorothioate linkage is present at, at least one of the following positions: between the nucleotides at positions 1 and 2 from the 5' end of the sense strand, between the nucleotides at positions 2 and 3 from the 5' end of the sense strand, between the nucleotides at positions 1 and 2 from the 3' end of the sense strand, between the nucleotides at positions 2 and 3 from the 3' end of the sense strand, between the nucleotides at positions 1 and 2 from the 3' end of the antisense strand, between the nucleotides at positions 2 and 3 from the 3' end of the antisense strand, between nucleotides at positions 1 and 2 from the 5' end of the antisense strand, and nucleotides at positions 2 and 3 from the 5' end of the antisense strand; in some embodiments of the present invention, phosphorothioate linkages are present at, at least four of the above positions; in some embodiments of the present invention, phosphorothioate linkages are present at, at least six of the above positions; in some embodiments of the present invention, phosphorothioate linkages are present at all eight of the above positions.

In some embodiments of the present invention, phosphorothioate linkages are present between the nucleotides at positions 1 and 2, and between the nucleotides at positions 2 and 3 from the 5' end of the sense strand.

In some embodiments of the present invention, phosphorothioate linkages are present between the nucleotides at positions 1 and 2, and between the nucleotides at positions 2 and 3 from the 5' end of the sense strand, and a phosphorothioate linkage is present between the nucleotides at positions 1 and 2 from the 3' end.

In some embodiments of the present invention, phosphorothioate linkages are present between the nucleotides at positions 1 and 2, and between the nucleotides at positions 2 and 3 from the 5' end of the sense strand, and phosphorothioate linkages are present between the nucleotides at positions 1 and 2, and between the nucleotides at positions 2 and 3 from the 3' end.

In some embodiments of the present invention, phosphorothioate linkages are present between the nucleotides at positions 1 and 2, and between the nucleotides at positions 2 and 3 from the 3' end of the antisense strand, and phosphorothioate linkages are present between the nucleotides at positions 1 and 2, and between the nucleotides at positions 2 and 3 from the 5' end.

In some embodiments of the present invention, phosphorothioate linkages are present between the nucleotides at positions 1 and 2 from the 5' end of the sense strand, between the nucleotides at positions 2 and 3 from the 5 ' end of the sense strand, between the nucleotides at positions 1 and 2 from the 3' end of the antisense strand, between the nucleotides at positions 2 and 3 from the 3' end of the antisense strand, between the nucleotides at positions 1 and 2 from the 5' end of the antisense strand, and between the nucleotides at positions 2 and 3 from the 5' end of the antisense strand.

In some embodiments of the present invention, phosphorothioate linkages are present between the nucleotides at positions 1 and 2 from the 5' end of the sense strand, between the nucleotides at positions 2 and 3 from the 5 ' end of the sense strand, between the nucleotides at positions 1 and 2 from the 3' end of the sense strand, between the nucleotides at positions 1 and 2 from the 3' end of the antisense strand, between the nucleotides at positions 2 and 3 from the 3' end of the antisense strand, between the nucleotides at positions 1 and 2 from the 5 ' end of the antisense strand, and between the nucleotides at positions 2 and 3 from the 5' end of the antisense strand.

In some embodiments of the present invention, phosphorothioate linkages are present between the nucleotides at positions 1 and 2 from the 5' end of the sense strand, between the nucleotides at positions 2 and 3 from the 5 ' end of the sense strand, between the nucleotides at positions 1 and 2 from the 3' end of the sense strand, between the nucleotides at positions 2 and 3 from the 3' end of the sense strand, between the nucleotides at positions 1 and 2 from the 3' end of the antisense strand, between the nucleotides at positions 2 and 3 from the 3' end of the antisense strand, between the nucleotides at positions 1 and 2 from the 5' end of the antisense strand, and between the nucleotides at positions 2 and 3 from the 5' end of the antisense strand.

In some embodiments of the present invention, the sense strand may include one or more capping residues or moieties, referred to as "capping residues". A "capping residue" is a non-nucleotide compound or other moiety that can be incorporated at one or more ends of the nucleotide sequence of an siRNA. In some embodiments of the present invention, the capping residue is present at the 5' end, the 3' end, or both the 5' end and the 3' end of the sense strand.

In some embodiments of the present invention, an inverted abasic residue (iab) is added as a capping residue. Reference can be made to F. Czauderna, Nucleic Acids Res., 2003, 31(11), 2705-16. In some embodiments of the present invention, the sense strand may comprise more than one inverted abasic deoxyribose moiety as a capping residue at the 5' end and/or the 3' end.

In some embodiments of the present invention, one or more inverted abasic residues (iab) are added to the 3' end of the sense strand. In some embodiments of the present invention, one or more inverted abasic residues (iab) are added to the 5' end of the sense strand. In some embodiments of the present invention, the sense strand of the siRNA comprises one or more inverted abasic residues at or near one or more ends.

The inverted abasic residues may be linked via phosphate, phosphorothioate, or other internucleoside linkages.

In some embodiments of the present invention, the first nucleotide from the 5' end of the antisense strand comprises a modification of (E)-vinylphosphonate and its analogs, such as a (E)-VP modification. In some embodiments of the present invention, the first nucleotide may optionally comprise other modifications, such as a 2'-modification (e.g., a 2'-methoxy modification).

In some embodiments of the present invention, the nucleotides at positions 2 to 8 from the 5' end of the antisense strand are nucleotides with a modification of glycerol (GNA) and its analogs.

In some embodiments, the antisense strand comprises the nucleotide sequence set forth in any of SEQ ID NOs: 249-302.

In some embodiments, the sense strand comprises the nucleotide sequence set forth in any of SEQ ID NOs: 303-379.

In some embodiments, the sequences of the sense strand and antisense strand of the siRNA are selected from sense strand and antisense strand sequences of any of duplex 125 to duplex 172, duplex 177, duplex 180 to duplex 185, and duplex 187 to duplex 237 provided in Table 2.

In some embodiments, the antisense strand comprises the nucleotide sequence set forth in any of SEQ ID NOs: 256, 260, 263, 264, 278, 279, 281, 285, 286, 287 and 288. In some embodiments, the sense strand comprises the nucleotide sequence set forth in any of SEQ ID NOs: 310, 313, 314, 315, 318, 317, 321, 324, 325, 326, 329, 330, 331, 333, 334, 349, 350, 351, 352, 356, 357, 358, 359, 360, 361, 362, 363, 364 and 365. In some embodiments, the sequences of the sense strand and antisense strand of the siRNA are selected from the sense strand and antisense strand sequences of any of duplex 143, duplex 144, duplex 145, duplex 146, duplex 147, duplex 148, duplex 153, duplex 157, duplex 158, duplex 167, duplex 168, duplex 169, duplex 170, duplex 171, duplex 172, duplex 198, duplex 200, duplex 202, duplex 203, duplex 208, duplex 209, duplex 210, duplex 211, duplex 212, duplex 213, duplex 214, duplex 215, duplex 216, and duplex 217 provided in Table 2.

In some embodiments, the antisense strand comprises the nucleotide sequence set forth in any of SEQ ID NOs: 256, 264, 285, 286, 287 and 288. In some embodiments, the sense strand comprises the nucleotide sequence set forth in any of SEQ ID NOs: 310, 314, 313, 318, 317, 330, 331, 334, 356, 357, 358, 359, 360, 361, 362, 363, 364 and 365. In some embodiments, the sequences of the sense strand and antisense strand of the siRNA are selected from the sense strand and antisense strand sequences of any of duplex 143, duplex 144, duplex 145, duplex 146, duplex 148, duplex 167, duplex 169, duplex 171, duplex 208, duplex 209, duplex 210, duplex 211, duplex 212, duplex 213, duplex 214, duplex 215, duplex 216 and duplex 217 provided in Table 2.

In another aspect, the present invention further provides a small interfering RNA (siRNA), wherein the siRNA comprises a sense strand and an antisense strand, wherein the antisense strand comprises a modification pattern selected from the following 1) to 12):
1) AS(5'-3'): mN*fN*mNmNmNfNmNfNfNmNmNfNmNfNmNfNmNmNmNmNmN*mN*mN (SEQ ID NO: 380);
2) AS(5'-3'): mN*fN*mNmNmNmNmNmNfNmNmNfNmNfNmNfNmNmNmNmNmN*mN*mN (SEQ ID NO: 381);
3) AS(5'-3'): mN*fN*mNfNmNmNmNmNmNmNmNfNmNfNmNfNmNmNmNmNmN*mN*mN (SEQ ID NO: 382);
4) AS(5'-3'): mN*fN*mNmNmNfNmNfNfNfNmNmNmNfNmNfNmNmNmNmNmN*mN*mN (SEQ ID NO: 383);
5) AS(5'-3'): mN*fN*mNfNmNfNmNfNmNmNmNmNmNfNmNfNmNfNmNfNmN*mN*mN (SEQ ID NO: 384);
6) AS(5'-3'): mN*fN*mNmNmNfNmNmNmNmNmNmNmNfNmNfNmNfNmNmNmN*mN*mN (SEQ ID NO: 385);
7) AS(5'-3'): mN*fN*mNmNmNmNmNfNfNmNmNmNmNfNmNfNmNmNmNmNmN*mN*mN (SEQ ID NO: 386);
8) AS(5'-3'):mN*fN*mNfNmNfNmNfNmNfNmNmNmNfNmNfNmNfNmNfNmN*mN*mN (SEQ ID NO: 387);
9) AS(5'-3'): (E)-VPmN*fN*mNmNmNmNmNfNfNmNmNmNmNfNmNfNmNmNmNmNmN*mN*mN (SEQ ID NO: 388);
10) AS(5'-3'): (E)-VPmN*fN*mNmNmNfNmNfNfNmNmNfNmNfNmNfNmNmNmNmNmN*mN*mN (SEQ ID NO: 389);
11) AS(5'-3'):(E)-VPmN*fN*mNmNmNmNmNmNfNmNmNfNmNfNmNfNmNmNmNmNmN*mN*mN (SEQ ID NO: 390); or,
12) AS(5'-3'):(E)-VPmN*fN*mNmNmNfNmNfNfNfNmNmNmNfNmNfNmNmNmNmNmN*mN*mN (SEQ ID NO: 391);

wherein, mN is a methoxy-modified nucleotide, fN is a fluorinated nucleotide, (E)-VP indicates that the nucleotide adjacent to its right is a (E)-vinylphosphonate-modified nucleotide, and * indicates a phosphorothioate linkage;
   and/or,
the sense strand comprises a modification pattern selected from the following 1) to 25):
   1) SS(5'-3'): mN*mN*mNmNmNmNfNmNfNmNfNmNmNmNmNmNmNmNmNmNmN (SEQ ID NO: 392);
   2) SS(5'-3'): mN*mN*mNmNmNmNmNmNfNmNfNmNfNmNmNmNmNmNmNmNmN (SEQ ID NO: 393);
   3) SS(5'-3'): mN*mN*mNmNmNmNfNmNfNfNfNmNmNmNfNmNmNmNmNmNmN (SEQ ID NO: 394);
   4) SS(5'-3'): mN*mN*mNmNmNmNfNmNfNfNdNmNmNmNmNmNmNmNmNmNmN (SEQ ID NO: 395);
   5) SS(5'-3'): mN*mN*mNmNmNmNfNmNfNfNfNmNfNmNmNmNmNmNmNmNmN (SEQ ID NO: 396);
   6) SS(5'-3'): mN*mN*mNmNmNmNfNmNfNfNfNmNmNmNmNfNmNmNmNmNmN (SEQ ID NO: 397);
   7) SS(5'-3'): mN*mN*mNmNmNmNfNmNfNfNfNmNmNmNmNmNfNmNmNmNmN (SEQ ID NO: 398);
   8) SS(5'-3'): mN*mN*mNmNmNmNfNmNfNdNfNmNmNmNmNmNfNmNmNmN*mN (SEQ ID NO: 399);
   9) SS(5'-3'): mN*mN*mNmNmNmNfNmNdNfNfNmNmNmNmNmNfNmNmNmN*mN (SEQ ID NO: 400);
   10) SS(5'-3'): mN*mN*mNmNmNmNfNmNfNfNdNmNmNmNmNmNfNmNmNmN*mN (SEQ ID NO: 401);
   11) SS(5'-3'): mN*mN*mNmNmNmNmNmNfNfNfNmNmNmNmNmNfNmNmNmN*mN (SEQ ID NO: 402);
   12) SS(5'-3'): mN*mN*mNmNmNmNfNmNfNfNfNmNmNmNmNmNfNmNmNmN*mN (SEQ ID NO: 403);
   13) SS(5'-3'): mN*mN*mNmNmNmNfNmNfNfNfNmNmNmNmNmNfNmNmN*mN*mN (SEQ ID NO: 404);
   14) SS(5'-3'): mN*mN*mNmNmNmNfNmNfNfNfNmNmNmNmNfNfNmNmNmN*mN (SEQ ID NO: 405);
   15) SS(5'-3'): mN*mN*mNmNmNmNfNmNfNfNfNmNmNmNfNmNfNmNmNmN*mN (SEQ ID NO: 406);
   16) SS(5'-3'):mN*mN*mNmNmNmNfNmNfNdNfNmNmNmNmNmNmNmNmNmN*mN (SEQ ID NO: 407);
   17) SS(5'-3'):mN*mN*mNmNmNmNfNmNfNmNfNmNmNmNmNmNmNmNmN*mN*mN (SEQ ID NO: 408);
   18) SS(5'-3'):mN*mN*mNmNmNmNmNmNfNmNfNmNfNmNmNmNmNmNmN*mN*mN (SEQ ID NO: 409);
   19) SS(5'-3'):mN*mN*mNmNmNmNfNmNfNfNdNmNmNmNmNmNmNmNmN*mN*mN (SEQ ID NO: 410);
   20) SS(5'-3'): mN*mN*mNmNmNmNfNmNfNdNfNmNmNmNmNmNmNmNmN*mN*mN (SEQ ID NO: 411);
   21) SS(5'-3'): mN*mN*mNmNmNmNfNmNfNmNfNmNmNmNmNmNmNmNmNmNmN*(iab) (SEQ ID NO: 412);
   22) SS(5'-3'): mN*mN*mNmNmNmNmNmNfNmNfNmNfNmNmNmNmNmNmNmNmN*(iab) (SEQ ID NO: 413);
   23) SS(5'-3'): mN*mN*mNmNmNmNfNmNfNfNdNmNmNmNmNmNmNmNmNmNmN*(iab) (SEQ ID NO: 414);
   24) SS(5'-3'): mN*mN*mNmNmNmNfNmNfNdNfNmNmNmNmNmNmNmNmNmNmN*(iab) (SEQ ID NO: 415); or,
   25) SS(5'-3'): mN*mN*mNmNmNmNfNmNfNfNfNmNmNmNmNmNfNmNmNmNmN*(iab) (SEQ ID NO: 416);
wherein mN is a methoxy-modified nucleotide, fN is a fluorinated nucleotide, dN is a deoxynucleotide, * is a phosphorothioate linkage, and (iab) is an inverted abasic residue.

In some embodiments, the siRNA comprises a modification pattern selected from the following list, wherein the first SEQ ID NO corresponds to the antisense strand and the second SEQ ID NO corresponds to the sense strand: SEQ ID NO: 380 and SEQ ID NO: 392; SEQ ID NO: 380 and SEQ ID NO: 393; SEQ ID NO: 381 and SEQ ID NO: 393; SEQ ID NO: 382 and SEQ ID NO: 392; SEQ ID NO: 383 and SEQ ID NO: 394; SEQ ID NO: 383 and SEQ ID NO: 395; SEQ ID NO: 384 and SEQ ID NO: 395; SEQ ID NO: 385 and SEQ ID NO: 396; SEQ ID NO: 386 and SEQ ID NO: 397; SEQ ID NO: 386 and SEQ ID NO: 398; SEQ ID NO: 386 and SEQ ID NO: 399; SEQ ID NO: 386 and SEQ ID NO: 400; SEQ ID NO: 386 and SEQ ID NO: 401; SEQ ID NO: 386 and SEQ ID NO: 402; SEQ ID NO: 386 and SEQ ID NO: 403; SEQ ID NO: 386 and SEQ ID NO: 404; SEQ ID NO: 386 and SEQ ID NO: 405; SEQ ID NO: 386 and SEQ ID NO: 406; SEQ ID NO: 387 and SEQ ID NO: 395; SEQ ID NO: 387 and SEQ ID NO: 394; SEQ ID NO: 388 and SEQ ID NO: 403; SEQ ID NO: 388 and SEQ ID NO: 399; SEQ ID NO: 388 and SEQ ID NO: 407; SEQ ID NO: 388 and SEQ ID NO: 406; SEQ ID NO: 388 and SEQ ID NO: 415; SEQ ID NO: 388 and SEQ ID NO: 416; SEQ ID NO: 388 and SEQ ID NO: 404; SEQ ID NO: 388 and SEQ ID NO: 402; SEQ ID NO: 388 and SEQ ID NO: 411; SEQ ID NO: 389 and SEQ ID NO: 408; SEQ ID NO: 389 and SEQ ID NO: 412; SEQ ID NO: 390 and SEQ ID NO: 409; SEQ ID NO: 390 and SEQ ID NO: 413; SEQ ID NO: 391 and SEQ ID NO: 410; SEQ ID NO: 391 and SEQ ID NO: 414.

### Delivery

The siRNAs of the present invention can be delivered or introduced by any means known in the art (e.g., delivered or introduced into a cell in vitro, or delivered or introduced into a patient in vivo). For example, for in vivo delivery, the siRNA can be injected into a tissue site, or administered systemically. The in vivo delivery can also be performed via the β-glucan delivery system. Methods for in vitro introduction into cells include those known in the art, such as electroporation and lipofection.

In some embodiments, the delivery method includes, but is not limited to, delivery via a virus (retrovirus, adenovirus, lentivirus, baculovirus, AAV); liposomes (Lipofectamine, cationic DOTAP, neutral DOPC); nanoparticles (cationic polymers, PEI); bacterial delivery (tkRNAi); chemical modification on the siRNA (LNAs) to increase stability; lipid nanoparticles (LNPs); neutral liposomes (NLs); polymeric nanoparticles (low molecular weight polymers or high molecular weight polymers); double-stranded RNA binding motifs (dsRBMs); and other delivery systems known in the art to be suitable for nucleic acid or oligonucleotide delivery.

### Conjugates

In one aspect, the present invention provides a conjugate comprising at least one siRNA of the present invention and a pharmaceutically acceptable targeting molecule. The conjugate of the present invention is obtained by conjugating the siRNA of the present invention with a pharmaceutically acceptable targeting molecule. The conjugate comprises a pharmaceutically acceptable targeting molecule and an optional linker. The siRNA may be non-covalently coupled to the targeting molecule, or covalently coupled to the targeting molecule.

The pharmaceutically acceptable targeting molecule can be a targeting molecule conventionally used in the field of siRNA administration, which typically enhances the pharmacokinetic or biodistribution properties of the siRNA linked thereto, and improves the cell-specific (or organ-specific) distribution and cell-specific (or organ-specific) uptake of the siRNA. Representative targeting molecules include, but are not limited to, compounds having affinity for cell surface molecules, cell receptor ligands, haptens, antibodies or antibody fragments, antibody mimetics, and the like. In some embodiments, the targeting molecules include but are not limited to one or more of the following targeting molecules or their derivatives: integrin family (integrins); lipophilic molecules, such as cholesterol, bile acid, vitamins (such as vitamin E), lipid molecules of different chain lengths; polymers, such as polyethylene glycol; polypeptides, such as membrane-permeable peptides; aptamers; antibodies; quantum dots; carbohydrates, such as lactose, polylactose, mannose, galactose, N-acetylgalactosamine (GalNAc); folate; or receptor ligands expressed by hepatic parenchymal cells, such as desialylated glycoproteins, desialylated sugar residues, lipoproteins (such as high-density lipoproteins, low-density lipoproteins, etc.), glucagon, neurotransmitters (such as adrenaline), growth factors, transferrin, etc.

The linker can be a linker conventionally used in the field of siRNA administration, including but not limited to one or more of the following linkers or their derivatives: amide linker moieties, amino linker moieties, carbonyl linker moieties, carbamate linker moieties, urea linker moieties, ether linker moieties, disulfide linker moieties, succinylamino linker moieties, etc.

In some embodiments, the targeting molecule can be linked directly or indirectly to the siRNA of the present invention via a linker/linking group. In some embodiments, the targeting molecule is linked to the siRNA via an unstable, cleavable or reversible bond or linker. In some embodiments, the targeting molecule is linked to at least one end of the sense strand and/or antisense strand of the siRNA. In some embodiments, the targeting molecule is linked to the 5' end and/or 3' end of the sense strand. In some embodiments, the targeting molecule is linked to the 5' end and/or 3' end of the antisense strand.

In some embodiments, the siRNA of the present invention is delivered to a target cell or tissue by means of the targeting molecule linked thereto. In some embodiments, the targeting molecule comprises a ligand of a cell receptor. In some embodiments, the targeting molecule has an affinity for an epithelial cell surface receptor.

In some embodiments, the targeting molecule is an integrin ligand.

In some embodiments, the targeting molecule comprises αvβ6 integrin ligand. Integrin αvβ6 is an epithelium-specific integrin that is highly upregulated in damaged lung epithelial cells. As used herein, the expression "αvβ6 integrin ligand" refers to a molecule that has an affinity for integrin αvβ6, which can facilitate the targeting and delivery of an siRNA linked thereto to cells expressing integrin αvβ6, such as lung epithelial cells. The αvβ6 integrin ligands are known to those skilled in the art, and can be found in, for example, international patent application WO2023070082A2. In some embodiments, two or more (e.g., 3) αvβ6 integrin ligands are linked to the siRNA of the present invention, either directly or via a linker/linking group. In some embodiments, the two or more (e.g., 3) αvβ6 integrin ligands are linked to the 5' end of the sense strand.

### Pharmaceutical composition

In one aspect, the present invention provides a pharmaceutical composition comprising at least one of the siRNAs of the present invention.

In some embodiments of the present invention, the pharmaceutical composition comprises one of the siRNAs described hereinabove.

In other embodiments of the present invention, the pharmaceutical composition comprises at least 2 (for example, but not limited to, 2, 3, 4, 5, 6, 7, 8, 9, 10 or more) of the siRNAs described hereinabove as active ingredients. Preferably, the at least 2 of the siRNAs, each targeting different target sequences in the MMP7 gene, can be expected to simultaneously act on different target sequences and produce a synergistic effect. Here, the expression "different target sequences" refers to target sequences that do not overlap, or target sequences that overlap by less than 5 consecutive nucleotides (e.g., the number of overlapping consecutive nucleotides is 4, 3, 2, 1, or 0). In this case, the at least 2 of the siRNAs as described above may be present in any different ratio. Preferably, the at least 2 of the siRNAs described above may be present in a molar ratio of 1:100 to 100:1 relative to each other; more preferably, the at least 2 of the siRNAs described above may be present in a molar ratio of 1:10 to 10:1, 1:5 to 5:1 or 1:2 to 2:1 relative to each other. In some embodiments of the present invention, the at least 2 of the siRNAs described above are present in the same molar ratio.

In other embodiments of the present invention, the pharmaceutical composition comprises at least one siRNA of the present invention and further comprises at least one siRNA targeting another target (e.g., a gene other than MMP7). In this case, the siRNA of the present invention and the siRNA targeting another target can be present in any different ratio, for example, in a molar ratio of 1:100 to 100:1, for example, in a molar ratio of 1:10 to 10:1, 1:5 to 5:1 or 1:2 to 2:1, for example, in the same molar ratio.

In some embodiments, the pharmaceutical composition comprises an effective amount of the siRNA. The expression "effective amount" refers to an amount of the siRNA effective to produce a desired pharmacological, therapeutic or preventive result. For example, if a given clinical treatment is considered effective when there is at least a 10% reduction in a measurable parameter associated with a disease or condition, the therapeutically effective amount of the drug for treating that disease or condition is the amount required to achieve at least a 10% reduction of that parameter. For example, a therapeutically effective amount of an siRNA targeting MMP7 can reduce the MMP7 mRNA level by at least 10%.

In some embodiments, in the pharmaceutical composition described in any of the above embodiments, the siRNA can be linked to a targeting molecule to form a conjugate. Thus, in some embodiments, the pharmaceutical composition comprises the conjugate of the present invention.

In some embodiments, the pharmaceutical composition further comprises a pharmaceutically acceptable carrier and/or excipient.

In some embodiments, the pharmaceutically acceptable carrier and/or excipient is a delivery carrier. The delivery carrier is a substance that improves the delivery of a nucleic acid or oligonucleotide to a cell or tissue. Such substances may be any delivery carrier known in the art to be suitable for nucleic acid or oligonucleotide delivery, including but not limited to: viruses (retroviruses, adenoviruses, lentiviruses, baculoviruses, AAV); liposomes (Lipofectamine, cationic DOTAP, neutral DOPC); nanoparticles (cationic polymers, PEI); bacteria (tkRNAi); lipid nanoparticles (LNPs); neutral liposomes (NLs); polymeric nanoparticles (low-molecular-weight polymers or high-molecular-weight polymers); double-stranded RNA-binding motifs (dsRBMs), etc.

In some embodiments, the siRNA can be encapsulated by the delivery carrier.

In some embodiments, the siRNA can be linked to the delivery carrier directly or indirectly via a linker/linking group. In some embodiments, the delivery carrier is linked to the siRNA via an unstable, cleavable or reversible bond or linker. In some embodiments, the delivery carrier is linked to at least one end of the sense strand and/or antisense strand of the siRNA. In some embodiments, the delivery carrier is linked to the 5' end and/or 3' end of the sense strand. In some embodiments, the delivery carrier is linked to the 5' end and/or 3' end of the antisense strand.

In some embodiments, the pharmaceutical composition of the present invention is formulated into a dosage form compatible with its intended route of administration, for example, it can be administered locally (such as direct injection or implantation), systemically, or subcutaneously, intravenously, intraperitoneally, or parenterally, including intracranial (such as intraventricular, intrameningeal, or intrathecal), intramuscularly, transdermally, or by airway (aerosol), nasal, oral, rectal, or topical (including buccal and sublingual) administration.

In some embodiments, the pharmaceutical composition is administered by inhalation, intranasal administration, intratracheal administration, or oropharyngeal inhalation. A formulation suitable for inhalation administration can be prepared by incorporating a required amount of the active ingredient in an appropriate solvent, followed by sterile filtration. Typically, the formulation for inhalation administration is a sterile solution at physiological pH and has low viscosity. A salt can be added to the formulation to balance the tonicity. In some cases, a surfactant or cosolvent can be added to increase the solubility of the active ingredient and improve aerosol properties. In some cases, an excipient can be added to control viscosity in order to ensure the size and distribution of the aerosolized droplets.

In other embodiments, the pharmaceutical composition can be administered by injection, for example, intravenously, intramuscularly, subcutaneously, intradermally, intraarticularly, intraocularly, intraperitoneally, or topically. The pharmaceutical compositions suitable for injectable use include sterile aqueous solutions or dispersions and sterile powders for the extemporaneous preparation of sterile injectable solutions or dispersions. For intravenous administration, suitable carriers include physiological saline, bacteriostatic water, or phosphate buffered saline (PBS) and the like. Pharmaceutical compositions should be stable under the conditions of manufacture and storage and should be protected against the contaminating action of microorganisms such as bacteria and fungi. For example, the sterile injection solutions can be prepared by incorporating a necessary dose of the active ingredient in an appropriate solvent, and optionally, incorporating other desired ingredients (including but not limited to pH adjusting agents, surfactants, adjuvants, ionic strength enhancers, diluents, osmotic pressure-maintaining reagents, absorption-delaying reagents, preservatives, or any combination thereof), followed by performing filtration sterilization. Additionally, the sterile injectable solutions can be prepared as sterile lyophilized powders (e.g., by vacuum drying or freeze drying) for ease of storage and use.

The siRNA of the present invention can be formulated in dosage unit form for ease of administration. A dosage unit form refers to a physically discrete unit suitable for use as a single dosage for a subject to be treated; each unit contains a predetermined quantity of the active ingredient calculated to produce the desired therapeutic effect in combination with the required pharmaceutical carrier.

In the present invention, dosage regimens may be adjusted to obtain the optimum desired response (e.g., a therapeutic or prophylactic response). For example, a single administration may be performed, multiple administrations may be administered over a period of time, or the dose may be proportionally reduced or increased as indicated by the exigencies of the therapeutic situation.

### Application

### Inhibition of the expression of MMP7

The siRNA of the present invention can be used to inhibit the expression of MMP7 in vitro and/or in vivo.

In one aspect, the present invention provides a method for inhibiting MMP7 expression in a cell, comprising: introducing the siRNA, conjugate or pharmaceutical composition of the present invention into the cell. In some embodiments, the method is performed in vitro. The siRNA of the present invention can be introduced by any nucleic acid delivery means known in the art, such as electroporation or lipofection.

The term "inhibition of the expression of MMP7" refers to at least partial suppression of the MMP7 gene expression, which can be manifested as a decrease in the level of detectable MMP7 mRNA. The degree of inhibition is typically expressed as: (mRNA in control cells)-(mRNA in treated cells)/(mRNA in control cells)*100%. Alternatively, the degree of inhibition may be given as a reduction in a parameter functionally associated with MMP7 gene expression, such as the level of protein encoded by the MMP7 gene. In principle, MMP7 gene silencing can be determined in any cell expressing MMP7 (expressing constitutively or by genetic engineering) by any suitable assay. Measurements can be made at multiple time points, before, during, and after administration of the siRNA, to determine the effect of the siRNA. The level or expression of MMP7 can be measured by evaluation of the mRNA (e.g., by Northern blot or PCR) or protein (e.g., Western blot or ELISA). For example, the effect of the siRNA on MMP7 expression can be determined by measuring the transcription rate of the MMP7 gene (e.g., by RT-PCR).

In some embodiments, the expression of the MMP7 gene is suppressed by at least about 10%, 15%, 20%, 25%, 30%, 35%, 40%, 45%, or 50% by administering the siRNA of the present invention. In some embodiments, the expression of the MMP7 gene is suppressed by at least about 60%, 70%, or 80% by administering the siRNA of the present invention. In some embodiments, the expression of the MMP7 gene is suppressed by at least about 85%, 90%, or 95% by administering the siRNA of the present invention. In some embodiments, the expression of the MMP7 gene is suppressed by at least about 96%, 97%, 98%, 99% or 100% by administering the siRNA of the present invention.

In some embodiments, the siRNA, the conjugate, or the pharmaceutical composition is used alone or in combination with an additional pharmaceutically active agent (e.g., an siRNA targeting a different target sequence in the MMP7 gene or an siRNA targeting another target).

In some embodiments, one of the siRNAs provided in the present invention is used. In other embodiments, at least 2 of the siRNAs provided by the present invention (for example, but not limited to, 2, 3, 4, 5, 6, 7, 8, 9, 10 or more) are used, preferably each of the at least 2 of the siRNAs targets a different target sequence in the MMP7 gene. In other embodiments, at least one of the siRNAs provided by the present invention and an siRNA targeting another target (e.g., a gene other than MMP7) are used.

### Treatment of MMP7-related diseases

The siRNA of the present invention can be used for the treatment of diseases or conditions which will benefit from the reduction of MMP7 levels or the inhibition of MMP7 expression.

In one aspect, the present invention provides a method for preventing and/or treating an MMP7-related pathological condition or disease in a subject, comprising administering an effective amount of the siRNA, the conjugate, or the pharmaceutical composition of the present invention to a subject in need thereof. The present invention further relates to use of the siRNA, the conjugate, or the pharmaceutical composition of the present invention in the manufacture of a medicament for treating and/or preventing an MMP7-related pathological condition or disease.

In some embodiments, the MMP7-related pathological condition or disease involves overexpression of MMP7. Overexpression of MMP7 means that MMP7 levels (e.g., MMP7 levels present in plasma or tissues of a subject, preferably in damaged tissues) are higher than normal MMP7 levels (e.g., corresponding levels in healthy controls).

In some embodiments, the MMP7-related pathological condition or disease will benefit from reduced levels or inhibition of MMP7 expression.

In some embodiments, the MMP7-related pathological condition or disease is a pulmonary inflammatory disease, such as pulmonary fibrosis (e.g., idiopathic pulmonary fibrosis, interstitial lung disease) or chronic obstructive pulmonary disease.

In some embodiments, the MMP7-related pathological condition or disease is a fibrotic disease, such as renal fibrosis or hepatic fibrosis.

In some embodiments, the MMP7-related pathological condition or disease is idiopathic pulmonary fibrosis.

In some embodiments, if MMP7 expression is elevated for a particular disease, treatment with the siRNA of the present invention may preferably reduce the level or expression of MMP7 to a level that is within the range considered normal for individuals without such a disorder.

In some embodiments, the subject is a mammal, such as a human.

In some embodiments, the siRNA, the conjugate, or the pharmaceutical composition is used alone, or in combination with an additional pharmaceutically active agent (e.g., an siRNA targeting a different target sequence in the MMP7 gene or an siRNA targeting another target), for example, administrated simultaneously or successively.

In some embodiments, one of the siRNAs provided in the present invention is used. In other embodiments, at least 2 of the siRNAs provided by the present invention (for example, but not limited to, 2, 3, 4, 5, 6, 7, 8, 9, 10 or more) are used, preferably each of the at least 2 of the siRNAs targets a different target sequence in the MMP7 gene. In other embodiments, at least one of the siRNAs provided by the present invention and an siRNA targeting another target (e.g., a gene other than MMP7) are used.

In some embodiments, the siRNA of the present invention is administered by inhalation, intranasal administration, intratracheal administration, or oropharyngeal inhalation. In some embodiments, the siRNA of the present invention is administered by inhalation, e.g., via an inhaler device such as a metered-dose inhaler, or a nebulizer such as a jet-nebulizer or a vibrating mesh nebulizer, or a soft mist inhaler, for the treatment of a pulmonary inflammatory disease.

### Definitions of terms

In the present invention, unless otherwise specified, the scientific and technical terms used herein have the meanings commonly understood by those skilled in the art. Moreover, in order to better understand the present invention, definitions and explanations of relevant terms are provided below.

Herein, unless otherwise specified, capital letters C, G, U, A, and T represent the base composition of nucleotides, including modified and unmodified nucleotides; lowercase letter m indicates that the nucleotide adjacent to the right of the identifier m is a 2'-methoxynucleotide; lowercase letter f indicates that the nucleotide adjacent to the right of the identifier f is a 2'-fluoronucleotide; lowercase letter d indicates that the nucleotide adjacent to the right of the identifier d is a 2'-deoxy nucleotide; the identifier * indicates that the two nucleotides adjacent to the left and right of the identifier * are connected by thiophosphate groups; (E)-VP indicates that the nucleotide adjacent to the right is a (E)-vinylphosphonate-modified nucleotide; and iab indicates an inverted abasic residue.

As used herein, the term "modified nucleotide" refers to a nucleotide that independently has a modified ribose moiety, a modified internucleoside linkage, or a modified base. Thus, the term "modified nucleotide" encompasses substitutions, additions, or removals (e.g., using functional groups or atoms) of internucleoside linkages, ribose moieties, or bases. Modifications suitable for use in the present invention include all types of modifications disclosed herein or known in the art. A "methoxy-modified nucleotide" refers to a nucleotide in which the hydroxyl group at the 2'-position of the ribose group of the nucleotide is replaced by a methoxy group. A "fluorinated nucleotide" refers to a nucleotide in which the hydroxyl group at the 2'-position of the ribose group of the nucleotide is replaced by a fluoro group. "Nucleotide analogs" refer to groups that replace nucleotides in nucleic acids but have a structure different from adenine ribonucleotide, guanine ribonucleotide, cytosine ribonucleotide, uracil ribonucleotide or thymine deoxyribonucleotide. The examples are isonucleotides, bridged nucleic acids (simply called BNA) or acyclic nucleotides.

Among them, the structure of the fluorinated nucleotide is as follows:

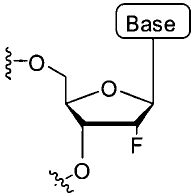

The structure of the methoxy modified nucleotide is as follows:

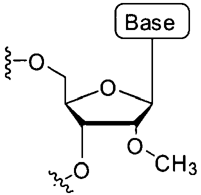

The structure of the locked nucleic acid modified nucleotide is as follows:

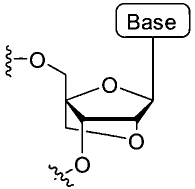

As used herein, the term "siRNA" refers to an RNA molecule that can sequence-specifically induce the RNAi phenomenon, is composed of a sense strand and an antisense strand, and has a partially or completely complementary double-stranded structure. In the siRNA involved in the present invention, the complementary double-stranded structure can have a length of 17-30 base pairs, e.g., 19, 20, 21, 22, 23, 24 or 25 base pairs. In some embodiments of the present invention, the siRNA can also contain modified nucleotides as needed, and the modified nucleotides will not significantly weaken or lose the function of the siRNA in inhibiting the MMP7 gene expression. Currently, there are many ways to modify siRNAs in the art, including, for example, backbone modification (such as phosphate group modification), ribose group modification, base modification and the like (Watts, J.K., G.F. Deleavey, and M.J. Damha, Chemically modified siRNA: tools and applications. Drug Discov Today, 2008. 13(19-20): p. 842-55).

The term "antisense strand" comprises a region that is substantially complementary to the target sequence. The term "sense strand" as used herein refers to the strand whose region is substantially complementary to the region of the antisense strand. The term "complementarity region" refers to a region on the antisense strand that is substantially complementary to MMP7 mRNA or a region on the sense strand that is substantially complementary to the antisense strand. When the complementarity region is not completely complementary to the target sequence or antisense strand, the mismatch may be in internal or terminal regions of the molecule. Typically, the most tolerated mismatches are in the terminal regions, for example, within 5, 4, 3, 2, or 1 nucleotide of the 5' end and/or 3' end.

Herein, unless otherwise specified, the term "complementary" refers to the ability of an oligonucleotide of a first sequence to hybridize with an oligonucleotide of a second sequence under certain conditions and form a double-stranded structure. The expression "at least partially complementary" means that the two sequences can be completely complementary, or have no more than 6, 5, 4, 3, 2 or 1 mismatched base pairs overall, while retaining the ability to hybridize under relevant conditions. Additionally, where two oligonucleotides are designed to form one or more single-stranded overhangs upon hybridization, such overhangs should not be considered mismatches for the purpose of determining complementarity. Herein, the "complementary" sequences may also include or be formed entirely from non-Watson-Crick base pairs and/or base pairs formed from non-natural and modified nucleotides, as long as the above requirements of hybridization capability are met. Such non-Watson-Crick base pairs include, but are not limited to, G:U wobble base pairing or Hoogstein base pairing. Correspondingly, herein, unless otherwise specified, "mismatch" means that the bases at corresponding positions in the siRNA duplex molecule are not paired in a complementary form.

Those skilled in the art will be able to determine the most suitable conditions for testing the complementarity of two sequences depending on the ultimate application of the hybridized nucleotides. Such conditions can, for example, be stringent conditions, such as 400 mM NaCl, 40 mM PIPES pH 6.4, 1 mM EDTA, 50°C or 70°C for 12-16 hours, followed by washing. Other conditions, such as physiologically relevant conditions that may be encountered in an organism, may also apply.

Herein, unless otherwise specified, "nucleotide sequence difference" means that the base type of the nucleotide at the same or corresponding position has changed compared to the original nucleotide sequence. For example, when one nucleotide base in the original nucleotide sequence is A, if the nucleotide base at the same or corresponding position is changed to U, C, G or dT, dC, dG, etc., it is considered that there is a difference in the nucleotide sequence at that position. It should be noted here that, when compared to the original nucleotide sequence, the nucleotides at the same or corresponding positions differ only in the presence or absence of modification or the type of modification, it is not considered that there is a difference in the nucleotide sequence at that position.

As used herein, unless otherwise specified, the term "pharmaceutically acceptable carrier and/or excipient" refers to a carrier, delivery carrier, diluent, auxiliary material and/or formed salt/ester/hydrate thereof, which are generally chemically or physically compatible with other ingredients constituting a pharmaceutical dosage form (such as the siRNA of the present invention) and physiologically compatible with the subject. The "pharmaceutically acceptable carrier and/or excipient" does not exert, or is not intended to exert, a therapeutic effect at an intended dosage. Such ingredients may serve the following functions: a) assisting in the processing of the drug delivery system during manufacturing; b) protecting, supporting, or enhancing the stability, bioavailability, or patient acceptability of the active ingredient; c) aiding in product identification; and/or d) enhancing any other properties of the active ingredient during storage and use, such as overall safety, efficacy, and delivery. For example, the siRNA of the present invention can be encapsulated by a delivery carrier. The "pharmaceutically acceptable carrier and/or excipient" includes, but is not limited to, viruses, liposomes, nanoparticles, bacteria, lipid nanoparticles (LNP), neutral liposomes (NL), polymeric nanoparticles, double-stranded RNA binding motifs (dsRBMs), pH adjusting agents, surfactants, adjuvants, ionic strength enhancers, diluents, osmotic pressure-maintaining reagents, absorption-delaying reagents, and preservatives. For example, viruses include, but are not limited to, retroviruses, adenoviruses, lentiviruses, baculoviruses, and AAV. Liposomes include, but are not limited to, Lipofectamine, cationic DOTAP, and neutral DOPC. Nanoparticles include, but are not limited to, cationic polymers, and PEI. Bacteria include, but are not limited to, tkRNAi. Polymeric nanoparticles include, but are not limited to, low molecular weight polymers or high molecular weight polymers. The pH adjusting agents include, but are not limited to, phosphate buffers. Surfactants include, but are not limited to, cationic, anionic or nonionic surfactants, such as Tween-80. Ionic strength enhancers include, but are not limited to, sodium chloride. Preservatives include, but are not limited to, various antibacterial reagents and antifungal reagents such as parabens, chlorobutanol, phenol, sorbic acid, etc. Reagents for maintaining osmotic pressure include, but are not limited to, sugars, NaCl, and analogs thereof. Reagents for delaying absorption include, but are not limited to, monostearates and gelatin. Diluents include, but are not limited to, water, aqueous buffers (e.g. buffered saline), alcohols, polyols (e.g. glycerol), etc.

Herein, unless otherwise specified, the term "inhibition" refers to the situation where the expression of the target gene is down-regulated due to siRNA-mediated degradation of the target gene mRNA. The "downregulation" refers to a decrease in target gene expression level by 5%, 10%, 15%, 20%, 25%, 30%, 35%, 40%, 45%, 50%, 55%, 60%, 65%, 70%, 75%, 80%, 85%, 90%, 95% or 99% or more, or even 100%, relative to the situation in the absence of siRNA treatment. The 100% decrease in target gene expression level means that there is no detectable level of the target gene expression.

Herein, the term "overhang" or "nucleotide overhang" refers to at least one unpaired nucleotide protruding from the siRNA duplex structure. For example, an overhang exists when the 3' end of one strand of an siRNA extends beyond the 5' end of the other strand (or vice versa). The siRNA can comprise an overhang of at least one nucleotide, or the overhang can comprise at least 2 nt, at least 3 nt, at least 4 nt, at least 5 nt, or more. The overhangs may comprise or be composed of nucleotides/nucleoside analogs, including deoxynucleotides/nucleosides. The overhang can be on the sense strand, the antisense strand, or any combination thereof. Nucleotides of the overhang may be present at the 5' end, 3' end, or both ends of the antisense or sense strand of the siRNA. Accordingly, the term "blunt end" refers to the absence of nucleotide overhangs.

As used herein, the term "prevention" refers to a method implemented for preventing or delaying the occurrence of a disease or disorder or symptom in a subject; and the term "treatment" refers to a method implemented for achieving beneficial or desired clinical outcomes. For purposes of the present invention, beneficial or desired clinical results include, but are not limited to, alleviation of symptoms, reduction in the extent of diseases, stabilization (i.e., no longer worsening) of disease states, delay or slowing of disease progression, amelioration or palliation of disease states, and relief of symptoms (regardless partial or complete), regardless detectable or undetectable. In addition, the "treatment" may also mean prolonging the survival time as compared to the expected survival time (in the absence of treatment).

Herein, the term "effective amount" refers to an amount sufficient to achieve or at least partially achieve desired effects. For example, an effective amount for preventing a disease refers to an amount sufficient to prevent, inhibit or delay the occurrence of the disease; and an effective amount for treating a disease refers to an amount sufficient to cure or at least partially inhibit the disease and complications thereof in a patient suffering from the disease. Determination of such effective amounts is completely within the capability of those skilled in the art. For example, amounts effective for therapeutic use will depend on the severity of the disease to be treated, the overall state of the immune system of the patient, the general conditions of the patient such as age, weight and sex, the manner of administration of a drug, other therapies administered simultaneously, and the like.

### Beneficial effects of the present invention

The siRNA of the present invention can effectively inhibit the MMP7 gene expression in vitro and/or in vivo, has good stability, and negligible cytotoxicity and immunostimulation, and can significantly reduce MMP7 protein levels at the animal level. Therefore, the siRNA of the present invention can be used for treating diseases or conditions benefiting from the reduction of MMP7 levels or the inhibition of MMP7 expression, and has an important clinical value in the treatment of pulmonary inflammation or fibrotic diseases.

The embodiments of the present invention will be described in detail below in conjunction with examples, but those skilled in the art will understand that the following examples are only used for illustrating the present invention rather than limiting the scope of the present invention. Various purposes and advantages of the present invention will become implementable to those skilled in the art according to the following detailed descriptions of the preferred embodiments.

### Sequence information

**Table 1: Duplex 1-Duplex 124**

| Duplex number | 5'-sense-3' (sense strand sequence) | SEQ ID NO | 5'-antisense-3' (antisense strand sequence) | SEQ ID NO | Positions in the target gene |
|---|---|---|---|---|---|
| Duplex 1 | GCGGGAGGCAUGAGU GAGCUA | 125 | UAGCUCACUCAUGCCUCCC GCCU | 1 | 117 |
| Duplex 2 | UCAGGACUAUCUCAA GAGAUU | 126 | AAUCUCUUGAGAUAGUCCU GAGC | 2 | 152 |
| Duplex 3 | CAGGACUAUCUCAAG AGAUUU | 127 | AAAUCUCUUGAGAUAGUCC UGAG | 3 | 153 |
| Duplex 4 | AGGACUAUCUCAAGA GAUUUU | 128 | AAAAUCUCUUGAGAUAGUC CUGA | 4 | 154 |
| Duplex 5 | GACUAUCUCAAGAGA UUUUAU | 129 | AUAAAAUCUCUUGAGAUAG UCCU | 5 | 156 |
| Duplex 6 | CUAUCUCAAGAGAUU UUAUCU | 130 | AGAUAAAUCUCUUGAGAU AGUC | 6 | 158 |
| Duplex 7 | UAUCUCUAUGACUCA GAAACA | 131 | UGUUUCUGAGUCAUAGAGA UAAA | 7 | 174 |
| Duplex 8 | UCUCUAUGACUCAGA AACAAA | 132 | UUUGUUUCUGAGUCAUAGA GAUA | 8 | 176 |
| Duplex 9 | CUCUAUGACUCAGAA ACAAAA | 133 | UUUUGUUUCUGAGUCAUAG AGAU | 9 | 177 |
| Duplex 10 | CAACAGUUUAGAAGC CAAACU | 134 | AGUUUGGCUUCUAAACUGU UGGC | 10 | 203 |
| Duplex 11 | ACAGUUUAGAAGCCA AACUCA | 135 | UGAGUUUGGCUUCUAAACU GUUG | 11 | 205 |
| Duplex 12 | CAGUUUAGAAGCCAA ACUCAA | 136 | UUGAGUUUGGCUUCUAAAC UGUU | 12 | 206 |
| Duplex 13 | AAGCCAAACUCAAGG AGAUGU | 137 | ACAUCUCCUUGAGUUUGGC UUCU | 13 | 214 |
| Duplex 14 | UACCUAUAACUGGAA UGUUAA | 138 | UUAACAUUCCAGUUAUAGG UAGG | 14 | 250 |
| Duplex 15 | CUAUAACUGGAAUGU UAAACU | 139 | AGUUUAACAUUCCAGUUAU AGGU | 15 | 253 |
| Duplex 16 | AUAAUGCAGAAGCCC AGAUGU | 140 | ACAUCUGGGCUUCUGCAUU AUUU | 16 | 288 |
| Duplex 17 | CAGAAGCCCAGAUGU GGAGUA | 141 | UACUCCACAUCUGGGCUUC UGCA | 17 | 294 |
| Duplex 18 | GCCCAGAUGUGGAGU GCCAGA | 142 | UCUGGCACUCCACAUCUGG GCUU | 18 | 299 |
| Duplex 19 | CCAGAUGUGGAGUGC CAGAUA | 143 | UAUCUGGCACUCCACAUCU GGGC | 19 | 301 |
| Duplex 20 | AGAAUACUCACUAUU UCCAAA | 144 | UUUGGAAAUAGUGAGUAU UCUGC | 20 | 326 |
| Duplex 21 | GAAUACUCACUAUUU CCAAAU | 145 | AUUUGGAAAUAGUGAGUA UUCUG | 21 | 327 |
| Duplex 22 | AAUACUCACUAUUUC CAAAUA | 146 | UAUUUGGAAAUAGUGAGU AUUCU | 22 | 328 |
| Duplex 23 | AUACUCACUAUUUCC AAAUAA | 147 | UUAUUUGGAAAUAGUGAG UAUUC | 23 | 329 |
| Duplex 24 | UACUCACUAUUUCCA AAUAGU | 148 | ACUAUUUGGAAAUAGUGA GUAUU | 24 | 330 |
| Duplex 25 | CUAUUUCCAAAUAGC CCAAAA | 149 | UUUUGGGCUAUUUGGAAA UAGUG | 25 | 336 |
| Duplex 26 | AUUUCCAAAUAGCCC AAAAUA | 150 | UAUUUUGGGCUAUUUGGA AAUAG | 26 | 338 |
| Duplex 27 | UUCCAAAUAGCCCAA AAUGGA | 151 | UCCAUUUUGGGCUAUUUGG AAAU | 27 | 340 |
| Duplex 28 | CAAAUAGCCCAAAAU GGACUU | 152 | AAGUCCAUUUUGGGCUAUU UGGA | 28 | 343 |
| Duplex 29 | AGCCCAAAAUGGACU UCCAAA | 153 | UUUGGAAGUCCAUUUUGGG CUAU | 29 | 348 |
| Duplex 30 | GCCCAAAAUGGACUU CCAAAA | 154 | UUUUGGAAGUCCAUUUGG GCUA | 30 | 349 |
| Duplex 31 | CCCAAAAUGGACUUC CAAAGU | 155 | ACUUUGGAAGUCCAUUUUG GGCU | 31 | 350 |
| Duplex 32 | UCGUAUCAUAUACUC GAGACU | 156 | AGUCUCGAGUAUAUGAUAC GAUC | 32 | 385 |
| Duplex 33 | GUAUCAUAUACUCGA GACUUA | 157 | UAAGUCUCGAGUAUAUGAU ACGA | 33 | 387 |
| Duplex 34 | AUCAUAUACUCGAGA CUUACU | 158 | AGUAAGUCUCGAGUAUAUG AUAC | 34 | 389 |
| Duplex 35 | AUAUACUCGAGACUU ACCGCA | 159 | UGCGGUAAGUCUCGAGUAU AUGA | 35 | 392 |
| Duplex 36 | UAUACUCGAGACUUA CCGCAU | 160 | AUGCGGUAAGUCUCGAGUA UAUG | 36 | 393 |
| Duplex 37 | GAUCGAUUAGUGUCA AAGGCU | 161 | AGCCUUUGACACUAAUCGA UCCA | 37 | 423 |
| Duplex 38 | AUCGAUUAGUGUCAA AGGCUU | 162 | AAGCCUUUGACACUAAUCG AUCC | 38 | 424 |
| Duplex 39 | UCGAUUAGUGUCAAA GGCUUU | 163 | AAAGCCUUUGACACUAAUC GAUC | 39 | 425 |
| Duplex 40 | CGAUUAGUGUCAAAG GCUUUA | 164 | UAAAGCCUUUGACACUAAU CGAU | 40 | 426 |
| Duplex 41 | GAUUAGUGUCAAAGG CUUUAA | 165 | UUAAAGCCUUUGACACUAA UCGA | 41 | 427 |
| Duplex 42 | AUUAGUGUCAAAGGC UUUAAA | 166 | UUUAAAGCCUUUGACACUA AUCG | 42 | 428 |
| Duplex 43 | UUAGUGUCAAAGGCU UUAAAU | 167 | AUUUAAAGCCUUUGACACU AAUC | 43 | 429 |
| Duplex 44 | UAGUGUCAAGGCUU UAAACA | 168 | UGUUUAAAGCCUUUGACAC UAAU | 44 | 430 |
| Duplex 45 | AGUGUCAAAGGCUUU AAACAU | 169 | AUGUUUAAAGCCUUUGACA CUAA | 45 | 431 |
| Duplex 46 | GUGUCAAAGGCUUUA AACAUA | 170 | UAUGUUUAAAGCCUUUGAC ACUA | 46 | 432 |
| Duplex 47 | UGUCAAAGGCUUUAA ACAUGU | 171 | ACAUGUUUAAAGCCUUUGA CACU | 47 | 433 |
| Duplex 48 | GUCAAAGGCUUUAAA CAUGUA | 172 | UACAUGUUUAAAGCCUUUG ACAC | 48 | 434 |
| Duplex 49 | GCUUUAAACAUGUGG GGCAAA | 173 | UUUGCCCCACAUGUUUAAA GCCU | 49 | 441 |
| Duplex 50 | AGCUAUGCGACUCAC CGUGCU | 174 | AGCACGGUGAGUCGCAUAG CUGC | 50 | 44 |
| Duplex 51 | CCUGCAUUUCAGGAA AGUUGU | 175 | ACAACUUUCCUGAAAUGCA GGGG | 51 | 470 |
| Duplex 52 | CUGCAUUUCAGGAAA GUUGUA | 176 | UACAACUUUCCUGAAAUGC AGGG | 52 | 471 |
| Duplex 53 | UGCAUUUCAGGAAAG UUGUAU | 177 | AUACAACUUUCCUGAAAUG CAGG | 53 | 472 |
| Duplex 54 | GCAUUUCAGGAAAGU UGUAUA | 178 | UAUACAACUUUCCUGAAAU GCAG | 54 | 473 |
| Duplex 55 | CAGGAAAGUUGUAUG GGGAAU | 179 | AUUCCCCAUACAACUUUCC UGAA | 55 | 479 |
| Duplex 56 | AGGAAAGUUGUAUG GGGAACU | 180 | AGUUCCCCAUACAACUUUC CUGA | 56 | 480 |
| Duplex 57 | UUGUAUGGGAACUG CUGACA | 181 | UGUCAGCAGUUCCCCAUAC AACU | 57 | 487 |
| Duplex 58 | GGGGAACUGCUGACA UCAUGA | 182 | UCAUGAUGUCAGCAGUUCC CCAU | 58 | 493 |
| Duplex 59 | GGGAACUGCUGACAU CAUGAU | 183 | AUCAUGAUGUCAGCAGUUC CCCA | 59 | 494 |
| Duplex 60 | GGAACUGCUGACAUC AUGAUU | 184 | AAUCAUGAUGUCAGCAGUU CCCC | 60 | 495 |
| Duplex 61 | AACUGCUGACAUCAU GAUUGA | 185 | UCAAUCAUGAUGUCAGCAG UUCC | 61 | 497 |
| Duplex 62 | CUGCUGACAUCAUGA UUGGCU | 186 | AGCCAAUCAUGAUGUCAGC AGUU | 62 | 499 |
| Duplex 63 | GCUGACAUCAUGAUU GGCUUU | 187 | AAAGCCAAUCAUGAUGUCA GCAG | 63 | 501 |
| Duplex 64 | CUGACAUCAUGAUUG GCUUUA | 188 | UAAAGCCAAUCAUGAUGUC AGCA | 64 | 502 |
| Duplex 65 | GACAUCAUGAUUGGC UUUGCA | 189 | UGCAAAGCCAAUCAUGAUG UCAG | 65 | 504 |
| Duplex 66 | CACGCUGGCUCAUGC CUUUGU | 190 | ACAAAGGCAUGAGCCAGCG UGUU | 66 | 569 |
| Duplex 67 | UCGGAGGAGAUGCUC ACUUCA | 191 | UGAAGUGAGCAUCUCCUCC GAGA | 67 | 604 |
| Duplex 68 | AUGCUCACUUCGAUG AGGAUA | 192 | UAUCCUCAUCGAAGUGAGC AUCU | 68 | 613 |
| Duplex 69 | UGCUCACUUCGAUGA GGAUGA | 193 | UCAUCCUCAUCGAAGUGAG CAUC | 69 | 614 |
| Duplex 70 | GCUCACUUCGAUGAG GAUGAA | 194 | UUCAUCCUCAUCGAAGUGA GCAU | 70 | 615 |
| Duplex 71 | CUCACUUCGAUGAGG AUGAAU | 195 | AUUCAUCCUCAUCGAAGUG AGCA | 71 | 616 |
| Duplex 72 | CACUUCGAUGAGGAU GAACGU | 196 | ACGUUCAUCCUCAUCGAAG UGAG | 72 | 618 |
| Duplex 73 | UUCGAUGAGGAUGAA CGCUGA | 197 | UCAGCGUUCAUCCUCAUCG AAGU | 73 | 621 |
| Duplex 74 | UCGAUGAGGAUGAAC GCUGGA | 198 | UCCAGCGUUCAUCCUCAUC GAAG | 74 | 622 |
| Duplex 75 | AUGAGGAUGAACGCU GGACGA | 199 | UCGUCCAGCGUUCAUCCUC AUCG | 75 | 625 |
| Duplex 76 | AUGAACGCUGGACGG AUGGUA | 200 | UACCAUCCGUCCAGCGUUC AUCC | 76 | 631 |
| Duplex 77 | AACGCUGGACGGAUG GUAGCA | 201 | UGCUACCAUCCGUCCAGCG UUCA | 77 | 634 |
| Duplex 78 | CUGGACGGAUGGUAG CAGUCU | 202 | AGACUGCUACCAUCCGUCC AGCG | 78 | 638 |
| Duplex 79 | UGGACGGAUGGUAGC AGUCUA | 203 | UAGACUGCUACCAUCCGUC CAGC | 79 | 639 |
| Duplex 80 | GGACGGAUGGUAGCA GUCUAA | 204 | UUAGACUGCUACCAUCCGU CCAG | 80 | 640 |
| Duplex 81 | CAUGAACUUGGCCAU UCUUUA | 205 | UAAAGAAUGGCCAAGUUCA UGAG | 81 | 687 |
| Duplex 82 | AUGAACUUGGCCAUU CUUUGA | 206 | UCAAAGAAUGGCCAAGUUC AUGA | 82 | 688 |
| Duplex 83 | ACUUGGCCAUUCUUU GGGUAU | 207 | AUACCCAAAGAAUGGCCAA GUUC | 83 | 692 |
| Duplex 84 | UUGGCCAUUCUUUGG GUAUGA | 208 | UCAUACCCAAAGAAUGGCC AAGU | 84 | 694 |
| Duplex 85 | GGCCAUUCUUUGGGU AUGGGA | 209 | UCCCAUACCCAAAGAAUGG CCAA | 85 | 696 |
| Duplex 86 | GCCAUUCUUGGUA UGGAU | 210 | AUCCCAUACCCAAAGAAUG GCCA | 86 | 697 |
| Duplex 87 | CCAUUCUUUGGGUAU GGGACA | 211 | UGUCCCAUACCCAAAGAAU GGCC | 87 | 698 |
| Duplex 88 | CAUUCUUUGGGUAUG GGACAU | 212 | AUGUCCCAUACCCAAAGAA UGGC | 88 | 699 |
| Duplex 89 | AUUCUUUGGGUAUGG GACAUU | 213 | AAUGUCCCAUACCCAAAGA AUGG | 89 | 700 |
| Duplex 90 | UUCUUUGGGUAUGGG ACAUUU | 214 | AAAUGUCCCAUACCCAAAG AAUG | 90 | 701 |
| Duplex 91 | GGAGAUCCCCAAAAU UUUAAA | 215 | UUUAAAAUUUUGGGAUC UCCAU | 91 | 762 |
| Duplex 92 | GAGAUCCCCAAAAUU UUAAAU | 216 | AUUUAAAAUUUUGGGAU CUCCA | 92 | 763 |
| Duplex 93 | AGAUCCCCAAAAUUU UAAACU | 217 | AGUUUAAAAUUUUGGGGA UCUCC | 93 | 764 |
| Duplex 94 | GAUCCCCAAAAUUUU AAACUU | 218 | AAGUUUAAAAUUUUGGGG AUCUC | 94 | 765 |
| Duplex 95 | AUCCCCAAAAUUUUA AACUUU | 219 | AAAGUUUAAAUUUUGGG GAUCU | 95 | 766 |
| Duplex 96 | UCCCCAAAAUUUUAA ACUUUU | 220 | AAAAGUUUAAAAUUUUGG GGAUC | 96 | 767 |
| Duplex 97 | CCCCAAAAUUUUAAA CUUUCU | 221 | AGAAAGUUUAAAUUUUG GGGAU | 97 | 768 |
| Duplex 98 | AUUUUAAACUUUCCC AGGAUA | 222 | UAUCCUGGGAAAGUUAAA AUUU | 98 | 775 |
| Duplex 99 | UUUUAAACUUUCCCA GGAUGA | 223 | UCAUCCUGGGAAAGUUUAA AAUU | 99 | 776 |
| Duplex 100 | UUUAAACUUUCCCAG GAUGAU | 224 | AUCAUCCUGGGAAAGUUUA AAAU | 100 | 777 |
| Duplex 101 | AACUUUCCCAGGAUG AUAUUA | 225 | UAAUAUCAUCCUGGGAAAG UUUA | 101 | 781 |
| Duplex 102 | ACUUUCCCAGGAUGA UAUUAA | 226 | UUAAUAUCAUCCUGGAAA GUUU | 102 | 782 |
| Duplex 103 | CUUUCCCAGGAUGAU AUUAAA | 227 | UUUAAUAUCAUCCUGGGAA AGUU | 103 | 783 |
| Duplex 104 | AGGAUGAUAUUAAA GGCAUUU | 228 | AAAUGCCUUUAAUAUCAUC CUGG | 104 | 790 |
| Duplex 105 | GAUAUUAAAGGCAUU CAGAAA | 229 | UUUCUGAAUGCCUUUAAUA UCAU | 105 | 795 |
| Duplex 106 | AUAUUAAAGGCAUUC AGAAAU | 230 | AUUUCUGAAUGCCUUUAAU AUCA | 106 | 796 |
| Duplex 107 | UAUUAAAGGCAUUCA GAAACU | 231 | AGUUUCUGAAUGCCUUUAA UAUC | 107 | 797 |
| Duplex 108 | GCAUUCAGAAACUAU AUGGAA | 232 | UUCCAUAUAGUUUCUGAAU GCCU | 108 | 805 |
| Duplex 109 | CAUUCAGAAACUAUA UGGAAA | 233 | UUUCCAUAUAGUUUCUGAA UGCC | 109 | 806 |
| Duplex 110 | UUCAGAAACUAUAUG GAAAGA | 234 | UCUUUCCAUAUAGUUUCUG AAUG | 110 | 808 |
| Duplex 111 | CAGAAACUAUAUGGA AAGAGA | 235 | UCUCUUUCCAUAUAGUUUC UGAA | 111 | 810 |
| Duplex 112 | CUAUAUGGAAAGAGA AGUAAU | 236 | AUUACUUCUCUUUCCAUAU AGUU | 112 | 816 |
| Duplex 113 | UAUGGAAAGAGAAG UAAUUCA | 237 | UGAAUUACUUCUCUUUCCA UAUA | 113 | 819 |
| Duplex 114 | UGGAAAGAGAAGUA AUUCAAA | 238 | UUUGAAUUACUUCUCUUUC CAUA | 114 | 821 |
| Duplex 115 | GGAAAGAGAAGUAA UUCAAGA | 239 | UCUUGAAUUACUUCUCUUU CCAU | 115 | 822 |
| Duplex 116 | GAAAGAGAAGUAAU UCAAGAA | 240 | UUCUUGAAUUACUUCUCUU UCCA | 116 | 823 |
| Duplex 117 | AAAGAGAAGUAAUUC AAGAAA | 241 | UUUCUUGAAUUACUUCUCU UUCC | 117 | 824 |
| Duplex 118 | AAGAGAAGUAAUUCA AGAAAA | 242 | UUUUCUUGAAUUACUUCUC UUUC | 118 | 825 |
| Duplex 119 | AGAGAAGUAAUUCAA GAAAGA | 243 | UCUUUCUUGAAUUACUUCU CUUU | 119 | 826 |
| Duplex 120 | GAGAAGUAAUUCAAG AAAGAA | 244 | UUCUUUCUUGAAUUACUUC UCUU | 120 | 827 |
| Duplex 121 | AGAAGUAAUUCAAGA AAGAAA | 245 | UUUCUUUCUUGAAUUACUU CUCU | 121 | 828 |
| Duplex 122 | GAAGUAAUUCAAGAA AGAAAU | 246 | AUUUCUUUCUUGAAUUACU UCUC | 122 | 829 |
| Duplex 123 | AAGUAAUUCAAGAAA GAAAUA | 247 | UAUUUCUUUCUUGAAUUAC UUCU | 123 | 830 |
| Duplex 124 | GUAAUUCAAGAAAGA AAUAGA | 248 | UCUAUUUCUUUCUUGAAUU ACUU | 124 | 832 |
| Here, C, G, U, and A represent the base composition of nucleotides. | | | | | |

**Table 2. Modified duplex 125-modified duplex 237**

| Duplex number | SEQ ID NO: | SS strand (5'-3') | SEQ ID NO: | AS strand (5'-3') | Positions in the target gene |
|---|---|---|---|---|---|
| Duplex 125 | 303 | mU*mC*mAmGmGmAfCm UfAmUfCmUmCmAmAmG mAmGmAmUmU | 249 | mA*fA*mUmCmUfCmUfUfG mAmGfAmUfAmGfUmCmCm UmGmA*mG*mC | 152 |
| Duplex 126 | 304 | mU*mC*mAmGmGmAmCm UfAmUfCmUfCmAmAmGm AmGmAmUmU | 249 | mA*fA*mUmCmUfCmUfUfG mAmGfAmUfAmGfUmCmCm UmGmA*mG*mC | |
| Duplex 127 | 304 | mU*mC*mAmGmGmAmCm UfAmUfCmUfCmAmAmGm AmGmAmUmU | 250 | mA*fA*mUmCmUmCmUmUf GmAmGfAmUfAmGfUmCmC mUmGmA*mG*mC | |
| Duplex 128 | 303 | mU*mC*mAmGmGmAfCm UfAmUfCmUmCmAmAmG mAmGmAmUmU | 251 | mA*fA*mUfCmUmCmUmUm GmAmGfAmUfAmGfUmCmC mUmGmA*mG*mC | |
| Duplex 129 | 305 | mU*mC*mAmGmGmAfCm UfAfUfCmUmCmAfAmGmA mGmAmUmU | 252 | mA*fA*mUmCmUfCmUfUfGf AmGmAmUfAmGfUmCmCm UmGmA*mG*mC | |
| Duplex 130 | 306 | mU*mC*mAmGmGmAfCm UfAfUdCmUmCmAmAmGm AmGmAmUmU | 252 | mA*fA*mUmCmUfCmUfUfGf AmGmAmUfAmGfUmCmCm UmGmA*mG*mC | |
| Duplex 131 | 306 | mU*mC*mAmGmGmAfCm UfAfUdCmUmCmAmAmGm AmGmAmUmU | 253 | mA*fA*mUfCmUfCmUfUmG mAmGmAmUfAmGfUmCfCm UfGmA*mG*mC | |
| Duplex 132 | 307 | mU*mC*mAmGmGmAfCm UfAfUfCmUfCmAmAmGmA mGmAmUmU | 254 | mA*fA*mUmCmUfCmUmUm GmAmGmAmUfAmGfUmCfC mUmGmA*mG*mC | |
| Duplex 133 | 308 | mU*mC*mAmGmGmAfCm UfAfUfCmUmCmAmAfGmA mGmAmUmU | 255 | mA*fA*mUmCmUmCmUfUfG mAmGmAmUfAmGfUmCmC mUmGmA*mG*mC | |
| Duplex 134 | 309 | mU*mC*mAmGmGmAfCm UfAfUfCmUmCmAmAmGfA mGmAmUmU | 255 | mA*fA*mUmCmUmCmUfUfG mAmGmAmUfAmGfUmCmC mUmGmA*mG*mC | |
| Duplex 135 | 310 | mU*mC*mAmGmGmAfCm UfAdTfCmUmCmAmAmGfA mGmAmU*mU | 255 | mA*fA*mUmCmUmCmUfUfG mAmGmAmUfAmGfUmCmC mUmGmA*mG*mC | |
| Duplex 136 | 311 | mU*mC*mAmGmGmAfCm UdAfUfCmUmCmAmAmGf AmGmAmU*mU | 255 | mA*fA*mUmCmUmCmUfUfG mAmGmAmUfAmGfUmCmC mUmGmA*mG*mC | |
| Duplex 137 | 312 | mU*mC*mAmGmGmAfCm UfAfUdCmUmCmAmAmGf AmGmAmU*mU | 255 | mA*fA*mUmCmUmCmUfUfG mAmGmAmUfAmGfUmCmC mUmGmA*mG*mC | |
| Duplex 138 | 313 | mU*mC*mAmGmGmAmCm UfAfUfCmUmCmAmAmGfA mGmAmU*mU | 255 | mA*fA*mUmCmUmCmUfUfG mAmGmAmUfAmGfUmCmC mUmGmA*mG*mC | |
| Duplex 139 | 314 | mU*mC*mAmGmGmAfCm UfAfUfCmUmCmAmAmGfA mGmAmU*mU | 255 | mA*fA*mUmCmUmCmUfUfG mAmGmAmUfAmGfUmCmC mUmGmA*mG*mC | |
| Duplex 140 | 315 | mU*mC*mAmGmGmAfCm UfAfUfCmUmCmAmAmGfA mGmA*mU*mU | 255 | mA*fA*mUmCmUmCmUfUfG mAmGmAmUfAmGfUmCmC mUmGmA*mG*mC | |
| Duplex 141 | 316 | mU*mC*mAmGmGmAfCm UfAfUfCmUmCmAmAfGfA mGmAmU*mU | 255 | mA*fA*mUmCmUmCmUfUfG mAmGmAmUfAmGfUmCmC mUmGmA*mG*mC | |
| Duplex 142 | 317 | mU*mC*mAmGmGmAfCm UfAfUfCmUmCmAfAmGfA mGmAmU*mU | 255 | mA*fA*mUmCmUmCmUfUfG mAmGmAmUfAmGfUmCmC mUmGmA*mG*mC | |
| Duplex 143 | 314 | mU*mC*mAmGmGmAfCm UfAfUfCmUmCmAmAmGfA mGmAmU*mU | 256 | (E)- VPmA*fA*mUmCmUmCmUf UfGmAmGmAmUfAmGfUmC mCmUmGmA*mG*mC | |
| Duplex 144 | 310 | mU*mC*mAmGmGmAfCm UfAdTfCmUmCmAmAmGfA mGmAmU*mU | 256 | (E)- VPmA*fA*mUmCmUmCmUf UfGmAmGmAmUfAmGfUmC mCmUmGmA*mG*mC | |
| Duplex 145 | 318 | mU*mC*mAmGmGmAfCm UfAdTfCmUmCmAmAmGm AmGmAmU*mU | 256 | (E)- VPmA*fA*mUmCmUmCmUf UfGmAmGmAmUfAmGfUmC mCmUmGmA*mG*mC | |
| Duplex 146 | 317 | mU*mC*mAmGmGmAfCm UfAfUfCmUmCmAfAmGfA mGmAmU*mU | 256 | (E)- VPmA*fA*mUmCmUmCmUf UfGmAmGmAmUfAmGfUmC mCmUmGmA*mG*mC | |
| Duplex 147 | 315 | mU*mC*mAmGmGmAfCm UfAfUfCmUmCmAmAmGfA mGmA*mU*mU | 256 | (E)- VPmA*fA*mUmCmUmCmUf UfGmAmGmAmUfAmGfUmC mCmUmGmA*mG*mC | |
| Duplex 148 | 313 | mU*mC*mAmGmGmAmCm UfAfUfCmUmCmAmAmGfA mGmAmU*mU | 256 | (E)- VPmA*fA*mUmCmUmCmUf UfGmAmGmAmUfAmGfUmC mCmUmGmA*mG*mC | |
| Duplex 149 | 319 | mC*mU*mCmUmAmUfGm AfCmUfCmAmGmAmAmA mCmAmAmAmA | 257 | mU*fU*mUmUmGfUmUfUfC mUmGfAmGfUmCfAmUmAm GmAmG*mA*mU | 177 |
| Duplex 150 | 320 | mC*mU*mCmUmAmUmGm AfCmUfCmAfGmAmAmAm CmAmAmAmA | 257 | mU*fU*mUmUmGfUmUfUfC mUmGfAmGfUmCfAmUmAm GmAmG*mA*mU | |
| Duplex 151 | 320 | mC*mU*mCmUmAmUmGm AfCmUfCmAfGmAmAmAm CmAmAmAmA | 258 | mU*fU*mUmUmGmUmUmUf CmUmGfAmGfUmCfAmUmA mGmAmG*mA*mU | |
| Duplex 152 | 319 | mC*mU*mCmUmAmUfGm AfCmUfCmAmGmAmAmA mCmAmAmAmA | 259 | mU*fU*mUfUmGmUmUmUm CmUmGfAmGfUmCfAmUmA mGmAmG*mA*mU | |
| Duplex 153 | 321 | mC*mU*mCmUmAmUfGm AfCfUfCmAmGmAfAmAmC mAmAmAmA | 260 | mU*fU*mUmUmGfUmUfUfCf UmGmAmGfUmCfAmUmAm GmAmG*mA*mU | |
| Duplex 154 | 322 | mC*mU*mCmUmAmUfGm AfCfUdCmAmGmAmAmAm CmAmAmAmA | 260 | mU*fU*mUmUmGfUmUfUfCf UmGmAmGfUmCfAmUmAm GmAmG*mA*mU | |
| Duplex 155 | 322 | mC*mU*mCmUmAmUfGm AfCfUdCmAmGmAmAmAm CmAmAmAmA | 261 | mU*fU*mUfUmGfUmUfUmC mUmGmAmGfUmCfAmUfAm GfAmG*mA*mU | |
| Duplex 156 | 323 | mC*mU*mCmUmAmUfGm AfCfUfCmAfGmAmAmAmC mAmAmAmA | 262 | mU*fU*mUmUmGfUmUmUm CmUmGmAmGfUmCfAmUfA mGmAmG*mA*mU | |
| Duplex 157 | 324 | mC*mU*mCmUmAmUfGm AfCfUfCmAmGmAmAfAmC mAmAmAmA | 263 | mU*fU*mUmUmGmUmUfUf CmUmGmAmGfUmCfAmUm AmGmAmG*mA*mU | |
| Duplex 158 | 325 | mC*mU*mCmUmAmUfGm AfCfUfCmAmGmAmAmAfC mAmAmAmA | 263 | mU*fU*mUmUmGmUmUfUf CmUmGmAmGfUmCfAmUm AmGmAmG*mA*mU | |
| Duplex 159 | 326 | mC*mU*mCmUmAmUfGm AfCdTfCmAmGmAmAmAfC mAmAmA*mA | 263 | mU*fU*mUmUmGmUmUfUf CmUmGmAmGfUmCfAmUm AmGmAmG*mA*mU | |
| Duplex 160 | 327 | mC*mU*mCmUmAmUfGm AdCfUfCmAmGmAmAmAf CmAmAmA*mA | 263 | mU*fU*mUmUmGmUmUfUf CmUmGmAmGfUmCfAmUm AmGmAmG*mA*mU | |
| Duplex 161 | 328 | mC*mU*mCmUmAmUfGm AfCfUdCmAmGmAmAmAf CmAmAmA*mA | 263 | mU*fU*mUmUmGmUmUfUf CmUmGmAmGfUmCfAmUm AmGmAmG*mA*mU | |
| Duplex 162 | 329 | mC*mU*mCmUmAmUmGm AfCfUfCmAmGmAmAmAfC mAmAmA*mA | 263 | mU*fU*mUmUmGmUmUfUf CmUmGmAmGfUmCfAmUm AmGmAmG*mA*mU | |
| Duplex 163 | 330 | mC*mU*mCmUmAmUfGm AfCfUfCmAmGmAmAmAfC mAmAmA*mA | 263 | mU*fU*mUmUmGmUmUfUf CmUmGmAmGfUmCfAmUm AmGmAmG*mA*mU | |
| Duplex 164 | 331 | mC*mU*mCmUmAmUfGm AfCfUfCmAmGmAmAmAfC mAmA*mA*mA | 263 | mU*fU*mUmUmGmUmUfUf CmUmGmAmGfUmCfAmUm AmGmAmG*mA*mU | |
| Duplex 165 | 332 | mC*mU*mCmUmAmUfGm AfCfUfCmAmGmAmAfAfC mAmAmA*mA | 263 | mU*fU*mUmUmGmUmUfUf CmUmGmAmGfUmCfAmUm AmGmAmG*mA*mU | |
| Duplex 166 | 333 | mC*mU*mCmUmAmUfGm AfCfUfCmAmGmAfAmAfC mAmAmA*mA | 263 | mU*fU*mUmUmGmUmUfUf CmUmGmAmGfUmCfAmUm AmGmAmG*mA*mU | |
| Duplex 167 | 330 | mC*mU*mCmUmAmUfGm AfCfUfCmAmGmAmAmAfC mAmAmA*mA | 264 | (E)- VPmU*fU*mUmUmGmUmUf UfCmUmGmAmGfUmCfAmU mAmGmAmG*mA*mU | |
| Duplex 168 | 326 | mC*mU*mCmUmAmUfGm AfCdTfCmAmGmAmAmAfC mAmAmA*mA | 264 | (E)- VPmU*fU*mUmUmGmUmUf UfCmUmGmAmGfUmCfAmU mAmGmAmG*mA*mU | |
| Duplex 169 | 334 | mC*mU*mCmUmAmUfGm AfCdTfCmAmGmAmAmAm CmAmAmA*mA | 264 | (E)- VPmU*fU*mUmUmGmUmUf UfCmUmGmAmGfUmCfAmU mAmGmAmG*mA*mU | |
| Duplex 170 | 333 | mC*mU*mCmUmAmUfGm AfCfUfCmAmGmAfAmAfC mAmAmA*mA | 264 | (E)- VPmU*fU*mUmUmGmUmUf UfCmUmGmAmGfUmCfAmU mAmGmAmG*mA*mU | |
| Duplex 171 | 331 | mC*mU*mCmUmAmUfGm AfCfUfCmAmGmAmAmAfC mAmA*mA*mA | 264 | (E)- VPmU*fU*mUmUmGmUmUf UfCmUmGmAmGfUmCfAmU mAmGmAmG*mA*mU | |
| Duplex 172 | 329 | mC*mU*mCmUmAmUmGm AfCfUfCmAmGmAmAmAfC mAmAmA*mA | 264 | (E)- VPmU*fU*mUmUmGmUmUf UfCmUmGmAmGfUmCfAmU mAmGmAmG*mA*mU | |
| Duplex 177 | 335 | mA*mC*mAmGmUmUfUm AfGfAfAmGmCmCfAmAmA mCmUmCmA | 265 | mU*fG*mAmGmUfUmUfGfGf CmUmUmCfUmAfAmAmCm UmGmU*mU*mG | 205 |
| Duplex 180 | 336 | mA*mC*mAmGmUmUfUm AfGfAfAmGfCmCmAmAmA mCmUmCmA | 266 | mU*fG*mAmGmUfUmUmGm GmCmUmUmCfUmAfAmAfC mUmGmU*mU*mG | |
| Duplex 181 | 337 | mA*mC*mAmGmUmUfUm AfGfAfAmGmCmCmAfAmA mCmUmCmA | 267 | mU*fG*mAmGmUmUmUfGf GmCmUmUmCfUmAfAmAm CmUmGmU*mU*mG | |
| Duplex 182 | 338 | mA*mC*mAmGmUmUfUm AfGfAfAmGmCmCmAmAfA mCmUmCmA | 267 | mU*fG*mAmGmUmUmUfGf GmCmUmUmCfUmAfAmAm CmUmGmU*mU*mG | |
| Duplex 183 | 339 | mC*mA*mGmUmUmUfAm GfAmAfGmCmCmAmAmA mCmUmCmAmA | 268 | mU*fU*mGmAmGfUmUfUfG mGmCfUmUfCmUfAmAmAm CmUmG*mU*mU | 206 |
| Duplex 184 | 340 | mC*mA*mGmUmUmUmAm GfAmAfGmCfCmAmAmAm CmUmCmAmA | 268 | mU*fU*mGmAmGfUmUfUfG mGmCfUmUfCmUfAmAmAm CmUmG*mU*mU | |
| Duplex 185 | 340 | mC*mA*mGmUmUmUmAm GfAmAfGmCfCmAmAmAm CmUmCmAmA | 269 | mU*fU*mGmAmGmUmUmUf GmGmCfUmUfCmUfAmAmA mCmUmG*mU*mU | |
| Duplex 187 | 341 | mC*mA*mGmUmUmUfAm GfAfAfGmCmCmAfAmAmC mUmCmAmA | 270 | mU*fU*mGmAmGfUmUfUfGf GmCmUmUfCmUfAmAmAm CmUmG*mU*mU | |
| Duplex 188 | 342 | mA*mU*mCmGmAmUfUm AfGmUfGmUmCmAmAmA mGmGmCmUmU | 271 | mA*fA*mGmCmCfUmUfUfG mAmCfAmCfUmAfAmUmCm GmAmU*mC*mC | 424 |
| Duplex 189 | 343 | mA*mU*mCmGmAmUmUm AfGmUfGmUfCmAmAmAm GmGmCmUmU | 271 | mA*fA*mGmCmCfUmUfUfG mAmCfAmCfUmAfAmUmCm GmAmU*mC*mC | |
| Duplex 190 | 343 | mA*mU*mCmGmAmUmUm AfGmUfGmUfCmAmAmAm GmGmCmUmU | 272 | mA*fA*mGmCmCmUmUmUf GmAmCfAmCfUmAfAmUmC mGmAmU*mC*mC | |
| Duplex 191 | 342 | mA*mU*mCmGmAmUfUm AfGmUfGmUmCmAmAmA mGmGmCmUmU | 273 | mA*fA*mGfCmCmUmUmUm GmAmCfAmCfUmAfAmUmC mGmAmU*mC*mC | |
| Duplex 192 | 344 | mA*mU*mCmGmAmUfUm AfGfUfGmUmCmAfAmAmG mGmCmUmU | 274 | mA*fA*mGmCmCfUmUfUfGf AmCmAmCfUmAfAmUmCm GmAmU*mC*mC | |
| Duplex 193 | 345 | mA*mU*mCmGmAmUfUm AfGfUdGmUmCmAmAmAm GmGmCmUmU | 274 | mA*fA*mGmCmCfUmUfUfGf AmCmAmCfUmAfAmUmCm GmAmU*mC*mC | |
| Duplex 194 | 345 | mA*mU*mCmGmAmUfUm AfGfUdGmUmCmAmAmAm GmGmCmUmU | 275 | mA*fA*mGfCmCfUmUfUmGf AmCmAmCfUmAfAmUfCmGf AmU*mC*mC | |
| Duplex 195 | 346 | mA*mU*mCmGmAmUfUm AfGfUfGmUfCmAmAmAmG mGmCmUmU | 276 | mA*fA*mGmCmCfUmUmUm GmAmCmAmCfUmAfAmUfC mGmAmU*mC*mC | |
| Duplex 196 | 347 | mA*mU*mCmGmAmUfUm AfGfUfGmUmCmAmAfAmG mGmCmUmU | 277 | mA*fA*mGmCmCmUmUfUfG mAmCmAmCfUmAfAmUmC mGmAmU*mC*mC | |
| Duplex 197 | 348 | mA*mU*mCmGmAmUfUm AfGfUfGmUmCmAmAmAfG mGmCmUmU | 277 | mA*fA*mGmCmCmUmUfUfG mAmCmAmCfUmAfAmUmC mGmAmU*mC*mC | |
| Duplex 198 | 349 | mG*mU*mGmUmCmAfAm AfGmGfCmUmUmUmAmA mAmCmAmUmA | 278 | mU*fA*mUmGmUfUmUfAfA mAmGfCmCfUmUfUmGmAm CmAmC*mU*mA | 432 |
| Duplex 199 | 350 | mG*mU*mGmUmCmAmAm AfGmGfCmUfUmUmAmAm AmCmAmUmA | 278 | mU*fA*mUmGmUfUmUfAfA mAmGfCmCfUmUfUmGmAm CmAmC*mU*mA | |
| Duplex 200 | 350 | mG*mU*mGmUmCmAmAm AfGmGfCmUfUmUmAmAm AmCmAmUmA | 279 | mU*fA*mUmGmUmUmUmAf AmAmGfCmCfUmUfUmGmA mCmAmC*mU*mA | |
| Duplex 201 | 349 | mG*mU*mGmUmCmAfAm AfGmGfCmUmUmUmAmA mAmCmAmUmA | 280 | mU*fA*mUfGmUmUmUmAm AmAmGfCmCfUmUfUmGmA mCmAmC*mU*mA | |
| Duplex 202 | 351 | mG*mU*mGmUmCmAfAm AfGfGfCmUmUmUfAmAmA mCmAmUmA | 281 | mU*fA*mUmGmUfUmUfAfAf AmGmCmCfUmUfUmGmAm CmAmC*mU*mA | |
| Duplex 203 | 352 | mG*mU*mGmUmCmAfAm AfGfGdCmUmUmUmAmAm AmCmAmUmA | 281 | mU*fA*mUmGmUfUmUfAfAf AmGmCmCfUmUfUmGmAm CmAmC*mU*mA | |
| Duplex 204 | 352 | mG*mU*mGmUmCmAfAm AfGfGdCmUmUmUmAmAm AmCmAmUmA | 282 | mU*fA*mUfGmUfUmUfAmAf AmGmCmCfUmUfUmGfAmCf AmC*mU*mA | |
| Duplex 205 | 353 | mG*mU*mGmUmCmAfAm AfGfGfCmUfUmUmAmAmA mCmAmUmA | 283 | mU*fA*mUmGmUfUmUmAm AmAmGmCmCfUmUfUmGfA mCmAmC*mU*mA | |
| Duplex 206 | 354 | mG*mU*mGmUmCmAfAm AfGfGfCmUmUmUmAfAmA mCmAmUmA | 284 | mU*fA*mUmGmUmUmUfAf AmAmGmCmCfUmUfUmGm AmCmAmC*mU*mA | |
| Duplex 207 | 355 | mG*mU*mGmUmCmAfAm AfGfGfCmUmUmUmAmAfA mCmAmUmA | 284 | mU*fA*mUmGmUmUmUfAf AmAmGmCmCfUmUfUmGm AmCmAmC*mU*mA | |
| Duplex 208 | 356 | mG*mU*mGmUmCmAfAm AfGmGfCmUmUmUmAmA mAmCmA*mU*mA | 285 | (E)- VPmU*fA*mUmGmUfUmUfA fAmAmGfCmCfUmUfUmGm AmCmAmC*mU*mA | |
| Duplex 209 | 357 | mG*mU*mGmUmCmAmAm AfGmGfCmUfUmUmAmAm AmCmA*mU*mA | 286 | (E)- VPmU*fA*mUmGmUmUmU mAfAmAmGfCmCfUmUfUm GmAmCmAmC*mU*mA | |
| Duplex 210 | 358 | mG*mU*mGmUmCmAfAm AfGfGdCmUmUmUmAmAm AmCmA*mU*mA | 287 | (E)- VPmU*fA*mUmGmUfUmUfA fAfAmGmCmCfUmUfUmGm AmCmAmC*mU*mA | |
| Duplex 211 | 359 | mG*mU*mGmUmCmAfAm AfGdGfCmUmUmUmAmAm AmCmA*mU*mA | 288 | (E)- VPmU*fA*mUmGmUmUmUf AfAmAmGmCmCfUmUfUmG mAmCmAmC*mU*mA | |
| Duplex 212 | 360 | mG*mU*mGmUmCmAfAm AfGfGfCmUmUmUmAmAfA mCmA*mU*mA | 288 | (E)- VPmU*fA*mUmGmUmUmUf AfAmAmGmCmCfUmUfUmG mAmCmAmC*mU*mA | |
| Duplex 213 | 361 | mG*mU*mGmUmCmAfAm AfGmGfCmUmUmUmAmA mAmCmAmUmA*(iab) | 285 | (E)- VPmU*fA*mUmGmUfUmUfA fAmAmGfCmCfUmUfUmGm AmCmAmC*mU*mA | |
| Duplex 214 | 362 | mG*mU*mGmUmCmAmAm AfGmGfCmUfUmUmAmAm AmCmAmUmA*(iab) | 286 | (E)- VPmU*fA*mUmGmUmUmU mAfAmAmGfCmCfUmUfUm GmAmCmAmC*mU*mA | |
| Duplex 215 | 363 | mG*mU*mGmUmCmAfAm AfGfGdCmUmUmUmAmAm AmCmAmUmA*(iab) | 287 | (E)- VPmU*fA*mUmGmUfUmUfA fAfAmGmCmCfUmUfUmGm AmCmAmC*mU*mA | |
| Duplex 216 | 364 | mG*mU*mGmUmCmAfAm AfGdGfCmUmUmUmAmAm AmCmAmUmA*(iab) | 288 | (E)- VPmU*fA*mUmGmUmUmUf AfAmAmGmCmCfUmUfUmG mAmCmAmC*mU*mA | |
| Duplex 217 | 365 | mG*mU*mGmUmCmAfAm AfGfGfCmUmUmUmAmAfA mCmAmUmA*(iab) | 288 | (E)- VPmU*fA*mUmGmUmUmUf AfAmAmGmCmCfUmUfUmG mAmCmAmC*mU*mA | |
| Duplex 218 | 366 | mU*mC*mGmGmAmGfGm AfGmAfUmGmCmUmCmA mCmUmUmCmA | 289 | mU*fG*mAmAmGfUmGfAfG mCmAfUmCfUmCfCmUmCm CmGmA*mG*mA | 604 |
| Duplex 219 | 367 | mU*mC*mGmGmAmGmGm AfGmAfUmGfCmUmCmAm CmUmUmCmA | 289 | mU*fG*mAmAmGfUmGfAfG mCmAfUmCfUmCfCmUmCm CmGmA*mG*mA | |
| Duplex 220 | 367 | mU*mC*mGmGmAmGmGm AfGmAfUmGfCmUmCmAm CmUmUmCmA | 290 | mU*fG*mAmAmGmUmGmAf GmCmAfUmCfUmCfCmUmC mCmGmA*mG*mA | |
| Duplex 221 | 366 | mU*mC*mGmGmAmGfGm AfGmAfUmGmCmUmCmA mCmUmUmCmA | 291 | mU*fG*mAfAmGmUmGmAm GmCmAfUmCfUmCfCmUmC mCmGmA*mG*mA | |
| Duplex 222 | 368 | mU*mC*mGmGmAmGfGm AfGfAfUmGmCmUfCmAmC mUmUmCmA | 292 | mU*fG*mAmAmGfUmGfAfGf CmAmUmCfUmCfCmUmCmC mGmA*mG*mA | |
| Duplex 223 | 369 | mU*mC*mGmGmAmGfGm AfGfAdTmGmCmUmCmAm CmUmUmCmA | 292 | mU*fG*mAmAmGfUmGfAfGf CmAmUmCfUmCfCmUmCmC mGmA*mG*mA | |
| Duplex 224 | 369 | mU*mC*mGmGmAmGfGm AfGfAdTmGmCmUmCmAm CmUmUmCmA | 293 | mU*fG*mAfAmGfUmGfAmGf CmAmUmCfUmCfCmUfCmCf GmA*mG*mA | |
| Duplex 225 | 370 | mU*mC*mGmGmAmGfGm AfGfAfUmGfCmUmCmAmC mUmUmCmA | 294 | mU*fG*mAmAmGfUmGmAm GmCmAmUmCfUmCfCmUfC mCmGmA*mG*mA | |
| Duplex 226 | 371 | mU*mC*mGmGmAmGfGm AfGfAfUmGmCmUmCfAmC mUmUmCmA | 295 | mU*fG*mAmAmGmUmGfAf GmCmAmUmCfUmCfCmUmC mCmGmA*mG*mA | |
| Duplex 227 | 372 | mU*mC*mGmGmAmGfGm AfGfAfUmGmCmUmCmAfC mUmUmCmA | 295 | mU*fG*mAmAmGmUmGfAf GmCmAmUmCfUmCfCmUmC mCmGmA*mG*mA | |
| Duplex 228 | 373 | mU*mU*mCmGmAmUfGm AfGmGfAmUmGmAmAmC mGmCmUmGmA | 296 | mU*fC*mAmGmCfGmUfUfC mAmUfCmCfUmCfAmUmCm GmAmA*mG*mU | 621 |
| Duplex 229 | 374 | mU*mU*mCmGmAmUmGm AfGmGfAmUfGmAmAmCm GmCmUmGmA | 296 | mU*fC*mAmGmCfGmUfUfC mAmUfCmCfUmCfAmUmCm GmAmA*mG*mU | |
| Duplex 230 | 374 | mU*mU*mCmGmAmUmGm AfGmGfAmUfGmAmAmCm GmCmUmGmA | 297 | mU*fC*mAmGmCmGmUmUf CmAmUfCmCfUmCfAmUmC mGmAmA*mG*mU | |
| Duplex 231 | 373 | mU*mU*mCmGmAmUfGm AfGmGfAmUmGmAmAmC mGmCmUmGmA | 298 | mU*fC*mAfGmCmGmUmUm CmAmUfCmCfUmCfAmUmC mGmAmA*mG*mU | |
| Duplex 232 | 375 | mU*mU*mCmGmAmUfGm AfGfGfAmUmGmAfAmCmG mCmUmGmA | 299 | mU*fC*mAmGmCfGmUfUfCf AmUmCmCfUmCfAmUmCm GmAmA*mG*mU | |
| Duplex 233 | 376 | mU*mU*mCmGmAmUfGm AfGfGdAmUmGmAmAmCm GmCmUmGmA | 299 | mU*fC*mAmGmCfGmUfUfCf AmUmCmCfUmCfAmUmCm GmAmA*mG*mU | |
| Duplex 234 | 376 | mU*mU*mCmGmAmUfGm AfGfGdAmUmGmAmAmCm GmCmUmGmA | 300 | mU*fC*mAfGmCfGmUfUmCf AmUmCmCfUmCfAmUfCmGf AmA*mG*mU | |
| Duplex 235 | 377 | mU*mU*mCmGmAmUfGm AfGfGfAmUfGmAmAmCmG mCmUmGmA | 301 | mU*fC*mAmGmCfGmUmUm CmAmUmCmCfUmCfAmUfC mGmAmA*mG*mU | |
| Duplex 236 | 378 | mU*mU*mCmGmAmUfGm AfGfGfAmUmGmAmAfCmG mCmUmGmA | 302 | mU*fC*mAmGmCmGmUfUfC mAmUmCmCfUmCfAmUmC mGmAmA*mG*mU | |
| Duplex 237 | 379 | mU*mU*mCmGmAmUfGm AfGfGfAmUmGmAmAmCfG mCmUmGmA | 302 | mU*fC*mAmGmCmGmUfUfC mAmUmCmCfUmCfAmUmC mGmAmA*mG*mU | |
| The m is a 2'-oMe modification; f is a 2'-F modification; d is a DNA; (E)-VP is an (E)-vinylphosphonate modification; * is a phosphorothioate linkage; and (iab) is an inverted abasic residue. | | | | | |

### DETAILED DESCRIPTION OF THE INVENTION

The present invention will now be described in the following non-limiting examples.

Those skilled in the art will appreciate that the Examples describe the present invention by way of examples and are not intended to limit the scope of protection claimed for the present application. The experimental methods in the examples are all conventional methods, unless otherwise specified. Examples, in which no specific conditions are specified, are carried out according to conventional conditions or conditions recommended by the manufacturers. The reagents and instruments used without indicating the manufacturer are all conventional products that can be purchased commercially.

Those skilled in the art will appreciate that the siRNA described in the present invention can be obtained by conventional siRNA preparation methods in the art (such as solid-phase synthesis and liquid-phase synthesis), wherein commercial custom services are available for both solid-phase synthesis and liquid-phase synthesis. It is also clear to those skilled in the art that modified nucleotide groups can be introduced into the siRNA described in the present invention by using nucleotide monomers with corresponding modifications. Methods for preparing nucleotide monomers with corresponding modifications are well known to those skilled in the art, and commercial monomers are also available on the market.

### Example 1: Synthesis of an siRNA

The siRNA sequences were designed against the MMP7 gene sequence (see NCBI Reference Sequence: NM_002423.5). For the sense and antisense strands of the siRNA sequence of the present invention and the sense and antisense strands of modified duplexes, deoxynucleoside CPG was used as a solid support; the sense strand was synthesized using the solid support, and the antisense strand was synthesized using universal CPG.

Sequence synthesis was performed using a 48-channel synthesizer at a 0.2 µmol scale. The phosphoramidite monomer was used at a concentration of 0.05 M, and the activator was 0.3 M BTT.

The cleavage and deprotection of the sequence were performed in 1.5 ml tubes using AMA in the first step and triethylamine trihydrofluoride in the second step to remove the 2-position protecting group. For sequences containing all modifications at 2-position, aminolysis with ammonia was required. The cleaved and deprotected sequences were precipitated with a mixture of acetone:ethanol (with a volume ratio of 80:20) and dissolved in RNase-free water. Each sequence was analyzed by LC-MS to confirm sequence accuracy, spectrophotometrically quantified, and by HPLC to determine purity.

After HPLC purification, lyophilization and quality inspection, salt exchange was performed via sodium acetate-alcohol precipitation, followed by desalting using a 3KD ultrafiltration tube. After desalting, the sense strand and antisense strand were quantitatively determined by a spectrophotometer and mixed in a 1:1 ratio and annealed to form siRNA duplexes. The obtained siRNA duplexes are shown in Table 1.

### Example 2: Purification and activity detection of siRNAs

### 2-1. Cell culture and transfection

A549 cell culture: A549 cells (ATCC No: CCL-185) were cultured in an environment of 37°C and 5% CO₂ using F-12K complete medium (Gibco, supplemented with 10% FBS) until nearly confluent. The cells were then trypsinized and plated. 8,000 A549 cells together with 0.1 mL of F-12K medium (Gibco, supplemented with 10% FBS) were added to each well of a 96-well plate and cultured for 24 h. After transfecting siRNAs using lipofectamine 2000 (Invitrogen), the cells were further cultured in an environment of 37°C and 5% CO₂ for 24 h, followed by RNA extraction. Single-dose experiments were performed at duplex concentrations of 10 nM, 1.0 nM, 0.3 nM, 0.1 nM, 0.03 nM, and 0.01 nM (2 or 3 concentrations were selected for testing).

### 2-2. RNA extraction

An RNA extraction kit (Yeasen corporation, Cat: 18600ES50) was used following the instructions of this RNA extraction kit; finally, 170 µL of RNase-free water was added and the RNAs were collected.

### 2-3. Real-time fluorescence quantitative PCR

One-step RT-qPCR was performed using Yeasen's one-step RT-qPCR kit (Cat: 11143ES80) according to the steps of instructions. Real-time fluorescence PCR was performed using the ΔΔCt assay in the ABI QuantStudio^{™} 6 real-time fluorescence PCR system. Each duplex was tested in three independent transfections, and each transfection was assayed in triplicate.

The testing results of the in vitro activity of duplexes are shown in Table 3 below. It can be seen from Table 3, duplexes 1-124 all have good inhibitory activity on MMP7 mRNA expression.

**Table 3: Testing results of the single-dose duplexes in A549 cells**

| Duplex number | 10 nM siRNA | | 0.1 nM siRNA | |
|---|---|---|---|---|
| | Mean* | SD | Mean* | SD |
| Blank control | 1.00 | 0.06 | 1.00 | 0.06 |
| Mock control | 1.10 | 0.09 | 1.10 | 0.09 |
| Negative control | 0.97 | 0.06 | 0.97 | 0.06 |
| Duplex 1 | 0.05 | 0.01 | 0.23 | 0.01 |
| Duplex 2 | 0.03 | 0.00 | 0.13 | 0.02 |
| Duplex 3 | 0.03 | 0.02 | 0.15 | 0.02 |
| Duplex 4 | 0.01 | 0.01 | 0.14 | 0.08 |
| Duplex 5 | 0.03 | 0.04 | 0.13 | 0.08 |
| Duplex 6 | 0.27 | 0.12 | 0.41 | 0.12 |
| Duplex 7 | 0.11 | 0.04 | 0.27 | 0.11 |
| Duplex 8 | 0.03 | 0.01 | 0.09 | 0.06 |
| Duplex 9 | 0.03 | 0.02 | 0.05 | 0.04 |
| Duplex 10 | 0.19 | 0.09 | 0.39 | 0.11 |
| Duplex 11 | 0.02 | 0.02 | 0.09 | 0.04 |
| Duplex 12 | 0.02 | 0.02 | 0.07 | 0.02 |
| Duplex 13 | 0.11 | 0.01 | 0.46 | 0.02 |
| Duplex 14 | 0.03 | 0.01 | 0.13 | 0.02 |
| Duplex 15 | 0.02 | 0.00 | 0.28 | 0.04 |
| Duplex 16 | 0.31 | 0.07 | 0.44 | 0.13 |
| Duplex 17 | 0.18 | 0.03 | 0.74 | 0.04 |
| Duplex 18 | 0.23 | 0.05 | 0.35 | 0.08 |
| Duplex 19 | 0.07 | 0.01 | 0.45 | 0.13 |
| Duplex 20 | 0.04 | 0.00 | 0.12 | 0.04 |
| Duplex 21 | 0.21 | 0.06 | 0.33 | 0.09 |
| Duplex 22 | 0.09 | 0.02 | 0.31 | 0.08 |
| Duplex 23 | 0.31 | 0.00 | 0.47 | 0.11 |
| Duplex 24 | 0.07 | 0.01 | 0.20 | 0.07 |
| Duplex 25 | 0.17 | 0.05 | 0.24 | 0.06 |
| Duplex 26 | 0.51 | 0.02 | 0.53 | 0.10 |
| Duplex 27 | 0.05 | 0.01 | 0.10 | 0.02 |
| Duplex 28 | 0.73 | 0.03 | 0.74 | 0.06 |
| Duplex 29 | 0.17 | 0.02 | 0.33 | 0.07 |
| Duplex 30 | 0.27 | 0.09 | 0.26 | 0.02 |
| Duplex 31 | 0.33 | 0.10 | 0.33 | 0.10 |
| Duplex 32 | 0.09 | 0.02 | 0.31 | 0.09 |
| Duplex 33 | 0.02 | 0.01 | 0.06 | 0.02 |
| Duplex 34 | 0.14 | 0.06 | 0.20 | 0.05 |
| Duplex 35 | 0.10 | 0.05 | 0.23 | 0.13 |
| Duplex 36 | 0.38 | 0.07 | 0.95 | 0.04 |
| Duplex 37 | 0.14 | 0.02 | 0.66 | 0.19 |
| Duplex 38 | 0.05 | 0.02 | 0.09 | 0.02 |
| Duplex 39 | 0.08 | 0.01 | 0.24 | 0.03 |
| Duplex 40 | 0.05 | 0.01 | 0.32 | 0.03 |
| Duplex 41 | 0.30 | 0.14 | 0.37 | 0.08 |
| Duplex 42 | 0.10 | 0.02 | 0.21 | 0.01 |
| Duplex 43 | 0.16 | 0.03 | 0.58 | 0.11 |
| Duplex 44 | 0.04 | 0.00 | 0.04 | 0.03 |
| Duplex 45 | 0.05 | 0.00 | 0.34 | 0.07 |
| Duplex 46 | 0.05 | 0.00 | 0.17 | 0.04 |
| Duplex 47 | 0.11 | 0.01 | 0.31 | 0.07 |
| Duplex 48 | 0.24 | 0.02 | 0.62 | 0.05 |
| Duplex 49 | 0.32 | 0.07 | 1.15 | 0.04 |
| Duplex 50 | 0.11 | 0.01 | 0.20 | 0.07 |
| Duplex 51 | 0.11 | 0.05 | 0.27 | 0.05 |
| Duplex 52 | 0.06 | 0.02 | 0.14 | 0.03 |
| Duplex 53 | 0.04 | 0.01 | 0.20 | 0.02 |
| Duplex 54 | 0.03 | 0.01 | 0.16 | 0.03 |
| Duplex 55 | 0.05 | 0.01 | 0.44 | 0.02 |
| Duplex 56 | 0.05 | 0.00 | 0.47 | 0.05 |
| Duplex 57 | 0.35 | 0.03 | 0.89 | 0.29 |
| Duplex 58 | 0.03 | 0.01 | 0.13 | 0.02 |
| Duplex 59 | 0.03 | 0.00 | 0.20 | 0.06 |
| Duplex 60 | 0.05 | 0.00 | 0.42 | 0.04 |
| Duplex 61 | 0.03 | 0.00 | 0.08 | 0.02 |
| Duplex 62 | 0.43 | 0.01 | 1.05 | 0.06 |
| Duplex 63 | 0.07 | 0.04 | 0.34 | 0.26 |
| Duplex 64 | 0.13 | 0.06 | 0.84 | 0.14 |
| Duplex 65 | 0.04 | 0.01 | 0.12 | 0.02 |
| Duplex 66 | 0.32 | 0.03 | 0.87 | 0.11 |
| Duplex 67 | 0.06 | 0.02 | 0.15 | 0.03 |
| Duplex 68 | 0.21 | 0.01 | 0.64 | 0.15 |
| Duplex 69 | 0.20 | 0.24 | 0.30 | 0.06 |
| Duplex 70 | 0.13 | 0.05 | 0.35 | 0.07 |
| Duplex 71 | 0.08 | 0.03 | 0.24 | 0.05 |
| Duplex 72 | 0.14 | 0.04 | 0.20 | 0.04 |
| Duplex 73 | 0.06 | 0.00 | 0.09 | 0.01 |
| Duplex 74 | 0.28 | 0.05 | 0.46 | 0.02 |
| Duplex 75 | 0.09 | 0.02 | 0.20 | 0.00 |
| Duplex 76 | 0.29 | 0.38 | 0.48 | 0.01 |
| Duplex 77 | 0.21 | 0.03 | 0.55 | 0.04 |
| Duplex 78 | 0.18 | 0.03 | 0.73 | 0.09 |
| Duplex 79 | 0.24 | 0.28 | 0.23 | 0.01 |
| Duplex 80 | 0.22 | 0.04 | 0.42 | 0.10 |
| Duplex 81 | 0.13 | 0.03 | 0.30 | 0.05 |
| Duplex 82 | 0.13 | 0.03 | 0.23 | 0.03 |
| Duplex 83 | 0.24 | 0.07 | 0.63 | 0.02 |
| Duplex 84 | 0.16 | 0.02 | 0.38 | 0.04 |
| Duplex 85 | 0.04 | 0.00 | 0.11 | 0.00 |
| Duplex 86 | 0.04 | 0.00 | 0.11 | 0.02 |
| Duplex 87 | 0.09 | 0.01 | 0.20 | 0.02 |
| Duplex 88 | 0.08 | 0.01 | 0.30 | 0.02 |
| Duplex 89 | 0.30 | 0.02 | 0.79 | 0.04 |
| Duplex 90 | 0.20 | 0.02 | 0.37 | 0.03 |
| Duplex 91 | 0.28 | 0.02 | 0.64 | 0.07 |
| Duplex 92 | 0.45 | 0.10 | 0.75 | 0.01 |
| Duplex 93 | 0.89 | 0.13 | 1.16 | 0.10 |
| Duplex 94 | 0.08 | 0.00 | 0.22 | 0.02 |
| Duplex 95 | 0.07 | 0.02 | 0.14 | 0.05 |
| Duplex 96 | 0.07 | 0.01 | 0.30 | 0.03 |
| Duplex 97 | 0.09 | 0.03 | 0.29 | 0.03 |
| Duplex 98 | 0.26 | 0.03 | 0.83 | 0.07 |
| Duplex 99 | 0.21 | 0.03 | 0.51 | 0.02 |
| Duplex 100 | 0.13 | 0.02 | 0.25 | 0.03 |
| Duplex 101 | 0.28 | 0.04 | 0.54 | 0.13 |
| Duplex 102 | 0.37 | 0.01 | 0.80 | 0.14 |
| Duplex 103 | 0.08 | 0.02 | 0.14 | 0.01 |
| Duplex 104 | 0.27 | 0.02 | 0.66 | 0.04 |
| Duplex 105 | 0.19 | 0.04 | 0.26 | 0.03 |
| Duplex 106 | 0.17 | 0.02 | 0.60 | 0.05 |
| Duplex 107 | 0.21 | 0.03 | 0.54 | 0.05 |
| Duplex 108 | 0.13 | 0.01 | 0.18 | 0.02 |
| Duplex 109 | 0.12 | 0.02 | 0.25 | 0.05 |
| Duplex 110 | 0.69 | 0.02 | 0.79 | 0.15 |
| Duplex 111 | 0.12 | 0.04 | 0.16 | 0.02 |
| Duplex 112 | 0.13 | 0.03 | 0.24 | 0.04 |
| Duplex 113 | 0.16 | 0.02 | 0.25 | 0.04 |
| Duplex 114 | 0.15 | 0.04 | 0.35 | 0.01 |
| Duplex 115 | 0.15 | 0.04 | 0.26 | 0.01 |
| Duplex 116 | 0.19 | 0.05 | 0.44 | 0.14 |
| Duplex 117 | 0.62 | 0.09 | 0.65 | 0.11 |
| Duplex 118 | 0.14 | 0.01 | 0.43 | 0.08 |
| Duplex 119 | 0.12 | 0.02 | 0.19 | 0.00 |
| Duplex 120 | 0.16 | 0.02 | 0.45 | 0.09 |
| Duplex 121 | 0.13 | 0.01 | 0.29 | 0.03 |
| Duplex 122 | 0.12 | 0.00 | 0.37 | 0.06 |
| Duplex 123 | 0.11 | 0.01 | 0.35 | 0.03 |
| Duplex 124 | 0.11 | 0.01 | 0.24 | 0.03 |
| *: This value is the relative expression level of the MMP7 mRNA compared with the blank control | | | | |
| Blank control: contains neither transfection reagent nor siRNA | | | | |
| Mock control: contains transfection reagent but no siRNA | | | | |
| Negative control: Luciferase siRNA | | | | |

### 2-4. Detection of IC₅₀ and KDmax of duplexes in A549 and HCC-78 cells

The culture and transfection conditions of A549 and HCC-78 cells (BeNa Culture Collection) were the same as in section 2-1. For the IC₅₀ detection experiment, siRNAs were tested at duplex concentrations of 10 nM, 0.1 nM, 0.01 nM, 0.0025 nM, 0.001 nM, 0.0004 nM, 0.0001 nM, and 0.000001 nM. RNAs were extracted 24 hours after transfection using the same RNA extraction method as in section 2-2. The inhibitory activity of the duplexes at various concentrations was detected by real-time fluorescence quantitative PCR using the same method as in section 2-3. GraphPad Prism software was used to draw the dose-response curve and calculate the IC₅₀ value. The highest knockdown rate obtained at the above concentration points is the KDmax value of the duplex.

The testing results of IC₅₀ and KDmax of the duplexes in A549 and HCC-78 cells are shown in Tables 4 and 5, respectively. It can be seen from Table 4 that the IC₅₀ values of the 13 tested duplexes in A549 cells were all less than 10 pM, and the KDmax values were all not less than 90%. Furthermore, it can be seen from Table 5 that the IC₅₀ values of the 8 tested duplexes in HCC-78 cells were also less than 10 pM, and the KDmax values were all not less than 95%.

**Table 4: Testing results of IC₅₀ and KDmax in A549 cells**

| Duplex number | IC₅₀/pM | KDmax |
|---|---|---|
| Duplex 2 | 1.40 | 96% |
| Duplex 5 | 3.92 | 98% |
| Duplex 8 | 4.67 | 98% |
| Duplex 9 | 1.37 | 98% |
| Duplex 11 | 1.86 | 96% |
| Duplex 12 | 4.26 | 95% |
| Duplex 33 | 3.74 | 97% |
| Duplex 35 | 3.97 | 94% |
| Duplex 38 | 2.24 | 98% |
| Duplex 46 | 7.10 | 94% |
| Duplex 50 | 3.11 | 90% |
| Duplex 67 | 4.58 | 98% |
| Duplex 73 | 4.20 | 93% |

**Table 5: Testing results of IC₅₀ and KDmax in HCC-78 cells**

| Duplex number | IC₅₀/pM | KDmax |
|---|---|---|
| Duplex 2 | 8.16 | 97% |
| Duplex 9 | 6.93 | 96% |
| Duplex 11 | 7.51 | 98% |
| Duplex 12 | 5.73 | 96% |
| Duplex 38 | 3.10 | 97% |
| Duplex 46 | 3.97 | 98% |
| Duplex 67 | 3.85 | 97% |
| Duplex 73 | 2.81 | 96% |

### 2-5. Detection of the activity of chemically modified duplexes in A549 and HCC-78 cells

The method for detecting the activity of chemically modified duplexes in A549 cells was the same as in sections 2-1 to 2-3.

The testing results of the in vitro activity of modified duplexes are shown in Table 6. It can be seen from Table 6 that the in vitro inhibitory activity of duplexes 125-172, 177, 180-185, and 187-237 at 0.1 nM on MMP7 mRNA expression can all reach not less than 30%.

**Table 6: Testing results of the single-dose modified duplexes in A549 cells**

| Duplex number | 0.1 nM siRNA | | 0.01 nM siRNA | |
|---|---|---|---|---|
| | Mean* | SD | Mean* | SD |
| Blank control | 1.00 | 0.20 | 1.00 | 0.20 |
| Mock control | 0.98 | 0.06 | 0.98 | 0.06 |
| Negative control | 0.91 | 0.06 | 1.01 | 0.10 |
| Duplex 125 | 0.13 | 0.02 | 0.48 | 0.05 |
| Duplex 126 | 0.18 | 0.04 | 0.52 | 0.10 |
| Duplex 127 | 0.25 | 0.07 | 0.71 | 0.03 |
| Duplex 128 | 0.15 | 0.04 | 0.65 | 0.06 |
| Duplex 129 | 0.09 | 0.03 | 0.38 | 0.08 |
| Duplex 130 | 0.12 | 0.00 | 0.44 | 0.05 |
| Duplex 131 | 0.14 | 0.04 | 0.65 | 0.04 |
| Duplex 132 | 0.09 | 0.04 | 0.42 | 0.04 |
| Duplex 133 | 0.08 | 0.04 | 0.39 | 0.07 |
| Duplex 134 | 0.09 | 0.01 | 0.35 | 0.05 |
| Duplex 135 | 0.12 | 0.04 | 0.41 | 0.10 |
| Duplex 136 | 0.16 | 0.05 | 0.51 | 0.12 |
| Duplex 137 | 0.16 | 0.03 | 0.56 | 0.07 |
| Duplex 138 | 0.12 | 0.03 | 0.46 | 0.10 |
| Duplex 139 | 0.13 | 0.03 | 0.46 | 0.08 |
| Duplex 140 | 0.19 | 0.02 | 0.56 | 0.04 |
| Duplex 141 | 0.18 | 0.01 | 0.49 | 0.03 |
| Duplex 142 | 0.09 | 0.03 | 0.39 | 0.04 |
| Duplex 143 | 0.07 | 0.02 | 0.22 | 0.06 |
| Duplex 144 | 0.04 | 0.02 | 0.16 | 0.02 |
| Duplex 145 | 0.05 | 0.01 | 0.12 | 0.03 |
| Duplex 146 | 0.03 | 0.02 | 0.15 | 0.03 |
| Duplex 147 | 0.05 | 0.02 | 0.22 | 0.04 |
| Duplex 148 | 0.04 | 0.02 | 0.18 | 0.04 |
| Duplex 149 | 0.13 | 0.02 | 0.52 | 0.04 |
| Duplex 150 | 0.23 | 0.02 | 0.75 | 0.06 |
| Duplex 151 | 0.50 | 0.06 | 0.85 | 0.03 |
| Duplex 152 | 0.43 | 0.05 | 0.82 | 0.03 |
| Duplex 153 | 0.08 | 0.02 | 0.25 | 0.06 |
| Duplex 154 | 0.10 | 0.04 | 0.43 | 0.05 |
| Duplex 155 | 0.08 | 0.03 | 0.39 | 0.04 |
| Duplex 156 | 0.07 | 0.02 | 0.33 | 0.04 |
| Duplex 157 | 0.06 | 0.01 | 0.30 | 0.05 |
| Duplex 158 | 0.08 | 0.01 | 0.26 | 0.03 |
| Duplex 159 | 0.11 | 0.04 | 0.40 | 0.07 |
| Duplex 160 | 0.13 | 0.04 | 0.45 | 0.10 |
| Duplex 161 | 0.14 | 0.04 | 0.53 | 0.18 |
| Duplex 162 | 0.08 | 0.02 | 0.35 | 0.11 |
| Duplex 163 | 0.09 | 0.03 | 0.35 | 0.13 |
| Duplex 164 | 0.10 | 0.05 | 0.42 | 0.15 |
| Duplex 165 | 0.08 | 0.03 | 0.38 | 0.12 |
| Duplex 166 | 0.09 | 0.04 | 0.40 | 0.13 |
| Duplex 167 | 0.07 | 0.03 | 0.24 | 0.06 |
| Duplex 168 | 0.04 | 0.02 | 0.22 | 0.03 |
| Duplex 169 | 0.05 | 0.01 | 0.15 | 0.01 |
| Duplex 170 | 0.05 | 0.01 | 0.25 | 0.04 |
| Duplex 171 | 0.04 | 0.01 | 0.19 | 0.03 |
| Duplex 172 | 0.07 | 0.01 | 0.31 | 0.01 |
| Duplex 177 | 0.42 | 0.03 | 0.92 | 0.03 |
| Duplex 180 | 0.64 | 0.04 | 1.03 | 0.04 |
| Duplex 181 | 0.57 | 0.06 | 1.00 | 0.04 |
| Duplex 182 | 0.61 | 0.04 | 1.01 | 0.04 |
| Duplex 183 | 0.59 | 0.02 | 0.90 | 0.04 |
| Duplex 184 | 0.49 | 0.05 | 0.91 | 0.02 |
| Duplex 185 | 0.64 | 0.07 | 0.93 | 0.02 |
| Duplex 187 | 0.27 | 0.04 | 0.71 | 0.05 |
| Duplex 188 | 0.17 | 0.01 | 0.60 | 0.04 |
| Duplex 189 | 0.16 | 0.03 | 0.61 | 0.04 |
| Duplex 190 | 0.22 | 0.04 | 0.64 | 0.03 |
| Duplex 191 | 0.26 | 0.04 | 0.76 | 0.05 |
| Duplex 192 | 0.15 | 0.03 | 0.50 | 0.06 |
| Duplex 193 | 0.25 | 0.02 | 0.67 | 0.04 |
| Duplex 194 | 0.35 | 0.01 | 0.86 | 0.05 |
| Duplex 195 | 0.16 | 0.03 | 0.56 | 0.03 |
| Duplex 196 | 0.11 | 0.04 | 0.48 | 0.02 |
| Duplex 197 | 0.11 | 0.02 | 0.46 | 0.07 |
| Duplex 198 | 0.06 | 0.02 | 0.24 | 0.01 |
| Duplex 199 | 0.07 | 0.03 | 0.31 | 0.02 |
| Duplex 200 | 0.05 | 0.01 | 0.23 | 0.02 |
| Duplex 201 | 0.07 | 0.01 | 0.34 | 0.02 |
| Duplex 202 | 0.05 | 0.01 | 0.24 | 0.02 |
| Duplex 203 | 0.06 | 0.01 | 0.24 | 0.01 |
| Duplex 204 | 0.10 | 0.01 | 0.38 | 0.06 |
| Duplex 205 | 0.08 | 0.01 | 0.33 | 0.02 |
| Duplex 206 | 0.08 | 0.02 | 0.35 | 0.04 |
| Duplex 207 | 0.08 | 0.01 | 0.32 | 0.04 |
| Duplex 208 | 0.04 | 0.01 | 0.09 | 0.02 |
| Duplex 209 | 0.06 | 0.02 | 0.12 | 0.02 |
| Duplex 210 | 0.05 | 0.02 | 0.17 | 0.04 |
| Duplex 211 | 0.05 | 0.01 | 0.15 | 0.04 |
| Duplex 212 | 0.04 | 0.01 | 0.10 | 0.03 |
| Duplex 213 | 0.04 | 0.00 | 0.07 | 0.03 |
| Duplex 214 | 0.05 | 0.01 | 0.14 | 0.02 |
| Duplex 215 | 0.06 | 0.01 | 0.18 | 0.05 |
| Duplex 216 | 0.05 | 0.00 | 0.12 | 0.01 |
| Duplex 217 | 0.05 | 0.01 | 0.12 | 0.03 |
| Duplex 218 | 0.23 | 0.03 | 0.68 | 0.02 |
| Duplex 219 | 0.22 | 0.04 | 0.74 | 0.05 |
| Duplex 220 | 0.26 | 0.03 | 0.74 | 0.01 |
| Duplex 221 | 0.42 | 0.02 | 0.94 | 0.03 |
| Duplex 222 | 0.14 | 0.02 | 0.53 | 0.03 |
| Duplex 223 | 0.19 | 0.03 | 0.69 | 0.02 |
| Duplex 224 | 0.29 | 0.02 | 0.84 | 0.05 |
| Duplex 225 | 0.25 | 0.02 | 0.75 | 0.04 |
| Duplex 226 | 0.28 | 0.03 | 0.68 | 0.10 |
| Duplex 227 | 0.24 | 0.02 | 0.66 | 0.02 |
| Duplex 228 | 0.20 | 0.01 | 0.62 | 0.03 |
| Duplex 229 | 0.25 | 0.03 | 0.74 | 0.01 |
| Duplex 230 | 0.22 | 0.02 | 0.68 | 0.03 |
| Duplex 231 | 0.24 | 0.04 | 0.73 | 0.03 |
| Duplex 232 | 0.20 | 0.02 | 0.61 | 0.03 |
| Duplex 233 | 0.31 | 0.00 | 0.77 | 0.04 |
| Duplex 234 | 0.29 | 0.01 | 0.79 | 0.05 |
| Duplex 235 | 0.21 | 0.01 | 0.62 | 0.01 |
| Duplex 236 | 0.19 | 0.04 | 0.67 | 0.04 |
| Duplex 237 | 0.17 | 0.03 | 0.59 | 0.01 |
| *: This value is the relative expression level of the MMP7 mRNA compared with the blank control | | | | |
| Blank control: contains neither transfection reagent nor siRNA | | | | |
| Mock control: contains transfection reagent but no siRNA | | | | |
| Negative control: Luciferase siRNA | | | | |

### 2-6. Detection of IC₅₀ and KDmax of chemically modified duplexes in A549 and HCC-78 cells

The method for detecting IC50 and KDmax of chemically modified duplexes in A549 and HCC-78 cells was the same as in section 2-4.

The testing results of IC₅₀ and KDmax of the modified duplexes in A549 and HCC-78 cells are shown in Tables 7 and 8, respectively. It can be seen from Table 7 that the IC₅₀ values of the 18 tested modified duplexes in A549 cells were all less than 5 pM, and the KDmax values were all not less than 90%. Furthermore, it can be seen from Table 8 that the IC₅₀ values of the 18 tested modified duplexes in HCC-78 cells were also less than 5 pM, and the KDmax values were all not less than 95%.

**Table 7: Testing results of IC50 and KDmax of modified duplexes in A549 cells**

| Duplex number | IC₅₀/pM | KDmax |
|---|---|---|
| Duplex 143 | 3.72 | 98% |
| Duplex 144 | 1.15 | 99% |
| Duplex 145 | 1.30 | 98% |
| Duplex 146 | 1.12 | 99% |
| Duplex 148 | 1.63 | 95% |
| Duplex 167 | 3.54 | 98% |
| Duplex 169 | 1.79 | 99% |
| Duplex 171 | 2.26 | 96% |
| Duplex 208 | 1.20 | 94% |
| Duplex 209 | 1.36 | 94% |
| Duplex 210 | 1.25 | 99% |
| Duplex 211 | 0.72 | 99% |
| Duplex 212 | 1.28 | 98% |
| Duplex 213 | 0.83 | 98% |
| Duplex 214 | 0.73 | 98% |
| Duplex 215 | 0.97 | 98% |
| Duplex 216 | 1.30 | 97% |
| Duplex 217 | 0.92 | 98% |

**Table 8: Testing results of IC50 and KDmax of modified duplexes in HCC-78 cells**

| Duplex number | IC₅₀/pM | KDmax |
|---|---|---|
| Duplex 143 | 1.25 | 99% |
| Duplex 144 | 1.02 | 98% |
| Duplex 145 | 1.84 | 97% |
| Duplex 146 | 1.08 | 98% |
| Duplex 148 | 1.22 | 98% |
| Duplex 167 | 1.47 | 99% |
| Duplex 169 | 2.21 | 97% |
| Duplex 171 | 1.01 | 98% |
| Duplex 208 | 1.14 | 96% |
| Duplex 209 | 2.58 | 96% |
| Duplex 210 | 0.78 | 97% |
| Duplex 211 | 0.68 | 96% |
| Duplex 212 | 0.80 | 96% |
| Duplex 213 | 1.03 | 96% |
| Duplex 214 | 1.32 | 97% |
| Duplex 215 | 2.12 | 96% |
| Duplex 216 | 1.48 | 96% |
| Duplex 217 | 1.21 | 96% |

### Example 3: In vitro stability test of chemically modified duplexes

### 3-1. Mouse serum stability test:

An siRNA was added to a stable serum matrix (mouse serum: DMEM = 5:4 (volume ratio)), with a final concentration of the siRNA of 1 µM and a total system volume of 50 µL. 3 replicates were set for each group, which will be used as the 48 h stability samples. Another group of a stable serum without the siRNA was taken as the 0 h sample. The two groups of samples were incubated at 37°C for 48 h. After incubation, 150 µL of Loading Buffer was added to the 48 h stability sample to terminate the reaction. 150 µL of Loading Buffer was added to the 0 h stability sample, followed by the siRNA. The amount of the siRNA added was the same as that of the 48 h sample group. After mixing uniformly, 50 µL of 100 nM internal standard siRNA was added to all samples, the mixture was purified using a Clarity OTX SPE column, and finally the response of the sense and antisense strands was detected in each group of samples by LC-MS. The residual amount was calculated by comparing the ratio of the response of the 48 h sample to that of the 0 h sample.

### 3-2. Mouse lung homogenate stability test:

An siRNA was added to a 12.5 mg/mL mouse lung homogenate solution, with a final concentration of the siRNA of 1 µM and a total system volume of 50 µL. 3 replicates were set for each group, which will be used as the 72 h stability samples. Another group of a mouse lung homogenate solution without the siRNA was taken as the 0 h sample. The two groups of samples were incubated at 37°C for 72 h. After incubation, 150 µL of Loading Buffer was added to the 72 h stability sample to terminate the reaction. 150 µL of Loading Buffer was added to the 0 h stability sample, followed by the siRNA. The amount of the siRNA added was the same as that of the 72 h sample group. After mixing uniformly, 50 µL of 100 nM internal standard siRNA was added to all samples, the mixture was purified using a Clarity OTX SPE column, and finally the response of the sense and antisense strands was detected in each group of samples by LC-MS. The residual amount was calculated by comparing the ratio of the response of the 72 h sample to that of the 0 h sample.

### 3-3. Lysosomal stability test:

An siRNA was added to lysosomes (Tritosome) at 0.1 mg/mL and pH 5.0, with a final concentration of the siRNA of 1 µM and a total system volume of 50 µL. 3 replicates were set for each group, which will be used as the 48 h stability samples. Another group of lysosomes (Tritosome) without the siRNA was taken as the 0 h sample. The two groups of samples were incubated at 37°C for 48 h. After incubation, 150 µL of Loading Buffer was added to the 48 h stability sample to terminate the reaction. 150 µL of Loading Buffer was added to the 0 h stability sample, followed by the siRNA. The amount of the siRNA added was the same as that of the 48 h sample group. After mixing uniformly, 50 µL of 100 nM internal standard siRNA was added to all samples, the mixture was purified using a Clarity OTX SPE column, and finally the response of the sense and antisense strands was detected in each group of samples by LC-MS. The residual amount was calculated by comparing the ratio of the response of the 48 h sample to that of the 0 h sample.

The stability results of the sense strands and antisense strands of the modified duplexes in serum, lung homogenate, and lysosomes are shown in Table 9. It can be seen from Table 9 that the sense and antisense strands of the modified duplexes have excellent stability in serum, lung homogenate and lysosomes.

**Table 9: Stability testing results of modified duplexes in serum, lung homogenate and lysosomes**

| Duplex number | Serum stability(remaining at 48 h) | | Lung homogenate stability (remaining at 72 h) | | Lysosomal stability(remaining at 48 h) | |
|---|---|---|---|---|---|---|
| | Sense strand (SS) | Antisense strand (AS) | Sense strand (SS) | Antisense strand (AS) | Sense strand (SS) | Antisense strand (AS) |
| Duplex 143 | 98% | 99% | 97% | 90% | 96% | 100% |
| Duplex 144 | 98% | 99% | 97% | 95% | 99% | 98% |
| Duplex 145 | 97% | 99% | 86% | 81% | 100% | 100% |
| Duplex 146 | 93% | 93% | 95% | 92% | 91% | 97% |
| Duplex 148 | 98% | 99% | 94% | 89% | 98% | 93% |
| Duplex 167 | 90% | 93% | 90% | 64% | 95% | 95% |
| Duplex 169 | 96% | 99% | 94% | 71% | 100% | 93% |
| Duplex 171 | 99% | 96% | 97% | 70% | 88% | 92% |
| Duplex 208 | 96% | 94% | 87% | 61% | 100% | 100% |
| Duplex 209 | 93% | 90% | 85% | 61% | 95% | 100% |
| Duplex 210 | 98% | 98% | 75% | 50% | 96% | 88% |
| Duplex 211 | 93% | 92% | 87% | 60% | 95% | 100% |
| Duplex 212 | 95% | 100% | 90% | 60% | 100% | 95% |
| Duplex 213 | 96% | 88% | 95% | 74% | 100% | 100% |
| Duplex 214 | 98% | 93% | 93% | 72% | 100% | 100% |
| Duplex 215 | 100% | 95% | 77% | 60% | 100% | 100% |
| Duplex 216 | 99% | 94% | 90% | 72% | 97% | 100% |
| Duplex 217 | 100% | 100% | 94% | 73% | 100% | 100% |

### Example 4: Detection experiment of fibrosis activity in vitro

### 4.1 TGF-β1 induced fibrosis in A549 cells

The A549 cells were cultured under culture conditions that were the same as in Example 2, until nearly confluent. The cells were trypsinized and plated. 1×10⁵ cells together with 0.5 mL of medium were added to each well of a 24-well plate and cultured overnight. Then the medium was replaced with serum-free medium. For the sample group, the modified duplexes with a final concentration of 2 nM were transfected using lipofectamine 2000 (Invitrogen), while the blank control group and the TGF-β1 alone treatment group contained no modified duplex. After 24 h, TGF-β1 with a final concentration of 10 ng/mL was added to the sample group and the TGF-β1 alone treatment group, and the corresponding volume of serum-free medium was added to the blank group. After continuing the culture for 2 days, RNA extraction was performed, and the mRNA levels of MMP7 and collagen1A were detected by real-time fluorescence quantitative PCR. The RNA extraction and real-time fluorescence quantitative PCR methods were the same as in Example 2.

### 4.2 Primary type II alveolar epithelial cells

Culture of primary type II alveolar epithelial cells (purchased from iCell Bioscience Inc.): The cells were cultured in an epithelial cell medium (purchased from iCell Bioscience Inc., supplemented with 10% FBS and 1% double antibiotics (penicillin-streptomycin (10,000 U/mL))) in an environment of 37°C and 5% CO₂ until near confluent. After trypsin digestion, the cells were plated. 6×10⁴ cells together with 0.5 mL of medium were added to each well of a 24-well plate. The methods for cell transfection, stimulation and detection were the same as those in Example 4.1.

### 4.3 Primary lung fibroblasts

Primary lung fibroblasts (purchased from iCell Bioscience Inc.) were cultured in a fibroblast medium (purchased from iCell Bioscience Inc., supplemented with 10% FBS and 1% double antibiotics (penicillin-streptomycin (10,000 U/mL))); other culture conditions, and the methods for cell transfection, stimulation, and detection were the same as those in Example 4.2.

The results show that after transfection of the modified duplexes in Table 10 in the three types of cells, the knockdown efficiency of MMP7 is greater than 90%. The relative level detection results of collagen 1A mRNA are shown in Table 10. It can be seen from the results that the modified duplexes can downregulate the expression of collagen 1A mRNA induced by TGF-β1.

**Table 10: Relative expression levels of Collagen 1A mRNA in fibrosis experiments**

| Duplex number | A549 | | Type II alveolar epithelial cells | | Lung fibroblasts | |
|---|---|---|---|---|---|---|
| | Mean* | SD | Mean* | SD | Mean* | SD |
| Blank control | 1.00 | 0.44 | 1.00 | 0.10 | 1.00 | 0.10 |
| TGF-β1 alone treatment | 38.49 | 3.90 | 2.45 | 0.23 | 2.26 | 0.20 |
| Duplex 144 | 21.26 | 3.96 | / | | 1.10 | 0.03 |
| Duplex 145 | 16.27 | 3.00 | 1.51 | 0.02 | 1.02 | 0.26 |
| Duplex 146 | / | | 1.54 | 0.06 | 0.50 | 0.03 |
| Duplex 148 | / | | | | 0.57 | 0.08 |
| Duplex 213 | 12.52 | 2.51 | 1.30 | 0.02 | 0.89 | 0.03 |
| Duplex 214 | 18.62 | 6.23 | 1.40 | 0.00 | / | |
| Duplex 216 | 19.07 | 3.39 | 1.31 | 0.04 | 0.77 | 0.01 |
| Duplex 169 | / | | 1.21 | 0.05 | 0.63 | 0.02 |
| *: This value is the relative expression level of the collagen 1A mRNA compared with the blank control | | | | | | |
| /: Not detected | | | | | | |

### Example 5: In vivo activity detection

For in vivo activity detection, all modified duplexes used were conjugated with an αvβ6 integrin ligand as the targeting head at the 5' end of the sense strand via a linker. The linker and targeting head used were the tridentate αvβ6 epithelial cell targeting ligand of Tri-SM6.1-αvβ6-(TA14) shown in FIG. 1 of patent WO2023070082A2. The conjugated modified duplexes were named by adding the suffix -TRiM to their original numbers.

### 5.1 In vivo activity detection in MMP7+/- heterozygous mice

In vivo administration: Commercial MMP7+/- heterozygous mice (purchased from Biocytogen) were used. These transgenic mice contained the full-length human-derived MMP7 gene (CDS+UTR). In the in vivo experiment, for the 0.5 mg/kg dose group, each group contained 4 mice, which were male and 6-8 weeks old; for the 3 mg/kg dose group, each group contained 4 mice, which were female and 6-8 weeks old. Candidate siRNA molecules at a dose of 0.5 mg/kg or 3 mg/kg were delivered to the lung via airway administration with an atomizing needle. For the normal saline group, normal saline was delivered instead. The volume for each atomization was 50 µl. Mice were anesthetized and sacrificed 3 or 7 days after administration. After perfusion, lung tissues were collected to detect the mRNA expression level of MMP7.

RNA expression detection: After removing the lung tissue, a tissue protection solution was added, and the mixture was placed at 4°C overnight, and then placed in a -80°C refrigerator for long-term storage. The tissue protection solution was discarded, and the formulated tissue lysis solution was added according to the instructions of TaKaRa Mini BEST Universal RNA Extraction Kit (Takara: 9767), and magnetic beads were added followed by homogenization on a tissue homogenizer. After homogenization, the subsequent RNA extraction steps were performed according to the instructions. The extracted RNA was reverse-transcribed using the Takara reverse transcription kit (RR036A). The cDNA obtained from reverse transcription was amplified using the SYBR^{®} Green Kit (Accurate Biology, AG11718) to detect the expression level of the target gene. Real-time fluorescence PCR was performed using the ΔΔCt assay in the ABI QuantStudio^{™} 6 real-time fluorescence PCR system, with GAPDH as the internal reference. Lung tissue samples of each mouse were prepared in 2-3 replicate wells.

The testing results of the in vivo activity of modified duplexes are shown in Table 11. It can be seen from the results that the modified duplexes can effectively downregulate MMP7 mRNA expression in vivo.

**Table 11: MMP7 mRNA expression levels in MMP7+/- heterozygous mice after airway administration via atomization**

| Duplex number | 0.5 mg/kg (3 days) | | 3 mg/kg (3 days) | | 3 mg/kg (7 days) | |
|---|---|---|---|---|---|---|
| | Mean* | SD | Mean* | SD | Mean* | SD |
| Normal saline group | 1.00 | 0.13 | 1.00 | 0.18 | 1.00 | 0.25 |
| Duplex 145-TRiM | 0.34 | 0.02 | 0.46 | 0.23 | 0.38 | 0.06 |
| Duplex 169-TRiM | 0.47 | 0.14 | 0.28 | 0.09 | 0.28 | 0.08 |
| Duplex 213-TRiM | 0.56 | 0.02 | 0.36 | 0.10 | 0.25 | 0.09 |
| Duplex 144-TRiM | 0.36 | 0.24 | 0.32 | 0.09 | / | |
| Duplex 216-TRiM | 0.47 | 0.18 | 0.41 | 0.11 | | |
| Duplex 214-TRiM | 0.43 | 0.21 | 0.58 | 0.20 | | |
| Duplex 146-TRiM | / | | 0.45 | 0.11 | / | |
| *: This value is the relative expression level of the MMP7 mRNA compared with the normal saline group | | | | | | |
| /: Not detected | | | | | | |

### 5.2 In vivo activity detection in MMP7 homozygous mice

In vivo administration: Commercial MMP7 homozygous mice (purchased from Biocytogen) were used. These transgenic mice contained the full-length human-derived MMP7 gene (CDS+UTR). In the in vivo experiment, each group contained 3-4 mice, which were female and 6-8 weeks old. Candidate siRNA molecules at a dose of 3 mg/kg were delivered to the lung via airway administration with an atomizing needle. For the normal saline group, normal saline was delivered instead. The volume for each atomization was 50 µl. Mice were anesthetized and sacrificed 15 days after administration. First, pre-cooled PBS was used to collect alveolar lavage fluid, which was then centrifuged to collect the supernatant for protein quantification (Pierce BCA Protein Assay Kits (Thermo-23225)). Then, the expression of MMP7 protein was detected using the MMP7 detection kit (Wuhan Cloud-Clone Corp., SEA102Hu). Lung tissues were collected after perfusion to detect the expression at the MMP7 mRNA level. The methods for mouse lung tissue processing, RNA extraction and RNA expression detection were the same as those in Example 5.1.

The testing results of the in vivo activity of modified duplexes are shown in Table 12. It can be seen from the results that the modified duplexes can effectively downregulate MMP7 mRNA and MMP7 protein expression in vivo.

**Table 12: MMP7 mRNA and protein expression levels in MMP7 homozygous mice 15 days after airway administration via atomization**

| Duplex number | Relative expression levels of the mRNA in lung tissues | | Relative expression levels of MMP7 protein in alveolar lavage fluid | |
|---|---|---|---|---|
| | Mean* | SD | Mean* | SD |
| Normal saline group | 1.00 | 0.29 | 1.00 | 0.25 |
| Duplex 144-TRiM | 0.37 | 0.06 | 0.68 | 0.07 |
| Duplex 148-TRiM | 0.57 | 0.03 | 0.48 | 0.21 |
| Duplex 145-TRiM | 0.70 | 0.34 | 0.57 | 0.15 |
| Duplex 213-TRiM | 0.58 | 0.23 | 0.69 | 0.24 |
| Duplex 216-TRiM | 0.58 | 0.20 | 0.47 | 0.27 |
| *: This value is the relative expression level of the MMP7 mRNA or protein compared with the normal saline group | | | | |

Although the specific embodiments of the present invention have been described in detail, those skilled in the art will understand that various modifications and changes may be made to the details based on all the teachings published, and these changes are all within the scope of protection of the present invention. The entire scope of the present invention is given by the appended claims and any equivalents thereof.

## Claims

1. A small interfering RNA (siRNA) for inhibiting the MMP7 gene expression, wherein the siRNA comprises a sense strand and an antisense strand, wherein the antisense strand comprises at least 17 consecutive nucleotides that differ from the nucleotide sequence set forth in any of SEQ ID NO: 1 to SEQ ID NO: 124 by no more than 4 nucleotides (e.g., 0, 1, 2, 3, or 4 nucleotides), and the sense strand is at least partially complementary to the antisense strand.

2. The siRNA according to claim 1, wherein the antisense strand comprises at least 17 consecutive nucleotides that differ from the nucleotide sequence set forth in any of SEQ ID NO: 1 to SEQ ID NO: 124 by 0 or 1 nucleotide.

3. The siRNA according to claim 1 or 2, wherein the sense strand comprises at least 17 consecutive nucleotides that differ from the nucleotide sequence set forth in any of SEQ ID NO: 125 to SEQ ID NO: 248 by no more than 4 nucleotides (e.g., 0, 1, 2, 3, or 4 nucleotides);
preferably, the sense strand comprises at least 17 consecutive nucleotides that differ from the nucleotide sequence set forth in any of SEQ ID NO: 125 to SEQ ID NO: 248 by 0 or 1 nucleotide; and
preferably, the sense strand has a region within the 17 consecutive nucleotides that is at least 85% complementary to the antisense strand.

4. The siRNA according to any of claims 1-3, wherein the siRNA comprises a blunt end and/or an overhang of 1-4 nucleotides;
preferably, the siRNA comprises an overhang of 1 or 2 nucleotides;
preferably, the overhang is present at the 5' end and/or the 3' end of the antisense strand and/or the sense strand; and
preferably, the 3' end of the antisense strand of the siRNA comprises an overhang of 2 nucleotides.

5. The siRNA according to any of claims 1-4, wherein each of the antisense strand and the sense strand independently has a length of 17-30 nucleotides; preferably, the antisense strand has a length of 19-27 nucleotides; and preferably, the sense strand has a length of 17-25 nucleotides.

6. The siRNA according to any of claims 1-5, wherein the antisense strand has a length of 21-23 nucleotides, and the sense strand has a length of 19-21 nucleotides.

7. The siRNA according to any of claims 1-6, wherein there are no more than 6 nucleotide mismatches (e.g., 0, 1, 2, 3, 4, 5, or 6 mismatches) between the sense strand and the antisense strand.

8. The siRNA according to any of claims 1-7, wherein the sequences of the sense strand and the antisense strand of the siRNA are selected from sense strand and antisense strand sequences of any of duplexes 1 to 124 described in Table 1.

9. The siRNA according to any of claims 1-8, wherein the siRNA at least comprises one modified nucleotide.

10. The siRNA according to claim 9, wherein all nucleotides in the sense strand and/or antisense strand of the siRNA are modified nucleotides or nucleotide analogs.

11. The siRNA according to claim 10, wherein the modified nucleotides or nucleotide analogs are selected from 2'-methoxynucleotides, 2'-fluoronucleotides, 2'-deoxynucleotides, 2',3'-seco-nucleotide analogs, 2'-fluoroarabinonucleotides, 2'-methoxyethyl nucleotides, 2'-amino-modified nucleotides, 2'-alkyl-modified nucleotides, 3'-methoxynucleotides, 2'-allyl-modified nucleotides, phosphorothioate group-containing nucleotides, methylphosphonate group-containing nucleotides, 5'-phosphate-containing nucleotides, 5'-phosphate mimic-containing nucleotides, diol-modified nucleotides, abasic nucleotides, morpholino nucleotides, locked nucleotides, unlocked nucleotides, or glycerol nucleotides.

12. The siRNA according to any of claims 9-11, wherein the nucleotides in the sense strand of the siRNA are selected from at least two of 2'-methoxynucleotides, 2'-fluoronucleotides, and deoxynucleotides; and/or the nucleotides in the antisense strand of the siRNA are selected from 2'-methoxynucleotides and 2'-fluoronucleotides.

13. The siRNA according to any of claims 9-12, wherein the 5' end and/or 3' end of the sense strand of the siRNA optionally comprises a capping residue (e.g., iab).

14. The siRNA according to any of claims 9-13, wherein the first nucleotide at the 5' end of the antisense strand of the siRNA optionally comprises an (E)-vinylphosphonate modification.

15. The siRNA according to any of claims 1-14, wherein the sense strand and/or antisense strand of the siRNA comprises a modified internucleoside linkage;
preferably, each of the 5' end and 3' end of the sense strand independently comprises 1 or 2 phosphorothioate linkages; and/or each of the 5' end and 3' end of the antisense strand independently comprises 1 or 2 phosphorothioate linkages.

16. The siRNA according to any of claims 9-15, wherein the antisense strand of the siRNA comprises the following modification pattern:
1) AS(5'-3'): mN*fN*mNmNmNfNmNfNfNmNmNfNmNfNmNfNmNmNmNmNmN*mN*mN (SEQ ID NO: 380);
2) AS(5'-3'): mN*fN*mNmNmNmNmNmNfNmNmNfNmNfNmNfNmNmNmNmNmN*mN*mN (SEQ ID NO: 381);
3) AS(5'-3'): mN*fN*mNfNmNmNmNmNmNmNmNfNmNfNmNfNmNmNmNmNmN*mN*mN (SEQ ID NO: 382);
4) AS(5'-3'): mN*fN*mNmNmNfNmNfNfNfNmNmNmNfNmNfNmNmNmNmNmN*mN*mN (SEQ ID NO: 383);
5) AS(5'-3'): mN*fN*mNfNmNfNmNfNmNmNmNmNmNfNmNfNmNfNmNfNmN*mN*mN (SEQ ID NO: 384);
6)AS(5'-3'): mN*fN*mNmNmNfNmNmNmNmNmNmNmNfNmNfNmNfNmNmNmN*mN*mN (SEQ ID NO: 385);
7)AS(5'-3'): mN*fN*mNmNmNmNmNfNfNmNmNmNmNfNmNfNmNmNmNmNmN*mN*mN (SEQ ID NO: 386);
8) AS(5'-3'): mN*fN*mNfNmNfNmNfNmNfNmNmNmNfNmNfNmNfNmNfNmN*mN*mN (SEQ ID NO: 10 387);
9)AS(5'-3'): (E)-VPmN*fN*mNmNmNmNmNfNfNmNmNmNmNfNmNfNmNmNmNmNmN*mN*mN (SEQ ID NO: 388);
10) AS(5'-3'): (E)-VPmN*fN*mNmNmNfNmNfNfNmNmNfNmNfNmNfNmNmNmNmNmN*mN*mN (SEQ ID NO: 389);
11) AS(5'-3'): (E)-VPmN*fN*mNmNmNmNmNmNfNmNmNfNmNfNmNfNmNmNmNmNmN*mN*mN (SEQ ID NO: 390); or,
12) AS(5'-3'): (E)-VPmN*fN*mNmNmNfNmNfNfNfNmNmNmNfNmNfNmNmNmNmNmN*mN*mN (SEQ ID NO: 391);
wherein, mN is a methoxy-modified nucleotide, fN is a fluorinated nucleotide, (E)-VP indicates that the nucleotide adjacent to its right is a (E)-vinylphosphonate-modified nucleotide, and * indicates a phosphorothioate linkage.

17. The siRNA according to any of claims 9-16, wherein the sense strand of the siRNA comprises the following modification pattern:
1) SS(5'-3'): mN*mN*mNmNmNmNfNmNfNmNfNmNmNmNmNmNmNmNmNmNmN (SEQ ID NO: 392);
2) SS(5'-3'): mN*mN*mNmNmNmNmNmNfNmNfNmNfNmNmNmNmNmNmNmNmN (SEQ ID NO: 393);
3) SS(5'-3'): mN*mN*mNmNmNmNfNmNfNfNfNmNmNmNfNmNmNmNmNmNmN (SEQ ID NO: 394);
4) SS(5'-3'): mN*mN*mNmNmNmNfNmNfNfNdNmNmNmNmNmNmNmNmNmNmN (SEQ ID NO: 395);
5) SS(5'-3'): mN*mN*mNmNmNmNfNmNfNfNfNmNfNmNmNmNmNmNmNmNmN (SEQ ID NO: 396);
6) SS(5'-3'): mN*mN*mNmNmNmNfNmNfNfNfNmNmNmNmNfNmNmNmNmNmN (SEQ ID NO: 397);
7) SS(5'-3'): mN*mN*mNmNmNmNfNmNfNfNfNmNmNmNmNmNfNmNmNmNmN (SEQ ID NO: 398);
8) SS(5'-3'): mN*mN*mNmNmNmNfNmNfNdNfNmNmNmNmNmNfNmNmNmN*mN (SEQ ID NO: 399);
9) SS(5'-3'): mN*mN*mNmNmNmNfNmNdNfNfNmNmNmNmNmNfNmNmNmN*mN (SEQ ID NO: 400);
10) SS(5'-3'): mN*mN*mNmNmNmNfNmNfNfNdNmNmNmNmNmNfNmNmNmN*mN (SEQ ID NO: 401);
11) SS(5'-3'): mN*mN*mNmNmNmNmNmNfNfNfNmNmNmNmNmNfNmNmNmN*mN (SEQ ID NO: 402);
12) SS(5'-3'): mN*mN*mNmNmNmNfNmNfNfNfNmNmNmNmNmNfNmNmNmN*mN (SEQ ID NO: 403);
13) SS(5'-3'): mN*mN*mNmNmNmNfNmNfNfNfNmNmNmNmNmNfNmNmN*mN*mN (SEQ ID NO: 404);
14) SS(5'-3'): mN*mN*mNmNmNmNfNmNfNfNfNmNmNmNmNfNfNmNmNmN*mN (SEQ ID NO: 405);
15) SS(5'-3'): mN*mN*mNmNmNmNfNmNfNfNfNmNmNmNfNmNfNmNmNmN*mN (SEQ ID NO: 406);
16) SS(5'-3'): mN*mN*mNmNmNmNfNmNfNdNfNmNmNmNmNmNmNmNmNmN*mN (SEQ ID NO: 407);
17) SS(5'-3'): mN*mN*mNmNmNmNfNmNfNmNfNmNmNmNmNmNmNmNmN*mN*mN (SEQ ID NO: 408);
18) SS(5'-3'): mN*mN*mNmNmNmNmNmNfNmNfNmNfNmNmNmNmNmNmN*mN*mN (SEQ ID NO: 409);
19) SS(5'-3'): mN*mN*mNmNmNmNfNmNfNfNdNmNmNmNmNmNmNmNmN*mN*mN (SEQ ID NO: 410);
20) SS(5'-3'): mN*mN*mNmNmNmNfNmNfNdNfNmNmNmNmNmNmNmNmN*mN*mN (SEQ ID NO: 411);
21) SS(5'-3'): mN*mN*mNmNmNmNfNmNfNmNfNmNmNmNmNmNmNmNmNmNmN*(iab) (SEQ ID NO: 412);
22) SS(5'-3'): mN*mN*mNmNmNmNmNmNfNmNfNmNfNmNmNmNmNmNmNmNmN*(iab) (SEQ ID NO: 413);
23) SS(5'-3'): mN*mN*mNmNmNmNfNmNfNfNdNmNmNmNmNmNmNmNmNmNmN*(iab) (SEQ ID NO: 414);
24) SS(5'-3'): mN*mN*mNmNmNmNfNmNfNdNfNmNmNmNmNmNmNmNmNmNmN*(iab) (SEQ ID NO: 415); or,
25) SS(5'-3'): mN*mN*mNmNmNmNfNmNfNfNfNmNmNmNmNmNfNmNmNmNmN*(iab) (SEQ ID NO: 416);
wherein mN is a methoxy-modified nucleotide, fN is a fluorinated nucleotide, dN is a deoxynucleotide, * is a phosphorothioate linkage, and (iab) is an inverted abasic residue.

18. The siRNA according to any of claims 9-17, wherein the antisense strand comprises a nucleotide sequence set forth in any of SEQ ID NOs: 249-302.

19. The siRNA according to any of claims 9-18, wherein the sense strand comprises a nucleotide sequence set forth in any of SEQ ID NOs: 303-379.

20. The siRNA according to any of claims 9-19, wherein the sequences of the sense strand and antisense strand of the siRNA are selected from sense strand and antisense strand sequences of any of duplex 125 to duplex 172, duplex 177, duplex 180 to duplex 185, and duplex 187 to duplex 237 described in Table 2.

21. A conjugate, wherein the conjugate comprises the siRNA according to any of claims 1-20 and a pharmaceutically acceptable targeting molecule;
preferably, the targeting molecule has an affinity for an epithelial cell surface receptor; and
preferably, the targeting molecule is an integrin ligand, such as αvβ6 ligand.

22. A pharmaceutical composition, wherein the pharmaceutical composition comprises the siRNA according to any of claims 1-20 or the conjugate according to claim 21, and a pharmaceutically acceptable carrier and/or excipient.

23. The pharmaceutical composition according to claim 22, wherein the pharmaceutically acceptable carrier is a 15 delivery carrier; preferably, the siRNA is encapsulated by the delivery carrier.

24. Use of the siRNA according to any of claims 1-20, the conjugate according to claim 21, or the pharmaceutical composition according to claim 22 or 23, in the manufacture of a medicament for treating and/or preventing an MMP7-related pathological condition or disease;
wherein preferably, the siRNA, the conjugate, or the pharmaceutical composition is used alone or in combination with an additional pharmaceutically active agent (e.g., an siRNA targeting a different target sequence in the MMP7 gene or an siRNA targeting another target).

25. The use according to claim 24, wherein the MMP7-related pathological condition or disease is a pulmonary inflammatory disease, such as pulmonary fibrosis (e.g., idiopathic pulmonary fibrosis, interstitial lung disease) or chronic obstructive pulmonary disease.

26. The use according to claim 24, wherein the MMP7-related pathological condition or disease is a fibrotic disease, such as renal fibrosis or hepatic fibrosis.

27. A method for preventing and/or treating an MMP7-related pathological condition or disease in a subject, comprising administering an effective amount of the siRNA according to any of claims 1-20, the conjugate according to claim 21, or the pharmaceutical composition according to claim 22 or 23 to a subject in need thereof;
wherein preferably, the siRNA, the conjugate, or the pharmaceutical composition is used alone or in combination with an additional pharmaceutically active agent (e.g., an siRNA targeting a different target sequence in the MMP7 gene or an siRNA targeting another target).

28. The method according to claim 27, wherein the MMP7-related pathological condition or disease is a pulmonary inflammatory disease, such as pulmonary fibrosis (e.g., idiopathic pulmonary fibrosis, interstitial lung disease) or chronic obstructive pulmonary disease.

29. The use according to claim 27, wherein the MMP7-related pathological condition or disease is a fibrotic disease, such as renal fibrosis or hepatic fibrosis.

30. A small interfering RNA (siRNA), wherein the siRNA comprises a sense strand and an antisense strand, wherein the antisense strand comprises a modification pattern selected from the following 1) to 12):
1) AS(5'-3'): mN*fN*mNmNmNfNmNfNfNmNmNfNmNfNmNfNmNmNmNmNmN*mN*mN (SEQ ID NO: 380);
2) AS(5'-3'): mN*fN*mNmNmNmNmNmNfNmNmNfNmNfNmNfNmNmNmNmNmN*mN*mN (SEQ ID NO: 381);
3) AS(5'-3'): mN*fN*mNfNmNmNmNmNmNmNmNfNmNfNmNfNmNmNmNmNmN*mN*mN (SEQ ID NO: 382);
4) AS(5'-3'): mN*fN*mNmNmNfNmNfNfNfNmNmNmNfNmNfNmNmNmNmNmN*mN*mN (SEQ ID NO: 383);
5) AS(5'-3'): mN*fN*mNfNmNfNmNfNmNmNmNmNmNfNmNfNmNfNmNfNmN*mN*mN (SEQ ID NO: 384);
6) AS(5'-3'): mN*fN*mNmNmNfNmNmNmNmNmNmNmNfNmNfNmNfNmNmNmN*mN*mN (SEQ ID NO: 385);
7) AS(5'-3'): mN*fN*mNmNmNmNmNfNfNmNmNmNmNfNmNfNmNmNmNmNmN*mN*mN (SEQ ID NO: 386);
8) AS(5'-3'): mN*fN*mNfNmNfNmNfNmNfNmNmNmNfNmNfNmNfNmNfNmN*mN*mN (SEQ ID NO: 387);
9) AS(5'-3'): (E)-VPmN*fN*mNmNmNmNmNfNfNmNmNmNmNfNmNfNmNmNmNmNmN*mN*mN (SEQ ID NO: 388);
10) AS(5'-3'): (E)-VPmN*fN*mNmNmNfNmNfNfNmNmNfNmNfNmNfNmNmNmNmNmN*mN*mN (SEQ ID NO: 389);
11) AS(5'-3'): (E)-VPmN*fN*mNmNmNmNmNmNfNmNmNfNmNfNmNfNmNmNmNmNmN*mN*mN (SEQ ID NO: 390); or,
12) AS(5'-3'): (E)-VPmN*fN*mNmNmNfNmNfNfNfNmNmNmNfNmNfNmNmNmNmNmN*mN*mN (SEQ ID NO: 391);
wherein, mN is a methoxy-modified nucleotide, fN is a fluorinated nucleotide, (E)-VP indicates that the nucleotide adjacent to its right is a (E)-vinylphosphonate-modified nucleotide, and * indicates a phosphorothioate linkage;
and/or,
the sense strand comprises a modification pattern selected from the following 1) to 25):
1) SS(5'-3'): mN*mN*mNmNmNmNfNmNfNmNfNmNmNmNmNmNmNmNmNmNmN (SEQ ID NO: 392);
2) SS(5'-3'): mN*mN*mNmNmNmNmNmNfNmNfNmNfNmNmNmNmNmNmNmNmN (SEQ ID NO: 393);
3) SS(5'-3'): mN*mN*mNmNmNmNfNmNfNfNfNmNmNmNfNmNmNmNmNmNmN (SEQ ID NO: 394);
4) SS(5'-3'): mN*mN*mNmNmNmNfNmNfNfNdNmNmNmNmNmNmNmNmNmNmN (SEQ ID NO: 395);
5) SS(5'-3'): mN*mN*mNmNmNmNfNmNfNfNfNmNfNmNmNmNmNmNmNmNmN (SEQ ID NO: 396);
6) SS(5'-3'): mN*mN*mNmNmNmNfNmNfNfNfNmNmNmNmNfNmNmNmNmNmN (SEQ ID NO: 397);
7) SS(5'-3'): mN*mN*mNmNmNmNfNmNfNfNfNmNmNmNmNmNfNmNmNmNmN (SEQ ID NO: 398);
8) SS(5'-3'): mN*mN*mNmNmNmNfNmNfNdNfNmNmNmNmNmNfNmNmNmN*mN (SEQ ID NO: 399);
9) SS(5'-3'): mN*mN*mNmNmNmNfNmNdNfNfNmNmNmNmNmNfNmNmNmN*mN (SEQ ID NO: 400);
10) SS(5'-3'): mN*mN*mNmNmNmNfNmNfNfNdNmNmNmNmNmNfNmNmNmN*mN (SEQ ID NO: 401);
11) SS(5'-3'): mN*mN*mNmNmNmNmNmNfNfNfNmNmNmNmNmNfNmNmNmN*mN (SEQ ID NO: 402);
12) SS(5'-3'): mN*mN*mNmNmNmNfNmNfNfNfNmNmNmNmNmNfNmNmNmN*mN (SEQ ID NO: 403);
13) SS(5'-3'): mN*mN*mNmNmNmNfNmNfNfNfNmNmNmNmNmNfNmNmN*mN*mN (SEQ ID NO: 404);
14) SS(5'-3'): mN*mN*mNmNmNmNfNmNfNfNfNmNmNmNmNfNfNmNmNmN*mN (SEQ ID NO: 405);
15) SS(5'-3'): mN*mN*mNmNmNmNfNmNfNfNfNmNmNmNfNmNfNmNmNmN*mN (SEQ ID NO: 406);
16) SS(5'-3'): mN*mN*mNmNmNmNfNmNfNdNfNmNmNmNmNmNmNmNmNmN*mN (SEQ ID NO: 407);
17) SS(5'-3'): mN*mN*mNmNmNmNfNmNfNmNfNmNmNmNmNmNmNmNmN*mN*mN (SEQ ID NO: 408);
18) SS(5'-3'): mN*mN*mNmNmNmNmNmNfNmNfNmNfNmNmNmNmNmNmN*mN*mN (SEQ ID NO: 409);
19) SS(5'-3'): mN*mN*mNmNmNmNfNmNfNfNdNmNmNmNmNmNmNmNmN*mN*mN (SEQ ID NO: 410);
20) SS(5'-3'): mN*mN*mNmNmNmNfNmNfNdNfNmNmNmNmNmNmNmNmN*mN*mN (SEQ ID NO: 411);
21) SS(5'-3'): mN*mN*mNmNmNmNfNmNfNmNfNmNmNmNmNmNmNmNmNmNmN*(iab) (SEQ ID NO: 412);
22) SS(5'-3'): mN*mN*mNmNmNmNmNmNfNmNfNmNfNmNmNmNmNmNmNmNmN*(iab) (SEQ ID NO: 413);
23) SS(5'-3'): mN*mN*mNmNmNmNfNmNfNfNdNmNmNmNmNmNmNmNmNmNmN*(iab) (SEQ ID NO: 414);
24) SS(5'-3'): mN*mN*mNmNmNmNfNmNfNdNfNmNmNmNmNmNmNmNmNmNmN*(iab) (SEQ ID NO: 415); or,
25) SS(5'-3'): mN*mN*mNmNmNmNfNmNfNfNfNmNmNmNmNmNfNmNmNmNmN*(iab) (SEQ ID NO: 416);
wherein mN is a methoxy-modified nucleotide, fN is a fluorinated nucleotide, dN is a deoxynucleotide, * is a phosphorothioate linkage, and (iab) is an inverted abasic residue.
